(19) Europäisches Patentamt
European Patent Office
Office européen des brevets

(11) Publication number : **0 323 050 B1**

(12) # EUROPEAN PATENT SPECIFICATION

(45) Date of publication of patent specification : **02.11.94 Bulletin 94/44**

(51) Int. Cl.$^5$ : **G03F 7/004**

(21) Application number : **88311462.1**

(22) Date of filing : **02.12.88**

(54) **Photosensitive compound.**

(30) Priority : **04.12.87 JP 306878/87**
**07.12.87 JP 309336/87**
**08.12.87 JP 310737/87**

(43) Date of publication of application : **05.07.89 Bulletin 89/27**

(45) Publication of the grant of the patent : **02.11.94 Bulletin 94/44**

(84) Designated Contracting States : **DE FR GB**

(56) References cited :
**CHEMISCHE BERICHTE, vol. 99, no. 10, 1966, WEINHEIM, DE, pp. 3128-3147 ; Manfred REGITZ : "Synthese von Diacyl-diazomethanen durch Diazogruppenübertragung"**
**IEEE TRANSACTIONS ON ELECTRON DEVICES, vol. ED-28, no. 11, November 1981, NEW YORK, US, pp. 1300-1305 ; B.D: GRANT et al. : "Deep UV Photoresists I. Meldrum's Diazo Sensitizer"**

(73) Proprietor : **WAKO PURE CHEMICAL INDUSTRIES LTD**
**1-2 Doshomachi-3-chome**
**Chuo-ku Osaka (JP)**
Proprietor : **MATSUSHITA ELECTRIC INDUSTRIAL CO., LTD.**
**1006, Oaza Kadoma**
**Kadoma-shi, Osaka-fu, 571 (JP)**

(72) Inventor : **Ogawa, Kazufumi**
**30-5 Higashinakaburi-1-chome**
**Hirakata-shi (JP)**
Inventor : **Ohno, Keiji**
**15-501 Higashisakado-1-chome**
**Sakado-shi (JP)**
Inventor : **Endo, Masayuki**
**782, Kurodoricho**
**Izumi-shi (JP)**
Inventor : **Nagoya, Mamoru**
**Kosumotopia 201**
**700-7 Tsurugaoka**
**Tsurugashimamachi Iruma-gun Saitama-ken (JP)**

(74) Representative : **Baillie, Iain Cameron et al**
**c/o Ladas & Parry**
**Altheimer Eck 2**
**D-80331 München (DE)**

**EP 0 323 050 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to a photosensitive compound useful as a photosensitive agent for lithography, e.g. for producing semiconductor devices.

With recent higher density and larger scale integration of semiconductor devices, wavelengths used in exposing devices for minute processing, particularly for lithography become shorter and shorter. Now, KrF excimer laser light (248.4 nm) is studied. But, there have been no photosensitive materials suitable for such a wavelength.

For example, even when MP 2400 (mfd. by Shipley Corp.), which is known among photoresists to have high sensitivity to KrF excimer laser light and good light transmittance, is used, pattern formation after development is very bad and cannot be used practically [K. Ogawa et al., J. Electrochem. Soc., 135, p2347 (1988)].

This seems to be derived from the fact that MP 2400 resist has a large surface absorption for the exposed light.

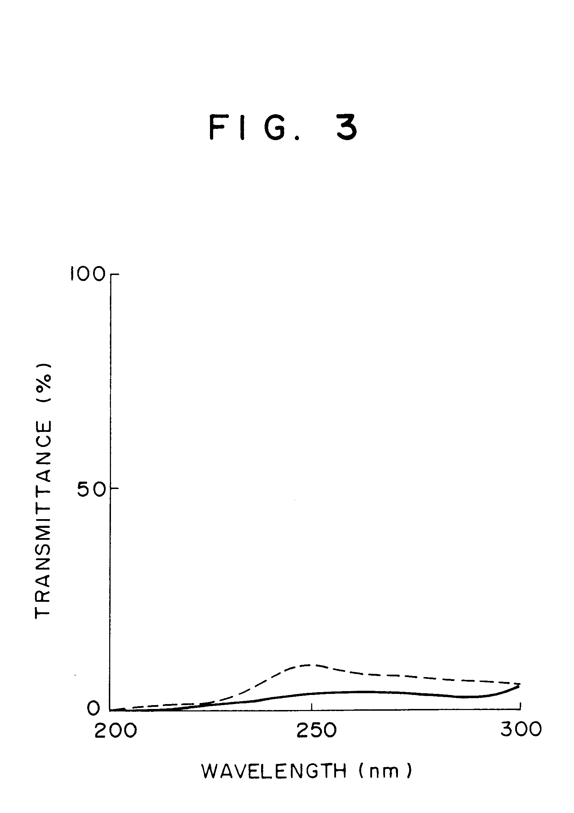

This can be interpreted that the main polymer (resin) per se used in the resist has a large light absorption against the exposed light, or a photosensitive material in the resist has no good light reactivity. That is, photosensitive materials such as naphthoquinone diazides heretofore used in known resists generally have a large absorption against a light near 248.4 nm and are hardly improved in transmittance after exposure to light. For example, in the case of MP 2400 with a film thickness of 1 μm, changes in light transmittance before and after exposure to light of KrF excimer laser (248.4 nm) is only about 3% at 248.4 nm as shown in Fig. 3 wherein the full line is before exposure and the dotted line is after exposure. This means that the reactivity is poor.

On the other hand, U.S. Patent No. 4,622,283 to Gray discloses a lithographic resist composition for use with deep UV light of less than 300 nm wavelength containing as a photosensitive solubilizing agent a compound of the formula:

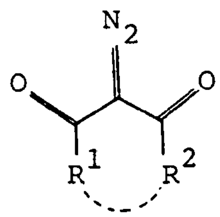

wherein $R^1$ and $R^2$ can each individually be alkyl, aryl, alkoxyalkyl, aralkyl or haloalkyl radicals or $R^1$ and $R^2$ taken together can be an alkylene radical. But this composition is insufficient in preventing non-light exposed portion from erosion of an alkaline developing solution to cause erosion of retaining portions of resist film (hereinafter referred to as "film erosion") in large amounts, which results in causing deterioration in contrast of the resist pattern.

Further, U.S. Patent No. 4,339,522 to Balanso et al disclose a lithographic resist composition comprising a phenolic-aldehyde resin and a deep ultraviolet sensitizer of the formula:

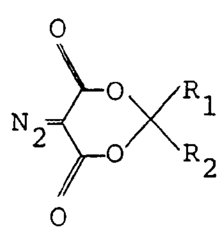

wherein $R_1$ is alkyl or aryl; and $R_2$ is H, alkyl or aryl, or together $R_1$ and $R_2$ are cycloalkyl. But this U.S. Patent is quite silent on the reactivity against KrF excimer laser, resistance to alkaline developing solution and improvement in the contrast.

## SUMMARY OF THE INVENTION

It is an object of the present invention to provide photosensitive compounds having good reactivity against deep ultraviolet light near 248.4 nm so as to overcome defects of known lithographic resist compositions.

It is another object of the present invention to provide process for producing the photosensitive compounds.

The present invention provides a photosensitive compound represented by the formula:

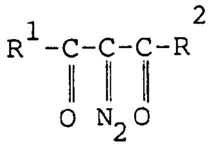

(I)

wherein $R^1$ is

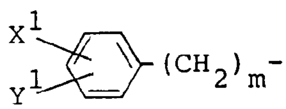 or 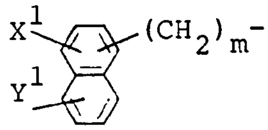 ;

$X^1$ and $Y^1$ are independently a hydrogen atom, a halogen atom, a lower alkyl group having 1 to 5 carbon atoms, a lower alkoxy group having 1 to 5 carbon atoms, $-SO_2Cl$, $-SO_2Br$,

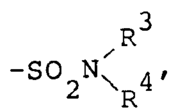

$-SO_3H$ or $-SO_3R^5$; $R^3$ and $R^4$ are independently a hydrogen atom or a lower alkyl group having 1 to 5 carbon atoms, which may have one or more substituents, or $R^3$, $R^4$ and N taken together may form a heterocyclic ring such as a piperazine ring, a piperidine ring, a pyrrolidine ring, a morpholine ring, or the like; $R^5$ is a lower alkyl group having 1 to 5 carbon atoms; the $-SO_2Cl$ or $-SO_2Br$ group may include a quaternary salt thereof; the $-SO_3H$ group may include an ammonium salt thereof, an organic base salt thereof and a quaternary salt thereof; m is an integer of 1 to 20; $R^2$ is an alkyl group, a cycloalkyl group, a hydroxyalkyl group, an alkoxyalkyl group,

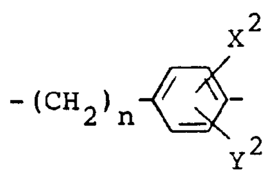

or

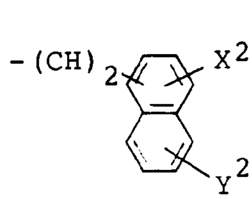

;

$X^2$ and $Y^2$ are independently a hydrogen atom, a halogen atom, a lower alkyl group having 1 to 5 carbon atoms, a lower alkoxy group having 1 to 5 carbon atoms, $-SO_2Cl$, $-SO_2Br$,

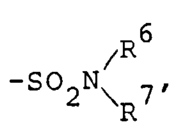

$-SO_3H$ or $-SO_3R^8$; $R^6$ and $R^7$ are independently a hydrogen atom or a lower alkyl group having 1 to 5 carbon atoms which may have one or more substituents, or $R^6$, $R^7$ and N taken together may form a heterocyclic ring

such as a piperazine ring, a piperidine ring, a pyrrolidine ring, a morpholine ring, or the like; $R^8$ is a lower alkyl group having 1 to 5 carbon atoms; the $-SO_2Cl$ or $-SO_2Br$ group may include a quaternary salt thereof; the $-SO_3H$ group may include an ammonium salt thereof, an organic base salt thereof and a quaternary salt thereof; n is an integer of 1 to 20; or $R^1$ and $R^2$ taken together may form a group of the formula:

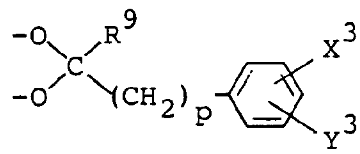

wherein $R^9$ is an alkyl group, an aralkyl group, a hydroxyalkyl group or an alkoxyalkyl group; $X^3$ and $Y^3$ are independently a hydrogen atom, a halogen atom, a lower alkyl group having 1 to 5 carbon atoms, a lower alkoxy group having 1 to 5 carbon atoms, $-SO_2Cl$, $-SO_2Br$,

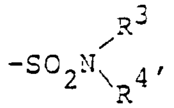

$-SO_3H$ or $-SO_3R^5$; $R^3$, $R^4$ and $R^5$ are as defined above; the $-SO_2Cl$ or $-SO_2Br$ group may include a quaternary salt thereof; the $-SO_3H$ group may include an ammonium salt thereof, an organic base thereof and a quaternary salt thereof; and p is an integer of 1 to 20, provided that $X^3$ and $Y^3$ cannot both together be a hydrogen atom.

The photosensitive compounds of the formula (I) can be produced by various ways.

Figs. 1(a) to (c) are cross-sectional views explaining one example of a pattern orming process using a photosensitive compound of the present invention.

Fig. 2 is a graph showing ultraviolet spectrophotometric characteristics of a photosensitive composition including a photosensitive compound of the present invention at near 248.4 nm.

Fig. 3 is a graph showing ultraviolet spectrophotometric characteristics of MP 2400 at near 248.4 nm.

DESCRIPTION OF THE PREFERRED EMBODIMENTS

The photosensitive compound of the present invention is represented by the formula:

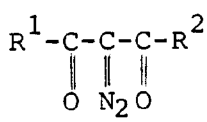

(I)

wherein $R^1$ is

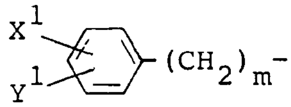 or 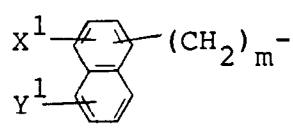 ;

$X^1$ and $Y^1$ are independently a hydrogen atom, a halogen atom, such as Cl, Br, I or F, a lower alkyl group having 1 to 5 carbon atoms, a lower alkoxy group having 1 to 5 carbon atoms, $-SO_2Cl$, $-SO_2Br$,

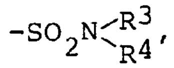

$-SO_3H$, or $-SO_3R^5$; $R^3$ and $R^4$ are independently a hydrogen atom or a lower alkyl group having 1 to 5 carbon

atoms, which may have one or more substituents, or $R^3$, $R^4$ and N taken together may form a heterocyclic ring such as a piperazine ring, a piperidine ring, a pyrrolidine ring, a morpholine ring, or the like; $R^5$ is a lower alkyl group having 1 to 5 carbon atoms; the $-SO_2Cl$ or $-SO_2Br$ group may include a quaternary salt thereof; the $-SO_3H$ group may include an ammonium salt thereof, an organic base salt thereof and a quaternary salt thereof; m is an integer of 1 to 20; $R^2$ is an alkyl group, a cycloalkyl group, a hydroxyalkyl group, an alkoxyalkyl group,

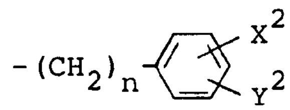 or 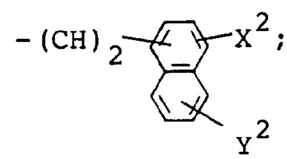;

$X^2$ and $Y^2$ are independently a hydrogen atom, a halogen atom such as Cl, Br, I or F, a lower alkyl group having 1 to 5 carbon atoms, a lower alkoxy group having 1 to 5 carbon atoms, $-SO_2Cl$, $-SO_2Br$,

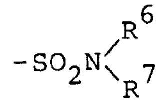,

$-SO_3H$ or $-SO_3R^8$; $R^6$ and $R^7$ are independently a hydrogen atom or a lower alkyl group having 1 to 5 carbon atoms which may have one or more substituents, or $R^6$, $R^7$ and N taken together may form a heterocyclic ring such as a piperazine ring, a piperidine ring, a pyrrolidine ring, a morpholine ring, or the like; $R^8$ is a lower alkyl group having 1 to 5 carbon atoms; the $-SO_2Cl$ or $-SO_2Br$ group may include a quaternary salt thereof, the $-SO_3H$ group may include an ammonium salt thereof, an organic base salt thereof and a quaternary salt thereof; n is an integer of 1 to 20; or $R^1$ and $R^2$ teken together may form a group of the formula:

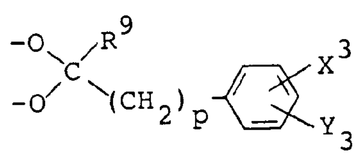

wherein $R^9$ is an alkyl group, an aralkyl group, a hydroxyalkyl group or an alkoxyalkyl group; $X^3$ and $Y^3$ are independently a hydrogen atom, a halogen atom such as Cl, Br, I or F, a lower alkyl group having 1 to 5 carbon atoms, a lower alkoxy group having 1 to 5 carbon atoms, $-SO_2Cl$, $-SO_2Br$,

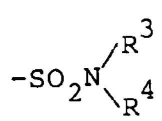,

$-SO_3H$ or $-SO_3R^5$; $R^3$, $R^4$ and $R^5$ are as defined above; the $-SO_2Cl$ or $-SO_2Br$ group may include a quaternary salt thereof; the $-SO_3H$ group may include an ammonium salt thereof, an organic base salt thereof and a quaternary salt thereof; and p is integer of 1 to 20, provided that $X^3$ and $Y^3$ cannot both together be a hydrogen atom.

As the base which can form a quaternary salt with $-SO_2Cl$ or $-SO_2Br$, there can be used pyridine, piperazine, piperidine, N-methylpyrrolidine, morpholine, diethylamine, triethylamine.

As the salt of organic base of $-SO_3H$ and the quaternary salt, there can be used salts of $-SO_3H$ and a base such pyridine, piperzine, piperidine, N-methylpyrrolidine, morpholine, diethylamine, or triethylamine.

The photosensitive compound of the formula (I) concretely includes the following compounds:

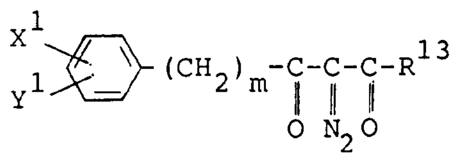

(II)

wherein $X^1$, $Y^1$ and m are as defined above; and $R^{13}$ is an alkyl group, a cycloalkyl group, a hydroxyalkyl group or an alkoxyalkyl group,

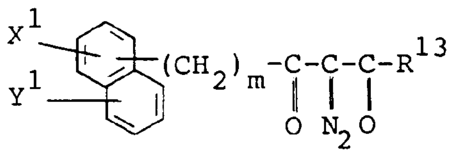

(III)

wherein $X^1$, $Y^1$, $R^{13}$ and m are as defined above,

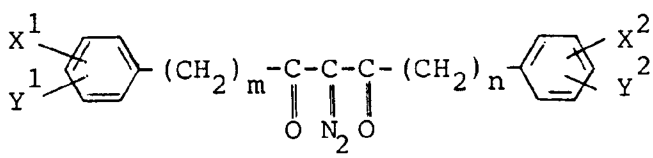

(IV)

wherein $X^1$, $Y^1$, $X^2$, $Y^2$, m and n are as defined above,

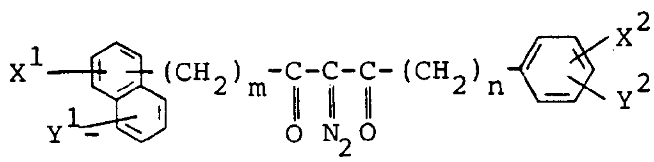

(V)

wherein $X^1$, $Y^1$, $X^2$, $Y^2$, m and n are as defined above,

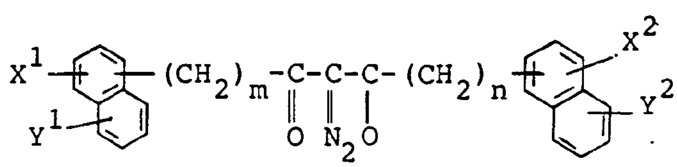

(VI)

wherein $X^1$, $Y^1$, $X^2$, $Y^2$, m and n are as defined above, and

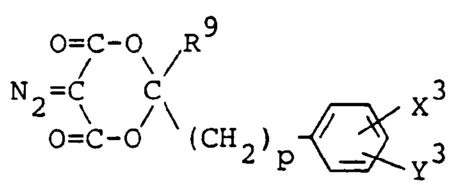

(VII)

wherein $X^3$, $Y^3$, $R^9$ and p are as defined above.

Preferable examples of the compounds of the formula (I) are as follows.

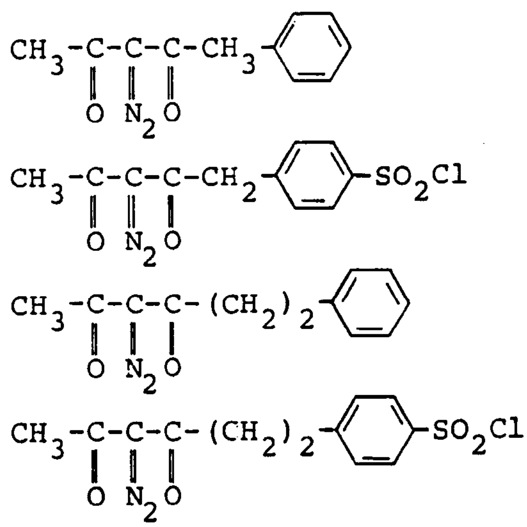

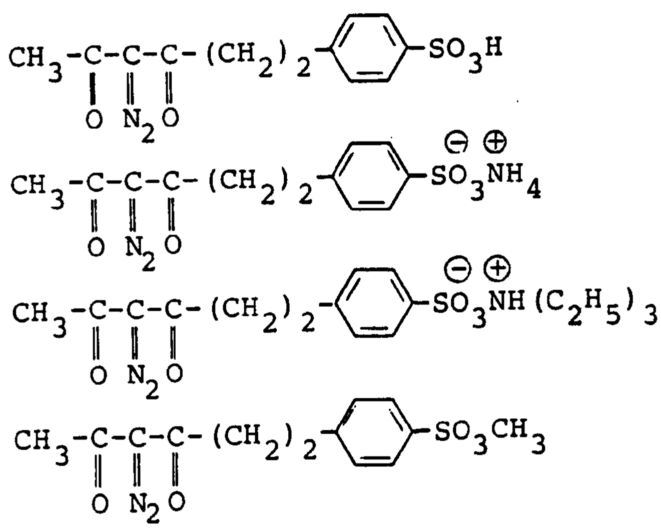

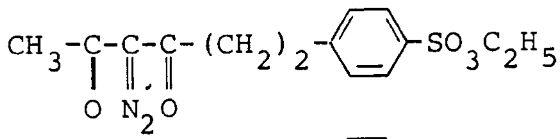

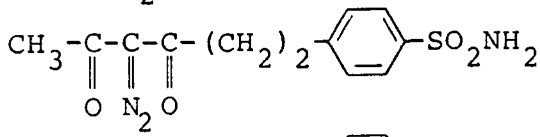

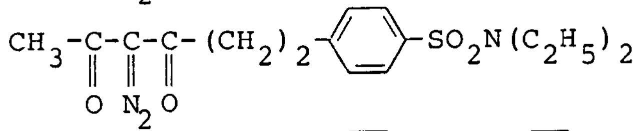

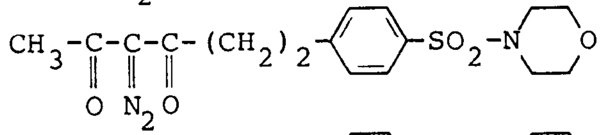

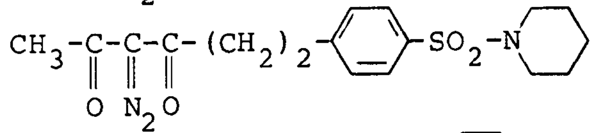

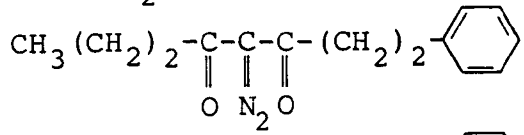

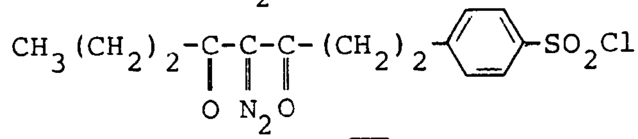

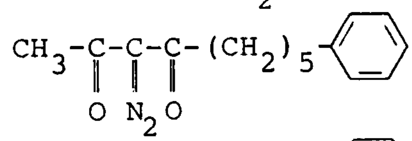

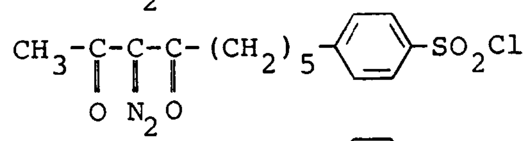

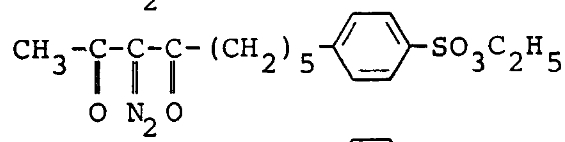

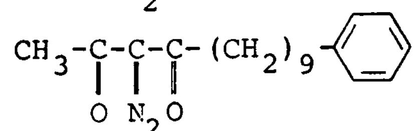

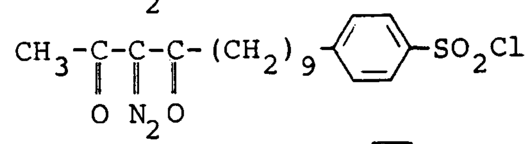

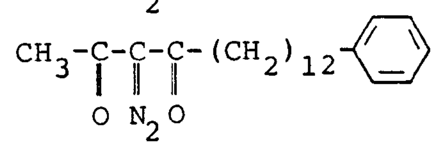

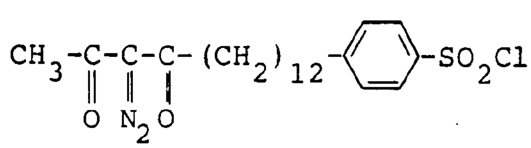

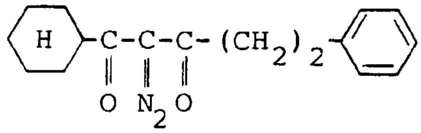

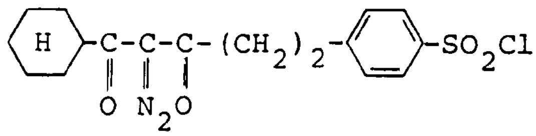

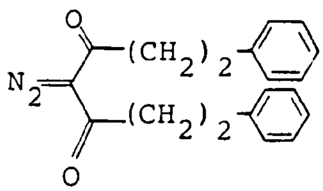

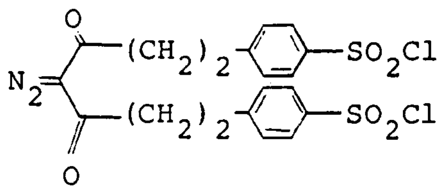

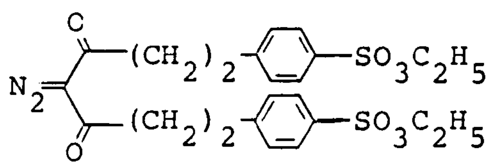

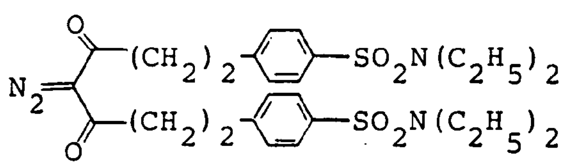

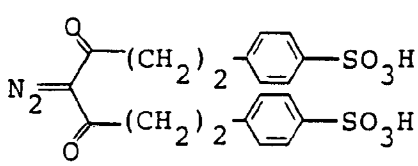

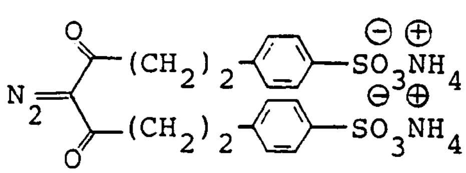

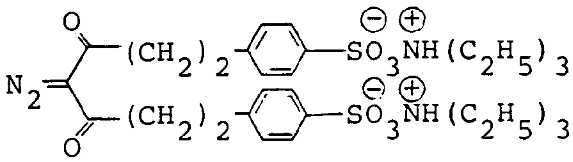

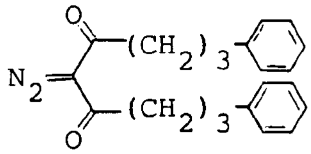

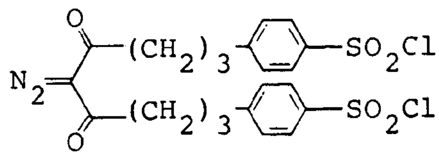

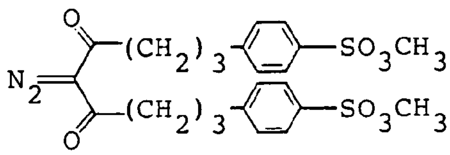

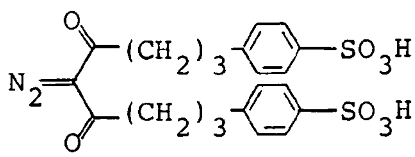

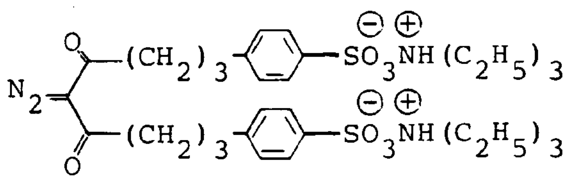

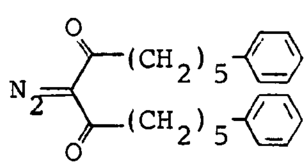

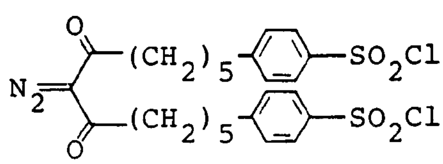

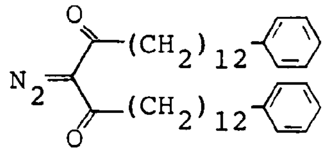

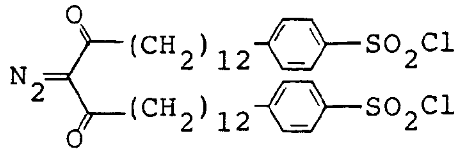

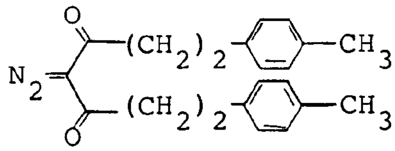

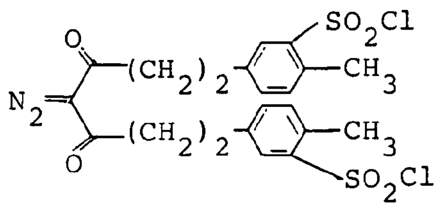

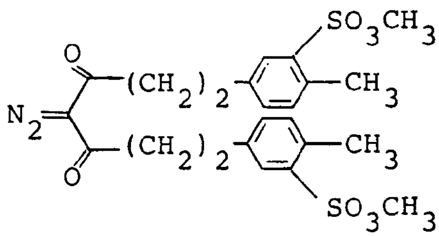

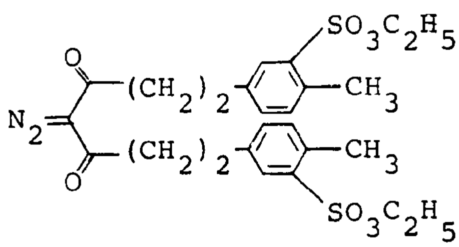

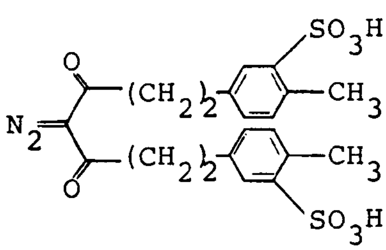

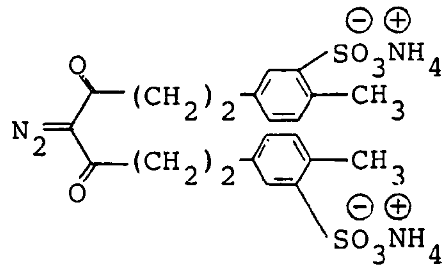

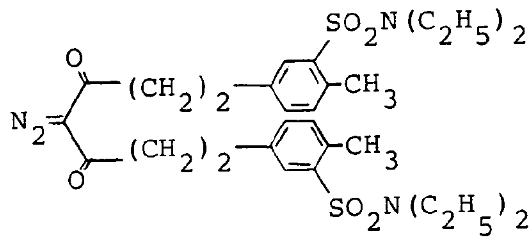

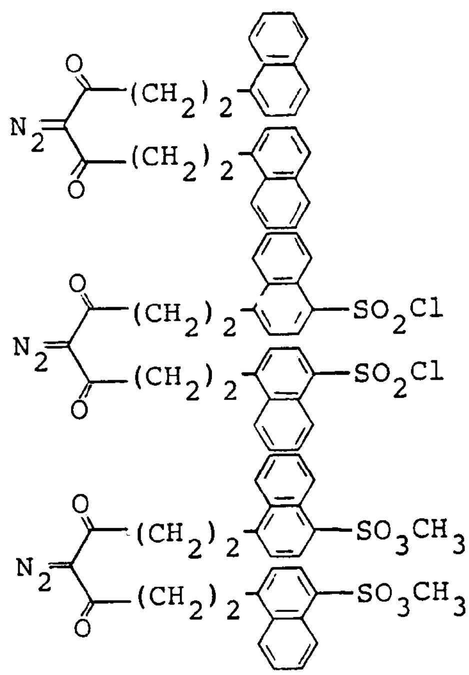

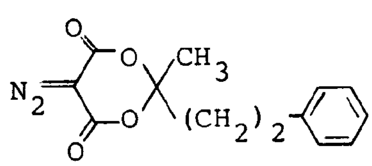

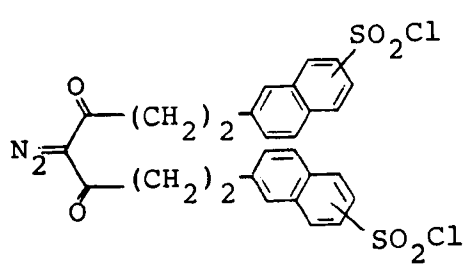

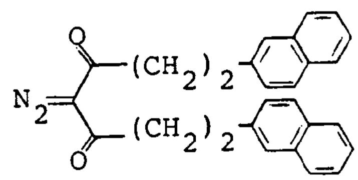

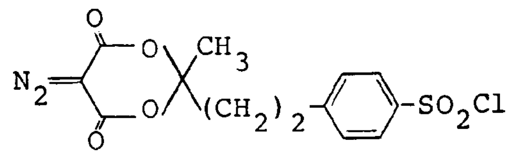

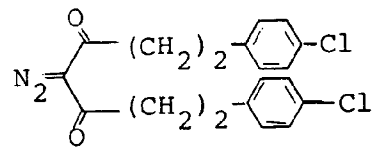

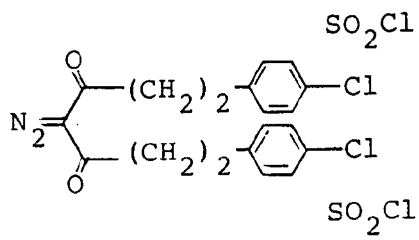

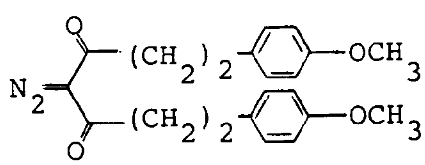

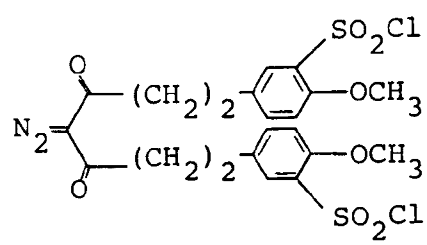

The compounds of the formula (I) can be produced by various ways.

For example, the production of the compound of the formula (II), wherein $X^1$ is a hydrogen atom, a halogen atom, a lower alkyl group or a lower alkoxy group (hereinafter defined as $Z^1$) and $Y^1$ is $-SO_2Cl$, that is, a compound of the formula:

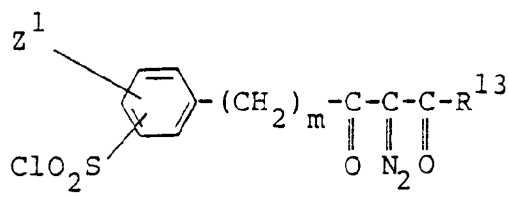

(II')

wherein $Z^1$, $R^{13}$ and m are as defined above, can be produced by reacting a compound of the formula:

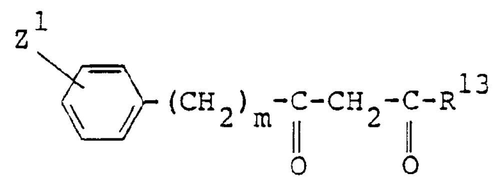

(II-1)

with a diazotizing agent in the presence of a base such as an organic base to form a compound of the formula:

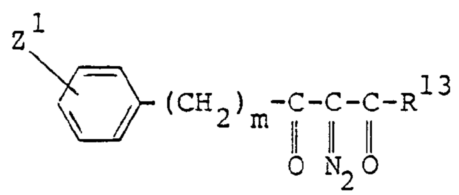

(II-4)

which is then reacted with chlorosulfonic acid.

In the above reactions, the diazotization can be carried out under the following conditions.

As the diazotizing agent, there can be used p-toluenesulfonylazide, benzenesulfonylazide, or 2-azide-3-ethylbenzothiazolium fluoroborate.

As the solvent, there can be used an alcohol such as ethanol, or isopropanol; an ether such as ethyl ether, isopropyl ether, or tetrahydrofuran; a halogenated hydrocarbon such as dichloromethane, chloroform, carbon tetrachloride, or 1,2-dichloroethane; a hydrocarbon such as n-hexane, cyclohexane, or toluene.

As the base, there can be used an organic base such as piperidine, triethylamine, N-methylpyrrolidine, N-methylmorpholine, pyridine, or diethylamine; an alcoholate such as $NaOCH_3$, $NaOC_2H_5$, $KOC(CH_3)_3$, or $KOC_2H_5$; and Na, NaH, or KH.

The diazotizing agent is used in an amount of preferably 0.5 to 3 moles, more preferably 0.8 to 1.5 moles per mole of the compound (II-1). The base is used in an amount of preferably 0.5 to 5 moles, more preferably 0.8 to 1.5 moles per mole of the compound (II-1).

The reaction is carried out preferably at a temperature of -10° to 30°C, more preferably -5° to 10°C, for preferably 15 minutes to 5 hours, more preferably 1 to 2 hours.

The chlorosulfonation can be carried out in a solvent or in the absence of a solvent under the following conditions.

As the solvent, there can be used a chlorinated hydrocarbon such as carbon tetrachloride, tetrachloroethane, chloroform, 1,2-dichloroethane, dichloromethane, etc., carbon disulfide, so as to suppress side reactions.

Chlorosulfonic acid is used in an amount of preferably 1 to 10 moles, more preferably 2.5 to 4.5 moles, per mole of the compound (II-4).

The reaction is carried out preferably at a temperature of -20° to 20°C, more preferably -10° to 5°C, for preferably 15 minutes to 5 hours, more preferably 1 to 2 hours.

The compound of the formula (II') can also be producee by reacting a compound of the formula:

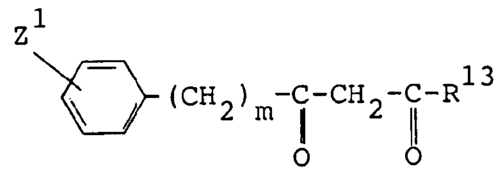

(II-1)

wherein $Z^1$, $R^{13}$ and m are as defined above, with a sulfonating agent, followed by neutralization to yield a compound of the formula:

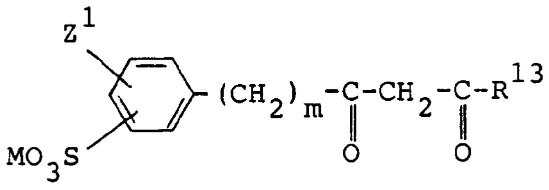

(II-2)

wherein M is an alkali metal atom, an ammonium group or a residue of an organic base; $Z^1$, $R^{13}$ and m are as defined above, reacting the compound of the formula (II-2) with a diazotizing agent in the presence of a base to yield a compound of the formula:

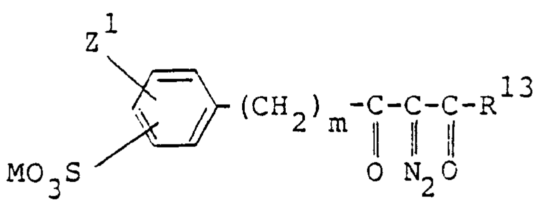

(II-3)

wherein M, $Z^1$, $R^{13}$ and m are as defined above, followed by reaction with a chlorinating agent.

In the above reactions, the sulfonation can be carried out under the following conditions.

As the sulfonating agent, there can be used chlorosulfonic acid, sulfuric acid, fuming sulfuricacid, anhydrous sulfuric acid (sulfur trioxide), a complex of dioxane and anhydrous sulfuric acid, a complex of pyridine and anhydrous sulfuric acid, etc.

As a solvent, in the case of using sulfuric acid, there can be used glacial acetic acid, acetic anhydride, ethyl acetate, ethyl ether, acetonitrile, or carbon tetrachloride; and in the case of using other sulfonating agents, there can be used carbon disulfide, 1,2-dichloroethane, tetrachloroethane, chloroform, carbon tetrachloride, dichloromethane, nitromethane, or nitrobenzene.

The sulfonating agent is used in an amount of preferably 1 to 10 moles, more preferably 1 to 3 moles, per mole of the compound (II-1).

The reaction can be carried out preferably at -10° to 30°C, more preferably -5° to 5°C, preferably in the presence of a Ta powder as a catalyst in the case of using chlorosulfonic acid, or preferably at 0° to 100°C, more preferably at 0° to 10°C during the dropwise addition of a sulfonating agent such as sulfonic acid, fuming sulfuric acid, anhydrous sulfuric acid or a complex, and finally raised to 50° to 100°C. After the dropwise addition of sulfonating agent, the reaction is preferably carried out for 1 to 3 hours.

The neutralization is carried out by using a neutralizing agent (for inserting M), for example, a potassium salt such as KCl, $K_2CO_3$, KOH, KOR (R is an alkyl group), or $KHCO_3$ - ; a sodium salt such as NaCl, $Na_2CO_3$, NaOH, NaOR (R is an alkyl group), or $NaHCO_3$; an ammonium salt such as $NH_4Cl$, $(NH_4)_2CO_3$, $NH_4HCO_3$, or $NH_3$ ; an organic base such as $(C_2H_5)_3N$, or $(C_2H_5)_2NH$.

The diazotization can be carried out under the following conditions.

As the diazotizing agent, there can be used p-toluenesulfonylazide, benzenesulfonylazide, or 2-azido-3-ethylbenzothiazolium fluoroborate.

As the solvent, there can be used an alcohol such as ethanol, or isopropanol; an ether such as ethyl ether, isopropyl ether, or tetrahydrofuran; a halogenated hydrocarbon such as dichloromethane, chloroform, or carbon tetrachloride; a hydrocarbon such as n-hexane, or cyclohexane; an amide such as N,N-dimethylformamide (DMF), formamide, or hexamethylphosphoramide (HMPA).

As the base, there can be used an organic base such as triethylamine, or piperidine; an alcoholate such

as $NaOCH_3$ or $NaOC_2H_5$.

The diazotizing agent is used in an amount of preferably 0.5 to 3 moles, more preferably 0.8 to 1.5 moles, per mole of the compound (II-2). The base is used in an amount of preferably 0.5 to 5 moles, more preferably 0.8 to 1.5 moles, per mole of the compound (II-2).

The reaction can be carried out at preferably -10° to 30°C, more preferably -5° to 10°C, for preferably 15 minutes to 5 hours, more preferably 1 to 2 hours.

The chlorination can be carried out under the following conditions.

As the chlorinating agent, there can be used thionyl chloride, phosgene (dimer), phosphorus pentachloride, phosphoryl chloride, chlorine, or chlorosulfonic acid.

As the solvent, there can be used DMF, formamide, chloroform, dichloromethane, carbon tetrachloride, or 1,2-dichloroethane.

The chlorinating agent is used in an amount of preferably 1 to 10 moles, more preferably 1 to 3 moles, per mole of the compound (II-3).

The reaction can be carried out preferably at 0° to 100°C, more preferably 0° to 25°C.

When the compound of the formula:

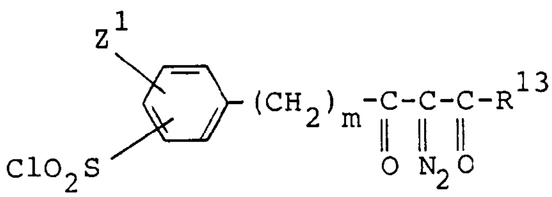

(II')

is subjected to hydrolysis, there can be obtained a compound of the formula:

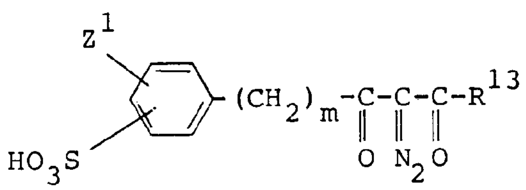

(II-5)

wherein $Z^1$, $R^{13}$ and m are as defined above.

When the compound of the formula (II') is subjected to alcoholysis in place of hydrolysis, there can be obtained a compound of the formula:

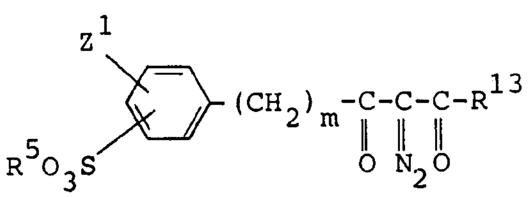

(II-6)

wherein $Z^1$, $R^5$, $R^{13}$ and m are as defined above.

When the compound of the formula (II') is reacted with ammonia, an aliphatic amine, or an organic base, there can be obtained a compound of the formula:

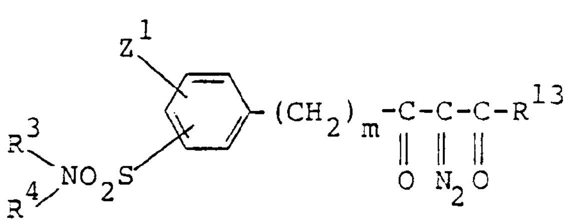

(II-7)

wherein $R^3$, $R^4$, $R^{13}$, $Z^1$ and m are as defined above.

Examples of the aliphatic amine are monomethylamine, monoethylamine, monopropylamine, monobutylamine, monopentylamine, dimethylamine, diethylamine, dipropylamine, monoethanolamine, diethanolamine, etc.

Examples of the organic base are piperidine, piperazine, pyrrolidine, or morpholine.

The compound of the formula (II-1) can be prepared by reacting an ester of the formula:

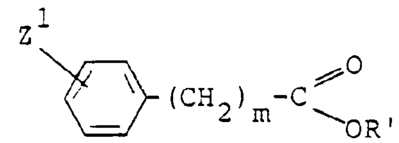

wherein $Z^1$ and m are as defined above; and R' is an alkyl group, with a ketone of the formula:

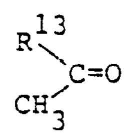

wherein $R^{13}$ is as defined above, in the presence of a condensing agent and preferably in a solvent.

As the condensing agent, there can be used Na, NaOR" (R" is an alkyl group), $NaNH_2$, KOR", $KNH_2$, or $(C_6H_5)_3CNa$

As the solvent, there can be used an ether such as isopropyl ether, or tetrahydrofuran; the ester per se, the ketone per se, or a hydrocarbon.

Alternatively, the compound of the formula (II-1) can be produced by reacting a ketone of the formula:

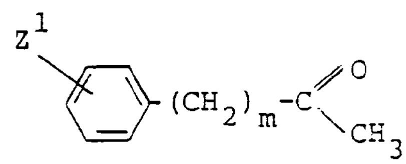

wherein $Z^1$ and m are as defined above, with an ester of the formula:

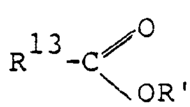

wherein $R^{13}$ and R′ are as defined above, in the presence of a condensing agent, preferably in a solvent.

As the condensing agent and the solvent, those mentioned above can also be used.

The compound of the formula (III), wherein $X^1$ is a hydrogen atom, a halogen atom, a lower alkyl group or a lower alkoxy group (= $Z^1$) and $Y^1$ is $-SO_2Cl$, that is, the compound of the formula:

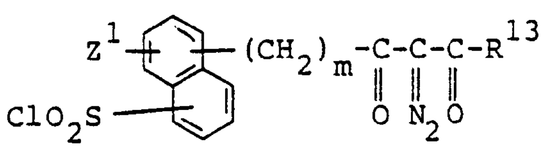

(III')

wherein $Z^1$, $R^{13}$ and m are as defined above, can be produced by reacting a compound of the formula:

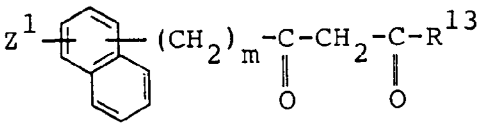

(III-1)

wherein $Z^1$, $R^{13}$ and m are as defined above with a diazotizing agent in the presence of a base to yield a compound of the formula:

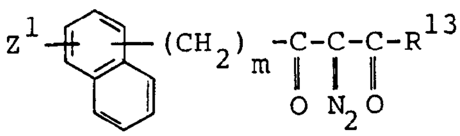

(III-4)

wherein $Z^1$, $R^{13}$ and m are as defined above, followed by a reaction with chlorosulfonic acid.

The diazotization and the chlorosulfonation can be carried out in the same manner as described in the case of the compound of the formula (II').

The compound of the formula (III') can also be produced by reacting a compound of the formula:

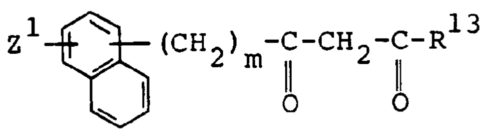

(III-1)

wherein $Z^1$, $R^{13}$ and m are as defined above, with a sulfonating agent, followed by neutralization to yield a compound of the formula:

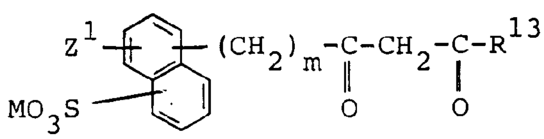

(III-2)

wherein $Z^1$, $R^{13}$, M and m are as defined above, reacting the compound of the formula (III-2) with a diazotizing agent in the present of a base to yield a compound of the formula:

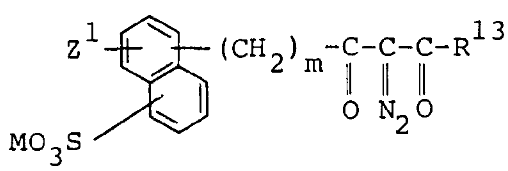

(III-3)

wherein M, $Z^1$, $R^{13}$ and m are as defined above, followed by reaction with a chlorinating agent.

The sulfonation, neutralization, diazotization and chlorination can be carried out in the same manner as described in the case of the compound of the formula (II').

The hydrolysis, the alcoholysis, and the reaction with ammonia, an aliphatic amine or an organic base of the compound of the formula (III'), can be carried out in the same manner as described in the case of the compound of the formula (II') to yield the following compounds:

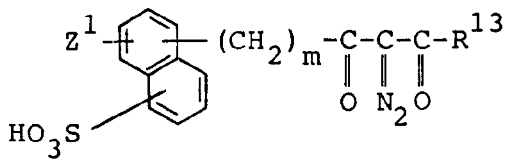

(III-5)

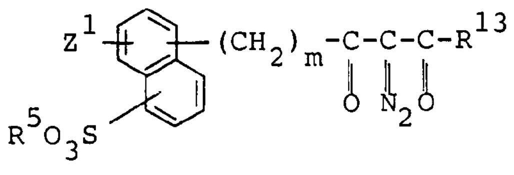

(III-6)

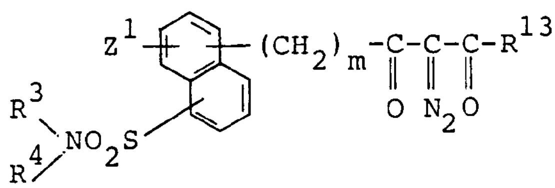

(III-7)

wherein $Z^1$, $R^3$, $R^4$, $R^5$, $R^{13}$ and m are as defined above.

In the formulae (III'), and (III-1) to (III-7), $Z^1$, $-SO_3M$, $-SO_2Cl$, $-SO_3H$, $-SO_3R^5$ and

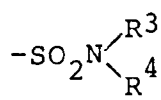

can be attached to any position of the naphthalene ring, that is, when the alkylene group is attached to the 1-position, $Z^1$, $-SO_3M$, $-SO_2Cl$, $-SO_3H$, $-SO_3R^5$ and

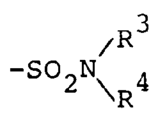

can be attached to any positions of 2- to 8-positions, or when the alkylene group is attached to the 2-position, $Z^1$, $-SO_3M$, $-SO_2Cl$, $-SO_3H$, $-SO_3R^5$ and

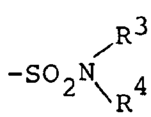

can be attached to any positions of 1-position and 3- to 8-positions.

The compound of the formula (III-1) can be prepared in the same manner as described in the case of the compound of the formula (II-1).

The compound of the formula (IV), wherein $X^1$ is a hydrogen atom, a halogen atom, a lower alkyl group or a lower alkoxy group (= $Z^1$), $X^2$ is a hydrogen atom, a halogen atom, a lower alkyl group or a lower alkoxy group (= $Z^2$), $Y^1$ is $-SO_2Cl$ and $Y^2$ is $-SO_2Cl$, that is, a compound of the formula:

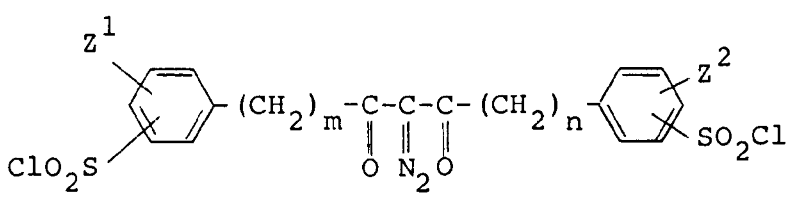

(IV')

wherein $Z^1$, $Z^2$, m and n are as defined above, can be produced by reacting a compound of the formula:

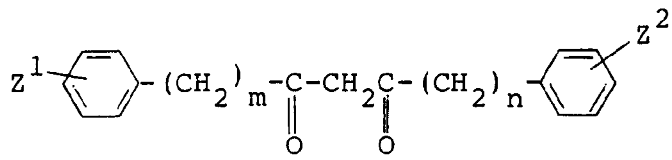

(IV-1)

wherein $Z^1$, $Z^2$, m and n are as defined above, with a diazotizing agent in the presence of an organic base to yield a compound of the formula:

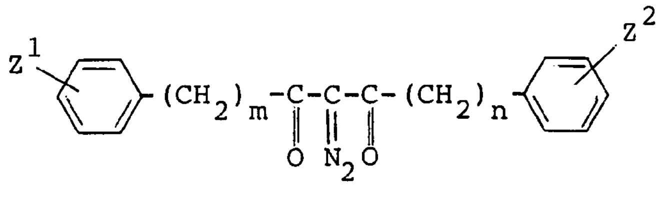

(IV-2)

wherein $Z^1$, $Z^2$, m and n are as defined above, followed by a reaction with chlorosulfonic acid.

The diazotization and the chlorosulfonation can be carried out in the same manner as described in the case of the compound of the formula (II'). In the chlorosulfonation, one of

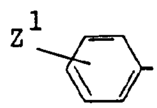

and

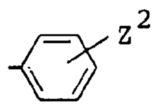

may be retained without attaching $-SO_2Cl$.

The compound of the formula (IV') can also be produced by reacting a compound of the formula:

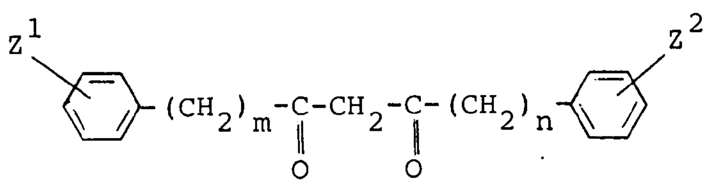

(IV-1)

wherein $Z^1$, $Z^2$, m and n are as defined above, with a sulfonating agent, followed by neutralization to yield a compound of the formula:

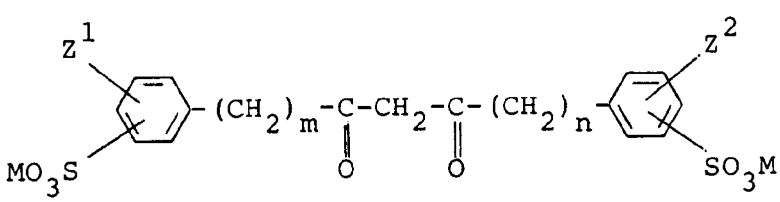

(IV-2)

wherein $Z^1$, $Z^2$, M, m and n are as defined above, reacting the compound of the formula(IV-2) with a diazotiting agent in the presence of an organic base to yield a compound of the formula:

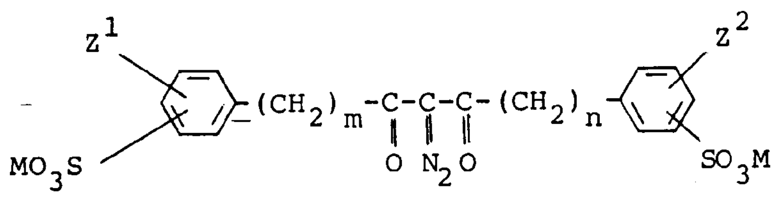

(IV-3)

wherein $Z^1$, $Z^2$, M, m and n are as defined above, followed by reaction with a chlorinating agent.

The sulfonation, neutralization, diazotization and chlorination can be carried out in the same manner as described in the case of the compound of the formula (II'). In the above-mentioned reactions, one of

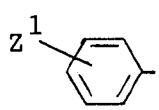 and 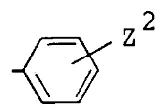

may be retained without attaching $-SO_2Cl$.

The hydrolysis, the alcoholysis, and the reaction with ammonia, an aliphatic amine or an organic base, of the compound of the formula (IV') can be carried out in the same manner as described in the case of the compound of the formula (II') to yield the following compounds:

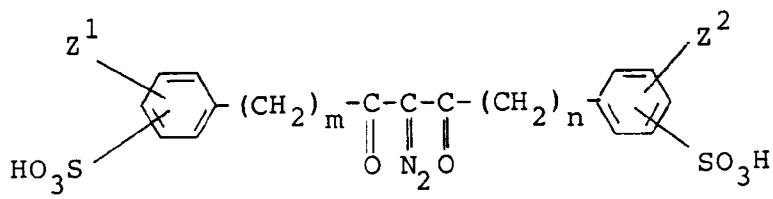

(IV-5)

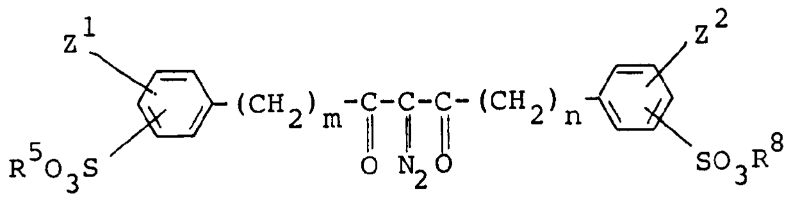

(IV-6)

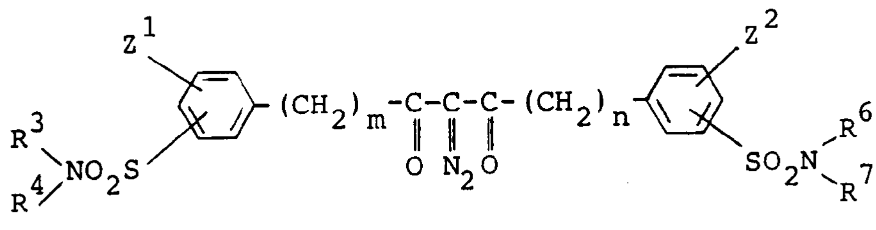

(IV-7)

wherein $Z^1$, $Z^2$, $R^3$. $R^4$, $R^5$, $R^6$, $R^7$, $R^8$, m and n are as defined above. In the above formulae (IV-5) to (IV-7), the following combinations may be possible;

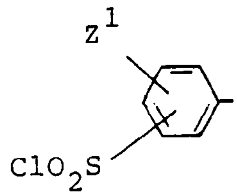 and 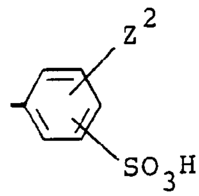

and vice versa,

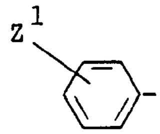 and 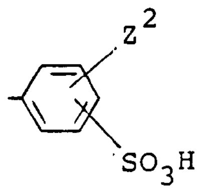

and vice versa;

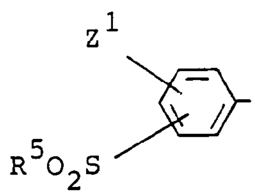

and

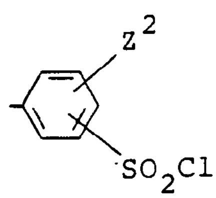

and vice versa,

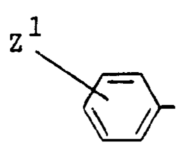

and

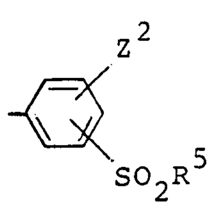

and vice versa;

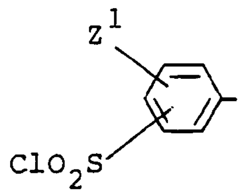

and

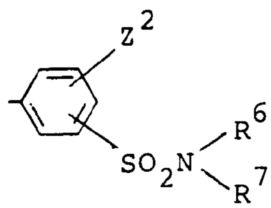

and vice versa,

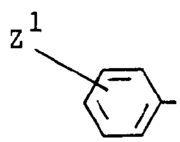

and

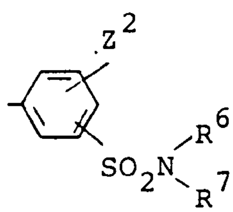

and vice versa.

The compound of the formula (IV-1) can be prepared in the same manner as described in the case of the compound of the formula (II-1). More concretely, a compound of the formula:

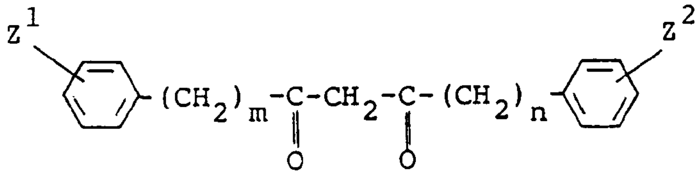

(IV-1)

can be prepared by reacting compounds of the formula:

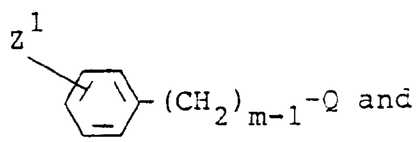

and

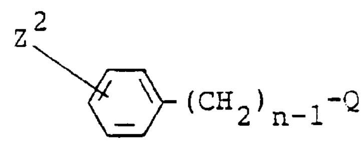

wherein $Z^1$, $Z^2$, m and n are as defined above; and Q is chlorine, bromine or iodine with 2,4-pentanedione in the presence of a condensing agent and a solvent at 10°C or lower. As the condensing agent, there can be used n-butyllithium, lithium diisopropylamide, lithium, 1,1,1,3,3,3-hexamethyldisilazane, KH/n-BuLi, NaH/n- or BuLi (Bu = butyl). As the solvent, there can be used cyclohexane, n-hexane, toluene, or isopropyl ether.

The compound of the formula (IV-1) can also be prepared by reacting a ketone of the formula:

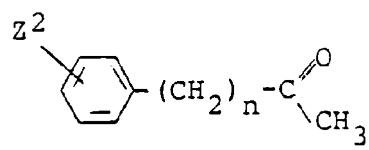

wherein $Z^2$ and m are as defined above, with an ester of the formula:

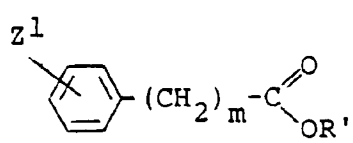

wherein R′ is an alkyl group; and $Z^1$ and m are as defined above, in the presence of Na, NaH, or a metal alkoxide to carry out a condensation reaction.

The compound of the forlula (V), wherein $X^1$ is a hydrogen atom, a halogen atom, a lower alkyl group or a lower alkoxy group (= $Z^1$); $X^2$ is a hydrogen atom, a halogen atom, a lower alkyl group or a lower alkoxy group (= $Z^2$ ; $Y^1$ is $-SO_2Cl$; and $Y^2$ is $-SO_2Cl$, that is, the compound of the formula:

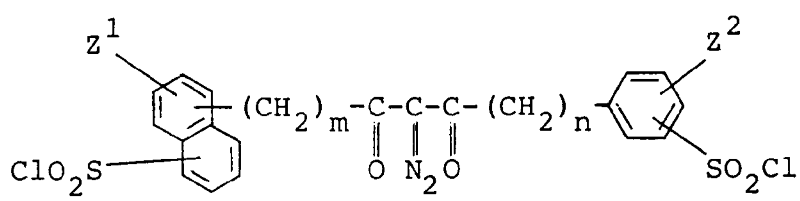

(V')

wherein $Z^1$, $Z^2$, m and n are as defined above, can be produced by reacting a compound of the formula:

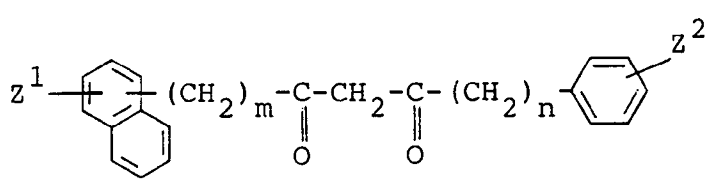

(V-1)

wherein $Z^1$, $Z^2$, m and n are as defined above, with a diazotizing agent in the presence of an organic base to yield a compound of the formula:

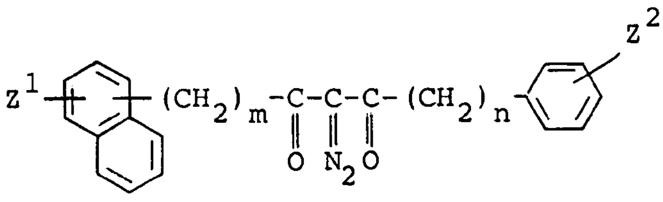

(V-4)

wherein $Z^1$, $Z^2$, m and n are as defined above, followed by a reaction with chlorosulfonic acid.

The diazotization and the chlorosulnation can be carried out in the same manner as described in the case of the compound of the formula (II').

The compound of the formula (V') can also be produced by reacting a compound of the formula:

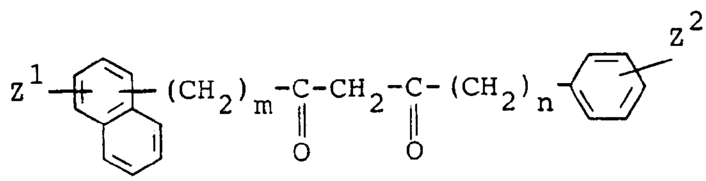

(V-1)

wherein $Z^1$, $Z^2$ , m and n are as defined above, with a sulfonating agent, followed by neutralization to yield a compound of the formula:

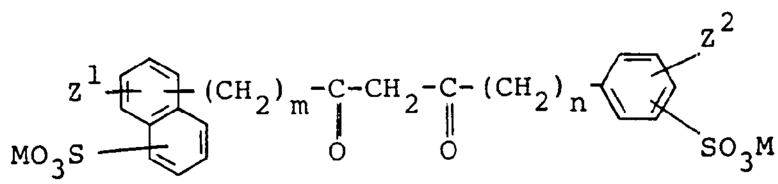

(V-2)

wherein $Z^1$, $Z^2$, M, m, and n are as defined above, reacting the compound of the formula (V-2) with a diazotizing agent in the presence of an organic base to yield a compound of the formula:

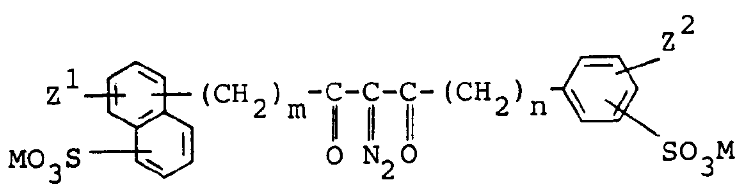

(V-3)

wherein $Z^1$, $Z^2$, M, m and n are as defined above, followed by reaction with a chlorinating agent.

The sulfonation, neutralization, diazotization and chlorination can be carried out in the same manner as described in the case of the compound of the formula (II').

The hydrolysis, the alcoholysis, and the reaction with ammonia, an aliphatic amine or an organic base, of the compound of the formula (V') can be carried out in the same manner as described in the case of the compound of the formula (II') to yield the following compounds:

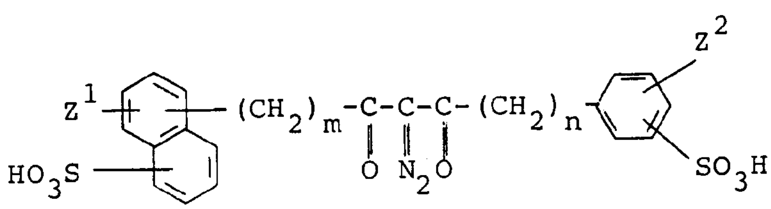

(V-5)

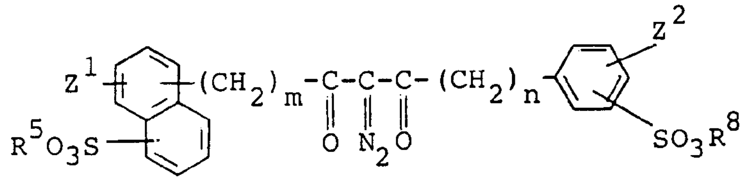
(V-6)

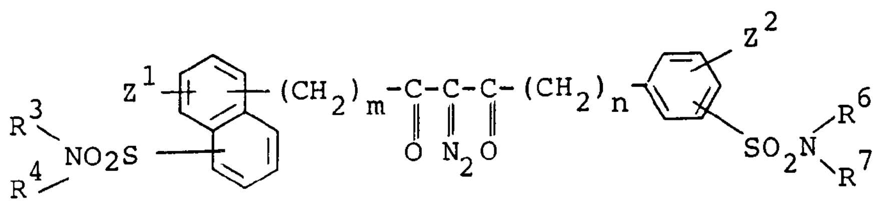
(V-7)

wherein $R^3$, $R^4$, $R^5$, $R^6$, $R^7$, $R^8$, $Z^1$, $Z^2$, m and n are as defined above.

In the formulae (V′) and (V-1) to (V-7) $Z^1$, $-SO_3M$, $-SO_2Cl$, $-SO_3H$, $-SO_3R^5$, and

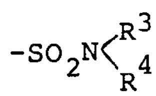

can be attached to any positions of the naphthalene ring, that is, when the alkylene group is attached to the 1-position, $Z^1$, $-SO_3M$, $-SO_2Cl$, $-SO_3H$, $-SO_3R^5$, and

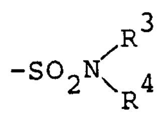

can be attached to any positons of 2- to 8-positions, or when the alkylene group is attached to the 2-position, $Z^1$ and the like substituents can be attached to any positions of 1-position and 3- to 8-positions.

The compound of the formula (V-1) can be prepared in the same manner as described in the case of the compound of the formula (IV-1).

The compound of the formula (VI), wherein $X^1$ is a hydrogen atom, a halogen atom, a lower alkyl group or a lower alkoxy group (= $Z^1$); $X^2$ is a hydrogen atom, a halogen atom, a lower alkyl group or a lower alkoxy group (= $Z^2$); $Y^1$ is $-SO_2Cl$; and $Y^2$ is $-SO_2Cl$, that is, the compound of the formula:

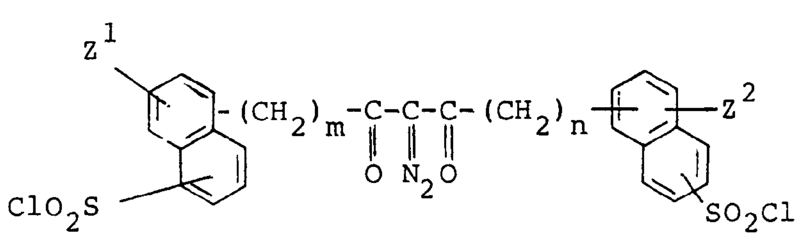
(VI')

wherein $Z^1$, $Z^2$, m and n are as defined above, can be produced by reacting a compound of the formula:

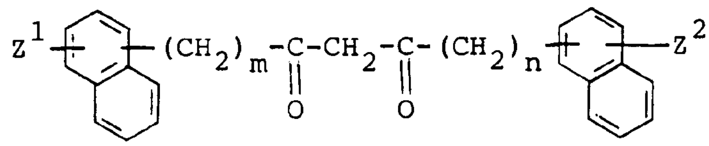
(VI-1)

wherein $Z^1$, $Z^2$, m and n are as defined above, with a diazotizing agent in the presence of an organic base to

yield a compound of the formula:

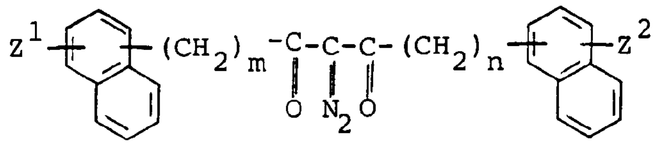

(VI-4)

wherein $Z^1$, $Z^2$, m and n are as defined above, followed by a reaction with chlorosulfonic acid.

The diazotization and the chlorosulfonation can be carried out in the same manner as described in the case of the compound of the formula (II').

The compound of the formula (VI') can also be produced by reacting a compound of the formula:

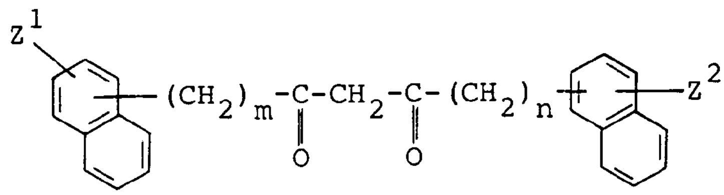

(VI-1)

wherein $Z^1$, $Z^2$, m and n are as defined above, with a sulfonating agent, followed by neutralization to yield a compound of the formula:

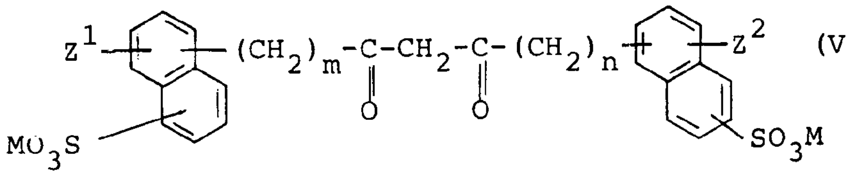

(VI-2)

wherein $Z^1$, $Z^2$, M, m and n are as defined above, reacting the compound of the formula (VI-2) with a diazotizing agent in the presence of a base to yield a compound of the formula:

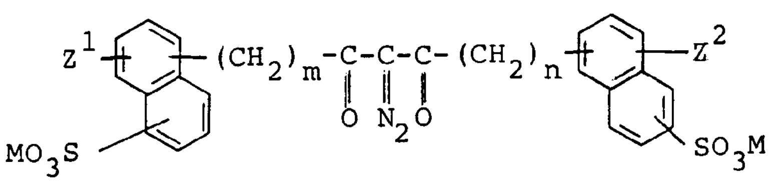

(VI-3)

wherein M, $Z^1$, $Z^2$, m and n are as defined above, followed by reaction with a chlorinating agent.

The sulfonation, neutralization, diazotization and chlorination can be carried out in the same manner as described in the case of the compound of the formula (II').

The hydrolysis, the alcoholysis, and the reaction with ammonia, an aliphatic amine or an organic base, of the compound of the formula (VI') can be carried out in the same manner as described in the case of the compound of the formula (II') to yield the following compounds:

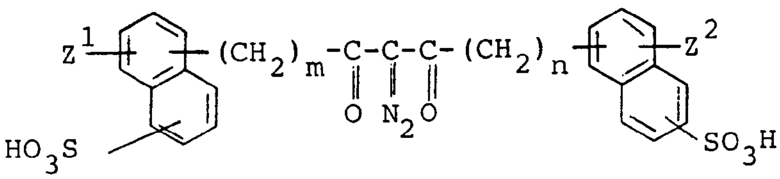

(VI-5)

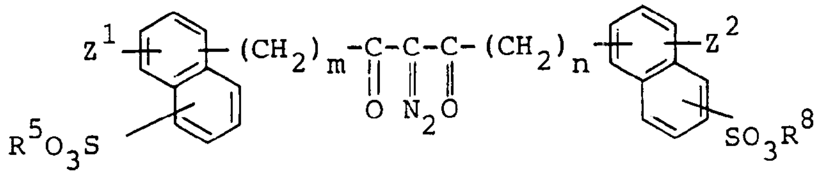

(VI-6)

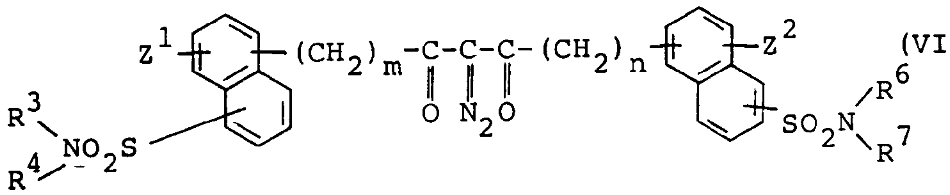

(VI-7)

wherein $Z^1$, $Z^2$, $R^3$, $R^4$, $R^5$, $R^6$, $R^7$, $R^8$, m and n are as defined above.

In the formulae (VI') and (VI-1) to (VI-7), $Z^1$, $Z^2$, $-SO_3M$, $-SO_2Cl$, $-SO_3H$, $-SO_3R^5$, $-SO_3R^8$,

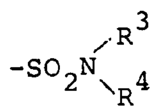 and 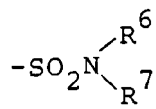

can be attached to any positions of the naphthalene ring, that is, when the alkylene group is attached to the 1-position, $Z^1$ and the like substituents can be attached to any positions of 2- to 8-positions, or when the alkylene group is attached to the 2-position, $Z^1$ and the like substituents can be attached to any positions of 1-position and 3- to 8-positions.

The compound of the formula (VI-1) can be prepared in the same manner as described in the case of the compound of the formula (IV-1).

The compound of the formula (VII), wherein $X^3$ is a hydrogen atom, a halogen atom, a lower alkyl group or a lower alkoxy group (= $Z^1$) and $Y^3$ is $-SO_2Cl$, that is, the compound of the formula:

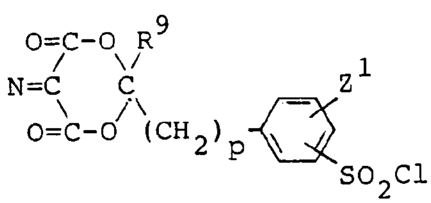

(VII')

wherein $Z^1$, $R^9$ and p are as defined above, can be produced by reacting a compound of the formula:

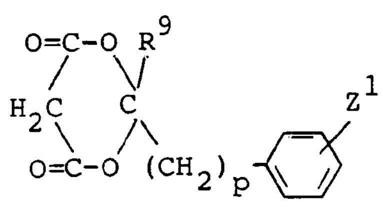

(VII-1)

wherein $Z^1$, $R^9$ and p are as defined above, with a diazotizing agent in the presence of an organic base to yield a compound of the formula:

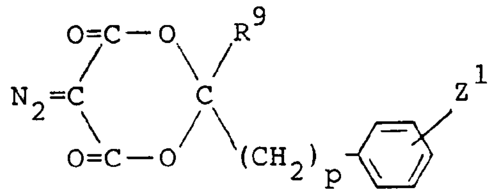

(VII-4)

wherein $Z^1$, $R^9$ and p are as defined above, followed by a reaction with chlorosulfonic acid.

The diazotization and the chlorosulfonatoin can be carried out in the same manner as described in the case of the compound of the formula (II').

The compound of the formula (VII') can also be produced by reacting a compound of the formula:

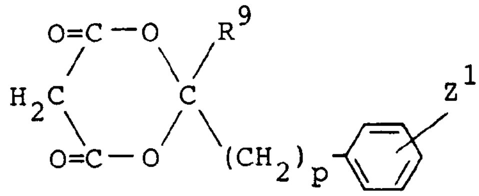

(VII-1)

wherein $Z^1$, $R^9$ and p are as defined above, with a sulfonating agent, followed by neutralization to yield a compound of the formula:

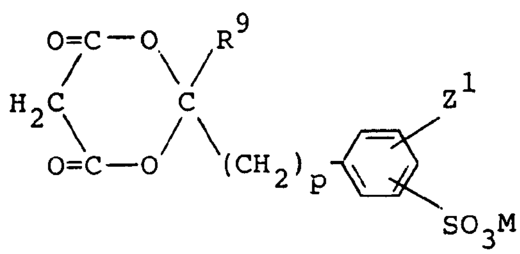

(VII-2)

wherein $Z^1$, $R^9$, M, and p are as defined above, reacting the compound of the formula (VII-2) with a diazotizing agent in the presence of an organic base to yield a compound of the formula:

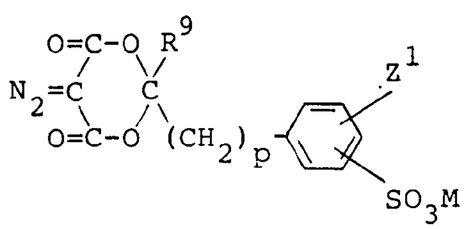

(VII-3)

wherein M, $Z^1$, $R^9$ and p are as defined above, followed by reaction with a chlorinating agent.

The sulfonation, neutralization, diazotization and chlorination can be carried out in the same manner as described in the case of the compound of the formula (II').

The hydrolysis, the alcoholysis, and the reaction with ammonia, an aliphatic amine or an organic base, of the compound of the formula (VII') can be carried out in the same manner as described in the case of the compound of the formula (II') to yield the following compounds:

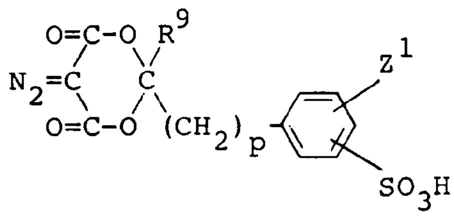

(VII-5)

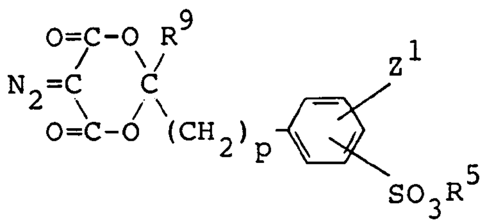

(VII-6)

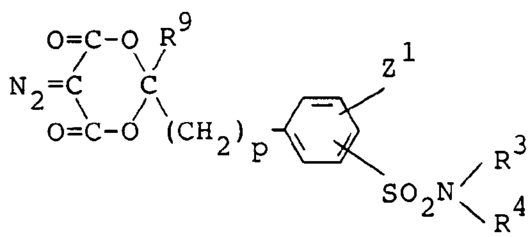

(VII-7)

wherein $R^3$, $R^4$, $R^5$ $R^9$, $Z^1$ and p are as defined above.

The compound of the formula (VII-1) can be prepared in the same manner as described in the case of the compound of the formula (II-1). More concretely, a compound of the formula:

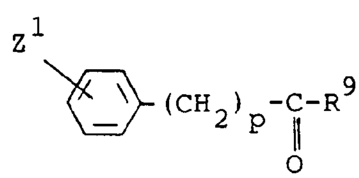

wherein $Z^1$, $R^9$ and p are as defined above, is reacted with malonic acid in the presence of acetic anhydride and sulfuric acid to form a compound of the formula:

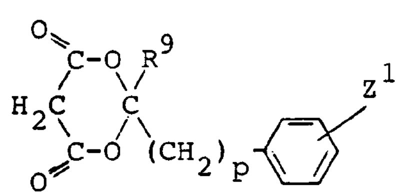

(VII-1)

The photosensitive compound of the formula (I) has a great reactivity against a light of 248.4 nm, that is, has a large change in light transmittance before and after exposure to the light (about 50% or more) and shows a transmittance of 65% or more after exposure to the light. Further, in a photosensitive composition comprising a compound of the formula (I) having a functional group such as $-SO_2Cl$, $-SO_3H$ or the like reactive with a resin as at least one of $X^1$, $X^2$, $X^3$, $Y^1$, $Y^2$ and $Y^3$, and a resin having OH groups, when heated after coating of the photosensitive composition, a crosslinking reaction proceeds, which results in producing an effect for decreasing a solubility of the resin in a developing solution. Thus, by using such a photosensitive composition as a resist or the like material, there can be obtained a resin pattern having a good (non-eroded) shape.

That is, the group of the formula:

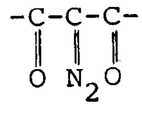

in the compound of the formula (I) is changed to a group of the formula:

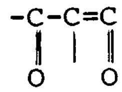

(VIII)

by deep UV light such as KrF excimer laser light, or the like. By the action of an alkaline aqueous solutoin, the group of the formula (VIII) changes as follows:

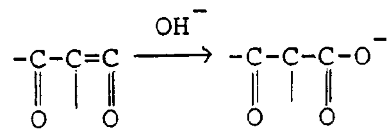

Thus, the photosensitive composition containing the photosensitive compound of the formula (I) becomes alkali-soluble in only light-exposed portions to form a so-called positive-type resist.

Generally speaking, a positive-type resist material is a combination of a photosensitive substance, a resin and a solvent. As the resin, an alkali-soluble novolac resin is mainly used. Therefore, it is desirable that alkali solubility of non-light exposed portions of novolac resin is suppressed.

In the compound of the formula [I] having one or more functional groups reactive with the resin as at least one of $X^1$, $X^2$, $X^3$, $Y^1$, $Y^2$ and $Y^3$, since there are one or more functional groups such as $-SO_2Cl$, $-SO_2Br$ or $-SO_3H$, at terminal groups, crosslinking proceeds with heating by forming, for example, ester bonds such as

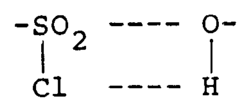

with -OH groups which are alkali-soluble portions of the resin, which results in suppressing alkali-solubility of the resin. Therefore, when the photosensitive composition of the present invention is used, the problem of erosion of retaining portions of resist film on the unexposed portions can be improved greatly.

Further, since the photosensitive compound of the formula (I) has one or two methylene chains such as $-(CH_2)_m-$ in the molecule, the molecule as a whole is stabilized, which results in improving thermal stability of the compound of the formula (I). This also makes it possible to produce the compound of the formula (I) in indistrially large amounts and to store the compound of the formula (I) stably for a long period of time.

In the compound of the formula (I), the position of functional group such as $-SO_2Cl$,

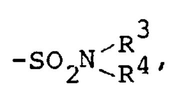

$-SO_3H$, $-SO_3R^5$, or the like can be any one of o-, m- and p-position of a benzene ring with regard to the methylene group, or can be any one of 2- to 8-positions when the methylene group is attached to the 1-position of a naphthalene ring or any one of 1-position and 3-to 8-positions when the methylene group is attached to the 2-position of the naphthalene ring. In any cases, the effect as a photosensitizer is not influenced considerably. As to the length of the methylene group, the effect is not so influenced when m, n and p are in the range of 1 to 20. Generally speaking, when the methylene group chain becomes longer, there is a tendency to attain the same effect with a smaller amount and the solubility of the photosensitive compound of the formula (I) per se in a developing solution is lowered. From this point of view, the longer the methylene group chain becomes, the better the results become.

The photosensitive compound of the formula (I) can be used not only in the production of semiconductor

devices but also as a photosensitive reagent in the production of photoengraved plates, printing materials and the like applying the light reaction.

The present invention is illustrated by way of the following Examples, in which all percents are by weight unless otherwise specified.

Example 1

Synthesis of p-toluenesulfonyl azide

Sodium azide (22.5 g, 0.35 mol) was dissolved in $H_2O$(65 ml) and diluted with 90% aqueous ethanol (130 ml). To this mixture, a solution of p-toluenesulfonyl chloride (60 g, 0.32 mol) in ethanol (300 ml) was added dropwise at 10-25°C and stirring was continued fo 2.5 hours at room temperature. The reaction mixture was evaporated under reduced pressure, the resultant residue was washed with $H_2O$ for several times, dried over anhydrous $MgSO_4$ and filtered with suction to give the title compound as a colorless oil; yield: 50.7 g.

'HNMR δppm ($CDCl_3$): 2.43 (3H, s, C<u>H</u>$_3$), 7.24 (2H, d, J=8Hz, Ar3-H, 5-H), 7.67 (2H, d, J=8Hz, Ar 2-H, 6-H).

IR (Neat) $\nu cm^{-1}$: 2120.

Example 2

Synthesis of 1-(4-chlorosulfonylphenyl)-3-diazo-2,4-pentanedione

(Compound [II]; $R^{13}$=-$CH_3$, $X^1$=-$SO_2Cl$, $Y^1$=H, m=1)

(1) Synthesis of 1-phenyl-2,4-pentanedione

Sodium (28 g) was added in small portions to a solution of ethyl phenyl acetate (600 g, 3.66 moles) and acetone (70.7 g, 1.22 moles) under nitrogen, was dissolved at 30-40°C with stirring for 1 h and then the mixture was reacted at 70-80°C for 4.5 h. The reaction mixture was taken up in $H_2O$ (1.2 ℓ), neutralized with dilute hydrochloric acid and extracted with chloroform. The organic layer was washed with $H_2O$, dried over anhydrous $MgSO_4$ and then evaporated. The residue was distilled under reduced pressure to give the title compound as a colorless oil; yield: 53.2 g; bp 142-146°C/15 mmHg. (Lit. bp 133-136°C/10 mmHg; K.G. Hampton, T.M. Harris, C.R. Hauser, Org. Synth., <u>51</u>, 128 (1971)).

(2) Synthesis of 3-diazo-1-phenyl-2,4-pentadione

To a solution of 1-phenyl-2,4-pentadione (36.2 g, 0.21 mol) obtained in above (1) and piperidine (17.5 g, 0.21 mol) in dichloromethane (500 ml), p-toluenesulfonyl azide (44.5 g, 0.23 mol) obtained in Example 1 was added dropwise at 0-5°C, and stirring was continued for 1 h at the same temperature. The reaction mixture was washed with dilute aqueous potassium hydroxide and $H_2O$, dried over anhydrous $MgSO_4$ and evaporated in vacuo. The resultant residue (50 g) was chromatographed on silica gel (Wako Gel C-200, manufactured by Wako Pure Chemical Industries, LTd.) with n-hexane/ethyl acetate (10:1) as eluent to give the title compound as a orange-red viscous oil; yield: 33.0 g.

$^1$HNMR δppm ($CDCl_3$): 2.36 (3H, S, C<u>H</u>$_3$), 4.00 (2H, s, -C<u>H</u>$_2$-), 7.23 (5H, s, ArH).

IR (Neat) $\nu cm^{-1}$ : 2140, 1660.

(3) Synthesis of 1-(4-chlorosulfonylphenyl)-3-diazo-2,4-pentanedione

(Compound [II]; $R^{13}$=-$CH_3$, $X^1$=-$SO_2Cl$, $Y^1$=H, m=1)

To a solution of 3-diazo-1-phenyl-2,4-pentanedione (16.5 g, 0.082 mol) obtained in above (2) in chloroform (50 ml), chlorosulfonic acid (38 g) was added dropwise at -15~-10°C. The mixture was stirred for 2 h at -15~-10°C and for 3 h at 10-15°C, then poured into cold $H_2O$ (1.5 ℓ), and extracted with chloroform. The organic extract was washed with $H_2O$, dried over anhydrous $MgSO_4$, and then evaporated under reduced pressure. The resultant residue (20 g) was chromatographed on silica gel (Wako Gel C-200, manufactured by Wako Pure Chemical Industries, Ltd.) with n-hexane/ethyl acetate (5:1) as eluent to give the title compound as a yellow prisms after recrystallization from ethyl ether; yield: 4.0 g; mp 84.0-86.5°C (dec).

$^1$HNMR δppm ($CDCl_3$): 2.45 (3H, s, C<u>H</u>$_3$), 4.30 (2H, s, -C<u>H</u>$_2$-), 7.54 (2H, d, J=8Hz, Ar2-H, 6-H), 8.00 (2H,

d, J=8Hz, Ar3-H, 5-H).

IR (KBr) νcm$^{-1}$: 2125, 1660.

UV ($CH_3CN$) $\lambda_{max}$ nm (logε): 244.2 (4.40).

Anal. calcd. for $C_{11}H_9ClN_2O_4S$: C%, 43.94; H%, 3.02; N%, 9.32.

Found: C%, 44.02; H%, 3.14; N%, 9.30.

Example 3

Synthesis of 6-(4-chlorosulfonylphenyl)-3-diazo-2,4-hexanedione

(Compound [II]; $R^{13}$=-$CH_3$, $X^1$=-$SO_2Cl$, $Y^1$=H, m=2)

(1) Synthesis of 6-phenyl-2,4-hexanedione

Sodium (23 g) was added in small portions to a solution of 4-phenyl-2-butanone (148 g, 1 mol) and ethyl acetate (264 g) at 20-60°C with stirring under nitrogen and then the reaction mixture was refluxed for 2 h with stirring. After cooling, the mixture was taken up in $H_2O$ (1ℓ), neutralized with hydrochloric acid, the organic layer was separated, washed with $H_2O$, dried over anhydrous $Na_2SO_4$ and then evaporated. The resultant residue (171 g) was distilled under reduced pressure to give the title compound as a colorless oil; yield: 73.4 g; bp 128-133°C/3-4 mmHg (Lit. bp 133-136°C/5 mmHg; C.R. Hauser, T.M. Harris, J.Am. Chem. Soc., 80, 6360 (1958)).

(2) Synthesis of 3-diazo-6-phenyl-2,4-hexanedione

Using 6-phenyl-2,4-hexanedione (29.0 g, 0.15 mol) obtained in above (1), the reaction was carried out in the same manner as described in Example 2, (2) to give the title compound as a orange-red amorphous solid; yield: 27.0 g; mp 30.0-32.0°C

$^1$HNMR δppm ($CDCl_3$): 2.47 (3H, s, C$\underline{H_3}$), 3.10 (4H, s, -C$\underline{H_2}$-C$\underline{H_2}$-), 7.28 (5H, s, ArH).

IR (Neat) νcm$^{-1}$: 2100, 1650

UV ($CH_3CN$) $\lambda_{max}$ nm (log ε): 229.3 (4.27)

(3) Synthesis of 6-(4-chlorosulfonylphenyl)-3-diazo-2,4-hexanedione

(Compound [II]; $R^{13}$=-$CH_3$, $X^1$=-$SO_2Cl$, $Y^1$=H, m=2)

Using 3-diazo-6-phenyl-2,4-hexanedione (10.8 g, 0.05 mol) obtained in above (2), the reaction was carried out in the same manner as described in Example 2, (3) to give the title compound as a pale yellow amorphous solid; yield: 3.0 g; mp 58.0-59.5°C.

$^1$HNMR δppm ($CDCl_3$): 2.40 (3H, s, C$\underline{H_3}$), 3.20 (4H, s, -C$\underline{H_2}$-C$\underline{H_2}$-CO-), 7.32 (2H, dd, J=8Hz, Ar2-H, 6-H), 7.97 (2H, dd, J=8Hz, Ar3-H, 5-H).

IR (Neat) νcm$^{-1}$: 2130, 1660

UV ($CH_3CN$) $\lambda_{max}$ nm (log ε): 244.2 (4.36)

Anal. calcd. for $C_{12}$ $H_{11}ClN_2O_4S$: C%, 45.65; H%, 3.51; N%, 8.87.

Found: C%, 45.69; H%, 3.79; N%, 8.68.

Example 4

Synthesis of 4-(4-diazo-3,5-dioxohexyl)benzenesulfonic acid

(Compound [II]; $R^{13}$=-$CH_3$, $X^1$=-$SO_3H$, $Y^1$=H, m=2)

6-(4-Chlorosulfonylphenyl)-3-diazo-2,4-hexanedione (2.0 g; 6.4 mmol) obtained in Example 3, (3) was stirred in a solution of acetonitrile (15 ml) and $H_2O$ (45 ml) for 2 h at 30-35°C. The reaction mixture was washed with dichloromethane (40 ml x 2) and then the aqueous layer was evaporated to dryness under reduced pressure to give the title compound as a slightly yellow viscous oil; yield: 1.4 g.

$^1$HNMR δppm ($CDCl_3$-DMSO-$d_6$): 2.37 (3H, s, C$\underline{H_3}$), 2.88 (2H, t, J=7Hz, -C$\underline{H_2}$-CO-), 3.07 (2H, t, J=7Hz, -C$\underline{H_2}$-C$\underline{H_2}$ CO-), 7.21 (2H, d, J=8.5Hz, Ar3-H, 5-H), 7.58 (2H, d, J=8.5Hz, Ar2-H, 6-H), 10.53 (1H, bs, -$SO_3\underline{H}$).

IR (Neat) νcm$^{-1}$: 3370, 2120, 1630.

UV ($CH_3CN$) $\lambda_{max}$ nm (log ε): 226.4 (4.46).
Anal. calcd. for $C_{12}H_{12}N_2O_5S$: C%, 48.64; H%, 4.08; N%, 9.45.
Found: C%, 48.75; H%, 4.11; N%, 9.29.

Example 5

Synthesis of ammonium 4-(4-diazo-3,5-dioxohexyl) benzenesulfonate

(Compound [II], $R^{13}$=-$CH_3$, $X^1$=-$SO_3^{\ominus}N^{\oplus}H_4$, $Y^1$=H, m=2)

To a solution of 4-(4-diazo-3,5-dioxohexyl) benzenesulfonic acid (890 g, 30 mmol) obtained in Example 4 in acetonitrile (5 ml), 28% aqueous ammonia (3 ml) was added dropwise at 5-10°C, and stirring was continued for 2 h at the same temperature. Then the mixture was evaporated under reduced pressure, and the residue was crystallized on treatment with acetonitrile to give the title compound as a white amorphous solid; yield: 750 mg; mp 81°C (dec.).

$^1$HNMR δppm ($CDCl_3$-DMSO-$d_6$): 2.40 (3H, s, -CO-C$\underline{H_3}$), 2.93 (1H, t, J=8Hz, -C$\underline{H_2}$-CO-), 3.06 (2H, t, 8Hz, -C$\underline{H_2}$$CH_2$-CO-), 3.34 (4H, bs, -$N^{\oplus}\underline{H_4}$), 7.20 (2H, d, J=8Hz, Ar3-H, 5-H), 7.70 (2H, d, J=8Hz, Ar2-H, 6-H).
IR (KBr) $\nu cm^{-1}$: 3430, 2120, 1640.
UV ($H_2O$)$\lambda_{max}$ nm (log ε): 225.0 (4.36).

Example 6

Synthesis of triethylammonium 4-(4-diazo-3,5-dioxohexyl) benzenesulfonate

(Compound [II]; $R^{13}$=-$CH_3$, $X^1$=-$SO_3^{\ominus}N^{\oplus}H(C_2H_5)_3$, $Y^1$=H, m=2)

To a solution of 4-(4-diazo-3,5-dioxohexyl)benzenesulfonic acid (890 mg, 30 mmol) obtained in Example 4 in acetonitrile (5 ml), triethylamine (3 ml) was added dropwise at 5-10°C, and stirring was continued for 2 h at the same temperature. Then the mixture was evaporated under reduced pressure, the residue was washed thrice with ethyl ether and evaporated to dryness under reduced pressure to give the title compound as a slightly yellow viscous oil; yield: 700 mg.

$^1$HNMR δppm ($CDCl_3$): 1.36 (9H, t, J=7Hz, N-$CH_2CH_3$ x 3), 2.42 (3H, s, -CO-C$\underline{H_3}$), 2.96-3.06 (4H, m, -C$\underline{H_2}$C$\underline{H_2}$-), 3.16 (6H, q, J=7Hz, N-C$\underline{H_2}$$CH_3$ x 3), 7.24 (2H, d, J=8Hz, Ar3-H, 5-H), 7.81 (2H, d, J=8Hz, Ar2-H, 6-H).
IR (Neat ) $\nu cm^{-1}$: 3430, 2120, 1650.
UV ($CH_3CN$) $\lambda_{max}$ nm (log ε): 226.4 (4.37).
Anal. calcd. for $C_{18}H_{27}N_3O_5S$: C%, 54.39; H% 6.85; N%, 10.57
Found: C%, 54.11; H%, 7.14; N%, 10.36.

Example 7

Synthesis of methyl 4-(4-diazo-3,5-dioxohexyl)benzenesulfonate

(Compound [II]; $R^{13}$=-$CH_3$, $X^1$=-$SO_3CH_3$, $Y^1$=H, m=2)

6-(4-Chlorosulfonylphenyl)-3-diazo-2,4-hexanedione (2.0 g, 6.4 mmol) obtained in Example 3, (3) was stirred in triethylamine (1.5 ml), methanol (2.5 ml) and chloroform (8 ml) for 6 h at 10-15°C, then the mixture was poured into $H_2O$ and extracted thrice with chloroform. The organic extract was washed with $H_2O$, dried over anhydrous $MgSO_4$ and evaporated under reduced pressure. The residue was chromatographed on silica gel (Wako Gel C-200, manufactured by Wako Pure Chemical Industries, Ltd.) with chloroform as eluent to give the title compound as a pale yellow viscous oil; yield: 730 mg.

$^1$HNMR δppm ($CDCl_3$): 2.42 (3H, s, C$\underline{H_3}$), 3.03-3.18 (4H, m, -C$\underline{H_2}$C$\underline{H_2}$-), 3.76 (3H, s, $SO_3$C$\underline{H_3}$), 7.43 (2H, d, J=8.5Hz, Ar3-H, 5-H), 7.83 (2H, d, J=8.5Hz, Ar2-H, 6-H).
IR (Neat) $\nu cm^{-1}$: 2100, 1650.
UV ($CH_3CN$) $\lambda_{max}$ nm (log ε): 227.8 (4.48).
Anal. calcd. for $C_{13}H_{14}N_2O_5S$: C%, 50.32; H%, 4.55; N%, 9.03.
Found: C%, 50.25, H%, 4.61; N%, 8.97.

Example 8

Synthesis of ethyl 4-(4-diazo-3,5-dioxohexyl)benzenesulfonate

(Compound [II], $R^{13}$=$-CH_3$, $X^1$=$-SO_3C_2H_5$, $Y^1$=H, m=2)

6-(4-Chlorosulfonylphenyl)-3-diazo-2,4-hexanedione (2.0 g, 6.4 mmol) obtained in Example 3, (3) was stirred in triethylamine (1.5 ml) and ethanol (5 ml) for 16 h at room temperature, then the mixture was poured into $H_2O$ and extracted thrice with chloroform. The organic extract was washed with $H_2O$, dried over anhydrous $MgSO_4$ and evaporated under reduced pressure. The residue was chromatographed on silica gel (Wako Gel C-200) with chloroform as eluent to give the title compound as a pale yellow viscous oil; yield: 550 mg.

$^1$HNMR δppm ($CDCl_3$): 1.31 (3H, t, J=7Hz, $-SO_2CH_2C\underline{H}_3$), 2.42 (3H, s, $-COC\underline{H}_3$), 3.04-3.17 (4H, m, Ar-$C\underline{H}_2C\underline{H}_2$CO-), 4.11 (2H, q, J=7Hz, $-SO_3C\underline{H}_2CH_3$), 7.42 (2H, d, J=8.5 Hz, Ar3-H, 5-H), 7.82 (2H, d, J=8.5 Hz, Ar2=H, 6-H).

IR (Neat) $\nu cm^{-1}$: 2120, 1645.

UV ($CH_3CN$) $\lambda_{max}$ nm (log ε): 229.0 (4.46).

Anal. calcd. for $C_{14}H_{16}N_2O_5S$: C%, 51.84; H%, 4.97; N%, 8.64.

Found: C%, 51.77; H%, 4.85; N%, 8.73.

Example 9

Synthesis of 6-(4-aminosulfonylphenyl)-3-diazo-2,4-hexanedione

(Compound [II]; $R^{13}$=$-CH_3$, $X^1$=$-SO_2NH_2$, $Y^1$=H, m=2)

To a solution of 6-(4-chlorosulfonylphenyl)-3-diazo-2,4-hexanedione (1.0 g, 3.2 mmol) obtained in Example 3, (3) in acetonitrile (5 ml), 28% aqueous ammonia (2 g) was added dropwise at 5-10°C, and stirring was continued for 6 h at room temperature. Then the mixture was evaporated under reduced pressure and the residue was chromatographed on silica gel (Wako Gel C-200; manufactured by Wako Pure Chemical Industries, Ltd.) with n-hexane/ethyl acetate (from 5:1 to 2:1) as eluent to give the title compound as a white amorphous solid; yield: 800 mg; mp 109.5-111.5°C.

$^1$HNMR δppm ($CDCl_3$-DMSO-$d_6$): 2.41 (3H, s, $COC\underline{H}_3$), 3.02-3.13 (4H, m, $-C\underline{H}_2C\underline{H}_2-$), 6.42 (2H, bs, $-N\underline{H}_2$), 7.35 (2H, d, J=8Hz, phenyl-$C_2$, $C_6$), 7.83 (2H, d, J=8Hz, phenyl-C3, $C_5$).

IR (KBr) $\nu cm^{-1}$: 2120, 1625.

UV ($CH_3CN$) $\lambda_{max}$ nm (log ε): 228.8 (4.47)

Anal. calcd. for $C_{12}H_{13}N_3O_4S$: C%, 48.80; H%, 4.44; N%, 14.23.

Found C%, 48.69; H%, 4.39; N%, 14.41

Example 10

Synthesis of 6-(4-N,N-diethylaminosulfonylphenyl)-3-diazo-2,4-hexanedione

(Compound [II]; $R^{13}$=$-CH^3$, $X^1$=$-SO_2N(C_2H_5)_2$ $Y^1$=H, m=2)

Using 6-(4-chlorosulfonylphenyl)-3-diazo-2,4-hexanedione (1.0 g, 3.2 mmol) obtained in Example 3, (3) and diethylamine (0.6 g), the reaction was carried out in the same manner as described in Example 9 to give the title compound as a slightly yellow viscous oil; yield; 770 mg.

$^1$HNMR δppm ($CDCl_3$): 1.13 (6H, t, J=7Hz, N-$CH_2C\underline{H}_3$x 2), 2.42 (3H, s, $-COC\underline{H}_3$), 3.00-3.15 (4H, m, $-C\underline{H}_2C\underline{H}_2$CO-), 3.23 (4H, q, J=7Hz, N-$C\underline{H}_2CH_3$x 2), 7.35 (2H, d, J=8.5Hz, Ar3-H, 5-H), 7.72 (2H, d, J=8.5 Hz, Ar2-H, 6-H).

IR (Neat $\nu cm^{-1}$: 2110, 1640.

UV ($CH_3CN$) $\lambda_{max}$ nm (log ε): 232.5 (4.28)

Anal. calcd. for $C_{16}H_{21}N_3O_4S$: C%, 54.69; H%, 6.02; N%, 11.96

Found: C%, 54.60; H%, 5.86: N%, 12.11

Example 11

Synthesis of 6-(4-morpholinosulfonylphenyl)-3-diazo-2,4-hexanedione

(Compound [II]; $R^{13}$=-$CH_3$,

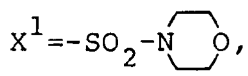

$Y^1$=H, m=2)

Using 6-(4-chlorosulfonylphenyl)-3-diazo-2,4-hexanedione (1.0 g, 3.2 mmol) obtained in Example 3, (3) and morpholine (0.83 g), the reaction was carried out in the same manner as described in Example 9 to give the title compound as a slightly yellow viscous oil; yield: 800 mg.

$^1$HNMR δppm ($CDCl_3$): 2.42 (3H, s, -$COCH_3$), 2.99 (4H, t, J=5Hz, morpholine 2-H, 2-H, 6-H, 6-H), 3.04-3.16 (4H, m, -$CH_2CH_2$CO-), 3.74 (4H, t, J=5Hz, morpholine 3-H, 3-H, 5-H, 5-H), 7.43 (2H, d, J=8Hz, Ar3-H, 5-H), 7.67 (2H, d, J=8Hz, Ar2-H, 6-H).

IR (Neat) $\nu cm^{-1}$: 2130, 1645.

UV ($CH_3CN$) $\lambda_{max}$ nm (log ε): 230.9 (4.47).

Anal. calcd. for $C_{16}H_{19}N_4O_5S$: C%, 52.59; H%, 5.24; N%, 11.50.

Found: C%, 52.42; H%, 5.30; N%, 11.66.

Example 12

Synthesis of 6-(4-piperidinosulfonylphenyl)-3-diazo-2,4-hexanedione

(Compound [II]; $R^{13}$=-$CH_3$,

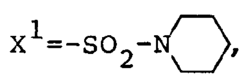

$Y^1$=H, m=2)

Using 6-(4-chlorosulfonylphenyl)-3-diazo-2,4-hexanedione (1.0 g, 3.2 mmol) obtained in Example 3, (3) and piperidine (0.65 g), the reaction was carried out in the same manner as described in Example 9 to give the title compound as a slightly yellow viscous oil; yield: 730 mg.

$^1$HNMR δppm ($CDCl_3$): 1.37-1.51 (2H, m, piperidine 4-H, 4-H), 1.60-1.74 (4H, m, piperidine 3-H, 3-H, 5-H, 5-H), 2.42 (3H, s, -$COCH_3$), 2.96-3.18 (8H, m, -$CH_2CH_2$CO- and piperidine 2-H, 2-H, 6-H, 6-H), 7.38 (2H, d, J=8Hz, Ar3-H, 5-H), 7.67 (2H, d, J=8Hz, Ar2=H, 6-H).

IR (Neat) $\nu cm^{-1}$: 2130, 1640.

UV ($CH_3CN$) $\lambda_{max}$ nm (log ε): 231.2 (4.46)

Anal. calcd. for $C_{17}H_{21}N_3O_4S$: C%, 56.18; H%, 5.82; N%, 11.56.

Found: C%, 56.01; H%, 6.10; N%, 11.49.

Example 13

Synthesis of 9-(4-chlorosulfonylphenyl)-3-diazo-2,4-nonanedione

(Compound [II]; $R^{13}$=-$CH_3$, $X^1$=-$SO_2Cl$, $Y^1$=H, m=5)

(1) Synthesis of 4-phenylbutanol

To a suspension of lithium aluminum hydride (67.2 g) in dry tetrahydrofuran (2.5 ℓ), a solution of 4-phenylbutyric acid (225 g, 1.37 mmoles) in dry tetrahydrofuran was added dropwise at 15-25°C under nitrogen. The resultant mixture was stirred for 1 h at room temperature, then poured into ice-cold $H_2O$ (3 ℓ) and sulfuric acid (200 g) and extracted thrice with ethyl acetate. The organic extract was dried over anhydrous $Na_2SO_4$, evaporated, and then the residue was distilled under reduced pressure to give the title compound as a colorless oil; yield: 191 g; bp 113-115°C/0.5 mmHg (Lit. bp 92-93°C/0.3 mmHg; B.H. Baker, W.B. Martin, J. Org. Chem.,

25, 1496 (1960)).

(2) Synthesis of 1-chloro-4-phenylbutane

Thionyl chloride (300 g) was added dropwise in 4-phenylbutanol (189 g, 1.26 mols) obtained in above (1) at room temperature and stirring was continued for 4 h under reflux. The reaction mixture was evaporated and the resultant residue was distilled under reduced pressure to give the title compound as a colorless oil; yield: 149 g; bp 122-123°C/17 mmHg (Lit. bp 90-91°C/2 mmHg; A.Iliceto, A. Fava, A. Simeone, Gazz. chim, ital., 90, 600 (1960)).

(3) Synthesis of 9-phenyl-2,4-nonanedione

To a suspension of sodium hydride (60% in oil, 10.7 g) in dry cyclohexane (140 ml), a solution of 2,4-pentanedione (26.7 g, 0.267 mol) in dry cyclohexane was added dropwise at room temperature with stirring under nitrogen, and the resultant mixture was stirred for 2 h at the same temperature. To this suspension, N,N,N',N'-tetramethylethylenediamine (34.1 g) was added dropwise, followed by the dropwise addition of n-butyllithium (1.6 M in n-hexane solution, 185 ml) at 0°C or lower and the resultant mixture was stirred for 2 h at room temperature. Then to this suspension, 1-chloro-4-phenylbutane (52.3 g, 0.31 mol) obtained in above (2) was added dropwise at 0-5°C and stirring was continued for 3 h at room temperature. The reaction mixture was poured into ice-cold $H_2O$ (1ℓ), neutralized with dilute hydrochloric acid and extracted twice with ethyl acetate. The organic extract was washed with $H_2O$, dried over anhydrous $MgSO_4$ and solvent was removed. The residue was chromatographed on silica gel (Wako Gel C-200) with benzene/n-hexane (4:1) as eluent to give the title compound as a pale yellow oil which was an ca 1:6 mixture of Keto/Enol as seen by the methylene singlet at δ3.55 ppm and the methine singlet at δ5.47 ppm in the $^1$HNMR spectrum; yield; 8.5 g.

$^1$HNMR δppm ($CDCl_3$): 1.34-1.71 (6H, m, $-C\underline{H}_2C\underline{H}_2C\underline{H}_2-CH_2CO-$), 2.04 (3H, s, $-COC\underline{H}_3$), 2.26 (2H, t, J=8Hz, $-C\underline{H}_2COCH_2CO-$), 2.61 (2H, t, J=8Hz, $Ar-C\underline{H}_2-$), 3.55 (2H, s, $-COC\underline{H}_2CO-$), 5.47 (1H, s, 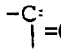 =C$\underline{H}$-CO-), 7.16 -7.30 (5H, m, ArH), 15.49 (1H, bs, -O$\underline{H}$).

IR (Neat) νcm$^{-1}$: 1725, 1600.

(4) Synthesis of 3-diazo-9-phenyl-2,4-nonanedione

Using 9-phenyl-2,4-hexanedione (7.8 g, 0.034 mol) obtained in above (3) and triethylamine (3.4 g, 0.034 mol), the reaction was carried out in the same manner as described in Example 2, (2) and the crude product was purified by column chromatography on silica gel (Wako Gel C-200) eluting with n-hexane/dichloromethane (2:1 → 1:1 → 1:4) to afford the title compound as a yellow viscous oil; yield;5.2 g.

$^1$HNMR δppm ($CDCl_3$): 1.35-1.74 (6H, m, $-C\underline{H}_2C\underline{H}_2C\underline{H}_2-CH_2CO-$), 2.43 (3H, s, $-COC\underline{H}_3$), 2.61 (2H, t, J=8Hz, $Ar-C\underline{H}_2-$), 2.70 (2H, t, J=8Hz, $-C\underline{H}_2CO-$), 7.15-7.30 (5H, m, ArH).

IR (Neat) νcm$^{-1}$: 2100, 1650.

(5) Synthesis of 9-(4-chlorosulfonylphenyl)-3-diazo-2,4-nonanedione

(Compound [II]; $R^{13}$=$-CH_3$, $X^1$=$-SO_2Cl$, $Y^1$=H, m=5)

Using 3-diazo-9-phenyl-2,4-nonanedione (3.6 g, mmol) obtained in above (4), the reaction was carried out in the same manner as described in Example 2, (3) to give the title compound as a slightly yellow viscous oil; yield: 1.4 g.

$^1$HNMR δppm ($CDCl_3$): 1.37-1.75 (6H, m, $-C\underline{H}_2C\underline{H}_2C\underline{H}_2-CH_2CO-$), 2.43 (3H, s, $-COC\underline{H}_3$), 2.74 (4H, m, $-C\underline{H}_2-CO-$ and $Ar-C\underline{H}_2$), 7.41 (2H, d, J=8Hz, Ar2-H, 6-H), 7.95 (2H, d, J=8Hz, Ar3-H, 5-H).

IR (Neat) νcm$^{-1}$: 2100, 1645

UV ($CH_3CN$) $\lambda_{max}$ nm (log ε): 242.2 (4.27).

Anal. Calcd. for $C_{15}H_{17}ClN_2O_4S$: C%, 50.49; H%, 4.80; N%, 7.85.

Found: C%; 50.63; H%, 4.69; N%, 7.77.

Example 14

Synthesis of ethyl 4-(7-diazo-6,8-dioxononyl)benzenesulfonate

(Compound [II]; $R^{13}$=$-CH_3$, $X^1$=$-SO_3C_2H_5$, $Y^1$=H, m=5)

Using 9-(4-chlorosulfonylphenyl)-3-diazo-2,4-nonanedione (1.1 g, 3.1 mmol) obtained in Examle 13, (5), the reaction was carried out in the same manner as described in Example 8 and the crude oil was chromatographed on silica gel (Wako Gel C-200) with chloroform as eluent to give the title compound as a pale yellow viscous oil; yield: 850 mg.

$^1$HNMR δppm ($CDCl_3$): 1.23-1.34 (5H, m, $-C\underline{H}_2CH_2CH_2CO-$ and $-SO_3CH_2C\underline{H}_3$), 1.59-1.64 (4H, m, $Ar-CH_2C\underline{H}_2$ and $-C\underline{H}_2CH_2CO-$), 2.36 (3H, s, $-COC\underline{H}_3$), 2.60-2.69 (4H, m, $Ar-C\underline{H}_2-$ and $-C\underline{H}_2CO-$), 4.04 (2H, q, J=7Hz, $-SO_3C\underline{H}_2CH_3$), 7.27 (2H, d, J=8Hz, Ar2-H, 6-H), 7.73 (2H, d, J=8Hz, Ar3-H, 5-H).

IR (Neat) $\nu cm^{-1}$: 2100, 1640.

Anal. calcd, for $C_{17}H_{22}N_2O_5S$: C%, 55.72; H%, 6.05; N%, 7.64.

Found: C%, 55.61; H%, 6.21; N%, 7.88.

Example 15

Synthesis of 1-(4-chlorosulfonylphenyl)-4-diazo-3,5-octanedione

(Compound [II]; $R^{13}$=$CH_3CH_2CH_2-$, $X^1$=$-SO_2Cl$, $Y^1$=H, m=2)

(1) Synthesis of 1-phenyl-3,5-octanedione

Sodium (7.3 g) was added in small portions to a solution of 4-phenyl-2-butanone (46.8 g, 0.31 mol) and ethyl butyrate (110 g, 0.95 mol) in toluene (120 ml) at 20-60°C under nitrogen, and stirring was continued for 9 h under reflux. After standing overnight, the reaction mixture was taken up in $H_2O$ (300 ml), neutralized with hydrochloric acid and extracted with ethyl acetate. The organic layer was washed with $H_2O$, dried over anhydrous $MgSO_4$ and then evaporated. The residual reddish orange oil (84 g) was distilled under reduced pressure to afford the title compound as a pale yellow oil which was an ca 3:7 mixture of Keto/Enol as seen by the methylene singlet at δ3.52 ppm and the methine singlet as δ5.45 ppm in the $^1$HNMR spectrum; yield: 15.4 g; bp. 166-168°C/2 mmHg.

$^1$HNMR δppm ($CDCl_3$): 0.93 (3H, t, J=7Hz, $-C\underline{H}_3$), 1.53-1.67 (2H, m, $-C\underline{H}_2CH_3$), 2.23 (2H, t, J=7Hz, $-C\underline{H}_2CH_2CH_3$), 2.60 (2H, t, J=7Hz, $Ar-CH_2C\underline{H}_2-$), 2.94 (2H, t, J=7Hz, $Ar-C\underline{H}_2-$), 3.52 (2H, s, $-COC\underline{H}_2CO-$), 5.45 (1H, s, $-C(=C\underline{H}CO-)$), 7.16-7.28 (5H, m, ArH), 15.46 (1H, bs, O$\underline{H}$).

IR (Neat) $\nu cm^{-1}$: 1600.

(2) Synthesis of 4-diazo-1-phenyl-3,5-octanedione

Using 1-phenyl-3,5-octanedione (7.6 g, 35 mmol) obtained in above (1), the reaction was carried out in the same manner as described in Example 3, (2), and the crude oil (12 g) was purified by column chromatography om silica gel (Wako Gel C-200) with n-hexane/ dichloromethane (5:1 → 3:1 → 1:1) as eluent to give the title compound as a yellow oil; yield: 5.3 g.

$^1$HNMR δppm ($CDCl_3$): 0.97 (3H, t, J=7Hz, $C\underline{H}_3$), 1.63-1.77 (2H, m, $-C\underline{H}_2CH_3$), 2.68 (2H, t, J= 7Hz, $-C\underline{H}_2CH_2CH_3$), 2.94-3.11 (4H, m, $A4-C\underline{H}_2C\underline{H}_2-$), 7.19-7.33 (5H, m, ArH).

IR (Neat) $\nu cm^{-1}$: 2140, 1650.

(3) Synthesis of 1-(4-chlorosulfonylphenyl)-4-diazo-3,5-octanedione

(Compound [II]; $R^{13}$=$CH_3CH_2CH_2-$, $X^1$=$-SO_2Cl$, $Y^1$=H, m=2)

Using 4-diazo-1-phenyl-3,5-octanedione (5.2 g, 21.3 mmol) obtained in above (2), the reaction was carried out in the same manner as described in Example 3, (3), and the crude oil (3 g) was chromatographed on silica gel (Wako Gel C-200, manufactured by Wako Pure Chemical Industries, Ltd.) with n-hexane/ethyl acetate (from 5:1 to 3:1) as eluent to give the title compound as a pale yellow viscous oil; yield: 900 mg.

$^1$HNMR δppm ($CDCl_3$): 0.98 (3H, t, J=7Hz, -C$\underline{H}_3$), 1.63-1.77 (2H, m, -C$\underline{H}_2$$CH_3$), 2.63 (2H, t, J=7Hz, -C$\underline{H}_2$$CH_2CH_3$), 3.09 (2H, t, J=7Hz, Ar-$CH_2$C$\underline{H}_2$-), 3.22 (2H, t, J=7Hz, Ar-C$\underline{H}_2$-), 7.49 (2H, d, J=9Hz, Ar2-H, 6-H), 7.95 (2H, d, J=9Hz, Ar3-H, 5-H).

IR (Neat) $\nu cm^{-1}$: 2140, 1650.

Anal. calcd. for $C_{14}H_{15}ClN_2O_4S$: C%, 49.05; H%, 4.41; N%, 8.17.

Found: C%, 48.89; H%, 4.66; N%, 8.34.

Example 16

Synthesis of 6-(4-chlorosulfonylphenyl)-3-diazo-2,4-hexanedione

(Compound [II]; $R^{13}$=-$CH_3$, $X^1$=-$SO_2Cl$, $Y^1$=H, m=2)

(1) Synthesis of potassium 4-(3,5-dioxohexyl)benzenesulfonate

To a suspensoin of 6-phenyl-2,4-hexanedione (15.2 g, 80 mmol) obtained in Example 3, (1) and tantalum powder (100 mg) in carbon tetrachloride (40 ml), chlorosulfonic acid (9.4 g, 80 mmol) was added dropwise at -5~0°C, and stirring was continued for 30 min at the same temperature. After reaction, supernatant solvent was decanted, the residue was treated with cold $H_2O$ (150 ml) and filtered to removed inorganic materials. Then to the filtrate, dilute aqueous potassium hydroxide was added, evaporated to dryness under reduced pressure and the residue was recrystallized from ethanol to give the title compound as a white prisms; yield: 16.0 g; mp 153°C (dec.).

$^1$HNMR δppm ($CDCl_3$-DMSO-$d_6$): 2.01 (3H, s, C$\underline{H}_3$), 2.54 (2H, t, J=7Hz, -$CH_2$C$\underline{H}_2$CO-), 2.85 (2H, t, J=7Hz, Ar-C$\underline{H}_2$-), 7.16 (2H, d, J=8Hz, Ar2-H, 6-H), 7.62 (2H, d, J=8Hz, Ar3-H, 5-H).

IR (KBr) $\nu cm^{-1}$: 1690.

(2) Synthesis of potassium 4-(4-diazo-3,5-dioxohexyl)benzenesulfonate

To a solution of potassium 4-(3,5-dioxohexyl)benzenesulfonate (15.4 g, 50 mmol) obtained in above (1) and triethylamine (5.7 g, 56 mmol) in N,N-dimethylformamide (100 ml) and dichloromethane (20 ml), p-toluenesulfonylazide (10.6 g, 54 mmol) obtained in Example 1 was added dropwise at 0-5°C, and stirring was continued for 4 h at the same temperature. Then the reaction mixture was taken up in dichloromethane (400 ml), the resultant precipitate was filtered, washed thrice with dichloromethane and dried in vacuo to give the title compound as a pale yellow solid; yield: 12.5 g; mp 75°C (dec.).

$^1$HNMR δppm ($CDCl_3$-DMSO-$d_6$): 2.38 (3H, s, C$\underline{H}_3$), 2.89 (2H, t, J=7Hz, -$CH_2$C$\underline{H}_2$CO-), 3.06 (2H, t, J=7Hz, Ar-C$\underline{H}_2$-), 7.18 (2H, d, J=8Hz, Ar2-H, 6-H), 7.63 (2H, d, J=8Hz, Ar3-H, 5-H).

IR (KBr) $\nu cm^{-1}$: 2120, 1640.

(3) Synthesis of 6-(4-chlorosulfonylphenyl)-3-diazo-2,4-hexanedione

(Compound [II]; $R^{13}$=-$CH_3$, $X^1$=-$SO_2Cl$, $Y^1$=H, m=2)

To a solution of potassium 4-(4-diazo-3,5-dioxohexyl)benzenesulfonate (11.7 g, 35 mmol) obtained in above (2) in N, N-dimethylformamide (40 ml), thionyl chloride (6.3 g, 53 mmol) was added dropwise at 10-15°C, and stirring was continued for 2 h at room temperature. The reaction mixture was poured into cold $H_2O$ (400 ml), extracted with chloroform, the organic layer was washed with $H_2O$, dried over anhydrous $MgSO_4$ and evaporated in vacuo. The resultant residue (6.5 g) was purified by column chromatography on silica gel (Wako Gel C-200, manufactured by Wako Pure Chemical Industries, Ltd.) with n-hexane/ethyl acetate (5:1) as eluent to give the title compound as a pale yellow viscous oil, which solidified rapidly to afford pale yellow solid; yield: 4.2 g; mp 58.0-59.5°C.

$^1$HNMR, IR and UV spectra of this product were identical with those of the product as described in Example 3,(3).

Example 17

Synthesis of 1,7-bis(4-chlorosulfonylphenyl)-4-diazo-3,5-heptanedione

(Compound [IV]; $Y^1=Y^2=H$, $X^1=X^2=-SO_2Cl$, m=n=2)

(1) Synthesis of 1,7-diphenyl-3,5-heptanedione

To a suspension of sodium hydride (60% in oil, 17 g) in dry cyclohexane (200 ml), a solution of 2,4-pentanedione (37 g, 0.37 mol) in dry cyclohexane was added dropwise at room temperature with stirring under nitrogen, and the resultant mixture was stirred for 40 min at the same temperature. To this suspension, N,N,N',N'-tetramethylethylenediamine (91.8 g) was added dropwise, followed by the dropwise addition of n-butyllithium (1.6 M in n-hexane solution, 337 g) at 0°C or lower and the resultant mixture was stirred for 24 h at room temperature. Then to this suspension, benzyl chloride (93.7 g) was added dropwise at 0-5°C, stirring was continued for 2 h at room temperature and standed for overnight. The reaction mixture was poured into dilute hydrochloric acid, the organic layer was separated, washed thrice with $H_2O$, dried over $MgSO_4$ and evaporated. The residue (79 g) was distilled under reduced pressure to give the title compound as a pale yellow oil; yield: 23.5 g; bp 185-190°C/0.4 mmHg.

(2) Synthesis of 4-diazo-1,7-diphenyl-3,5-heptanedione

To a solution of 1,7-diphenyl-3,5-heptanedione (16.6 g, 60 mmol) obtained in above (1) and piperidine (5.1 g, 60 mmol) in dichloromethane (120 ml), p-toluenesulfonylazide (12.4 g, 63 mmol) obtained in Example 1 was added dropwise at 0-5°C, and stirring was continued for 2 h at the same temperature. The reaction mixture was washed with dilute aqueous potassium hydroxide and $H_2O$, dried over anhydrous $MgSO_4$ and evaporated in vacuo. The resultant residue (19 g) was chromatographed on silica gel (Wako Gel C-200, manufactured by Wako Pure Chemical Industries, Ltd.) with dichloromethane as eluent to give the title compound as a slightly yellow crystals; yield: 8.0 g; mp 62.5-64.0°C.

$^1$HNMR δppm ($CDCl_3$): 2.93-3.08 (8H, m, $-C\underline{H_2}$-x4), 720 (10H, s, ArH x 2).

IR (KBr) $\nu cm^{-1}$: 2120, 1650.

UV ($CH_3CN$) $\lambda_{max}$ nm (log ε): 231.0 (4.28).

(3) Synthesis of 1,7-bis (4-chlorosulfonylphenyl)-4-diazo-3,5-heptanedione

(Compound [IV]; $Y^1=Y^2=H$, $X^1=X^2=-SO_2Cl$, m=n=2)

A solution of 4-diazo-1,7-diphenyl-3,5-heptanedione (7.5 g, 24 mmol) obtained in above (2) in chloroform was added dropwise into chlorosulfonic acid (22.4 g) at 0-5°C, and stirring was continued for 1 h at the same temperature. The reaction mixture was poured into cold $H_2O$ (0.5 ℓ), extracted with chloroform, the organic extract was washed with $H_2O$ for several times, dried over anhydrous $MgSO_4$ and evaporated in vacuo. The resultant residue (5 g) was chromatographed on silica gel (Wako Gel C-200, manufactured by Wako Pure Chemical Industries, Ltd.) with n-hexane/ethyl acetate (from 3:1 to 1:1) as eluent to give the title compound as yellow crystals; yield: 3.0 g; mp 121-122.5°C.

$^1$HNMR δppm ($CDCl_3$): 3.11 (8H, s, $-C\underline{H_2}$-x4), 7.40 (4H, d, J=8Hz, (Ar2-H, 6-H)x2), 7.53 (4H, d, J=8Hz, (Ar3-H, 5-H)x2).

IR (KBr) $\nu cm^{-1}$: 2130, 1640.

UV ($CH_3CN$) $\lambda_{max}$ nm (log ε): 245.6 (4.54).

Anal. calcd. for $C_{19}H_{16}Cl_2N_2O_6S_2$: C%, 45.34; H%, 3.20; N%, 5.57.

Found: C%, 45.25; H%, 3.36; N%, 5.56.

Example 18

Synthesis of 4-diazo-1,7-bis(4-ethoxysulfonylphenyl-3,5-heptanedione

(Compound [IV], $Y^1=Y^2=H$, $X^1=X^2=-SO_3C_2H_5$, m=n=2)

To a solution of 1,7-bis(4-chlorosulfonylphenyl)-4-diazo-3,5-heptanedione (700 mg, 1.4 mmol) obtained in Example 17, (3), in ethanol (15 ml) and dichloromethane (10 ml), triethylamine (420 mg) was added dropwise

at 5-10°C, and stirring was continued for 8 h at room temperature. The reaction mixture was concentrated under reduced pressure, diluted with dichloromethane (40 ml), washed thrice with $H_2O$, dried over anhydrous $MgSO_4$ and evaporated under reduced pressure. The resultant residue (820 mg) was chromatographed on silica gel (Wako Gel C-200) with n-hexane/ethyl acetate (from 5:1 to 3:1) as eluent to give the title compound as a slightly yellow viscous oil; yield: 450 mg.

$^1$HNMR δppm ($CDCl_3$): 1.31 (6H, t, J=7Hz, $SO_3CH_2C\underline{H}_3$ x2), 3.08 (8H, bs, Ar-$C\underline{H}_2C\underline{H}_2$CO- x 2), 4.12 (4H, q, J=7Hz, $SO_2C\underline{H}_2CH_3$ x 2), 7.41 (4H, d, J=8Hz, (Ar2-H, 6-H) x 2), 7.83 (4H, d, J=8Hz, (Ar 3-H, 5-H) x 2).

IR (Neat) $\nu cm^{-1}$: 2130, 1650.

UV ($CH_3CN$) $\lambda_{max}$ nm (log ε): 229.0 (4.62).

Anal. calcd. for $C_{23}H_{26}N_3O_8S_2$: C%, 52.86; H%, 5.01; N%, 5.36.

Found: C%, 52.65; H%, 5.21; N%, 5.31.

Example 19

Synthesis of 4-diazo-1,7-bis(4-N,N-diethylaminosulfonylphenyl)-3,5-heptanedione

(Compound [IV]; $Y^1=Y^2=H$, $X^1=X^2=-SO_2N(C_2H_5)_2$, m=n=2)

To a solution of 1,7-bis(4-chlorosulfonylphenyl)-4-diazo-3,5-heptanedione (700 mg, 1.4 mmol) obtained in Example 17, (3) in acetonitrile (15 ml), N,N-diethylamine (1 g) was added dropwise at 5-10°C, and stirring was continued for 10 h at room temperature. The reaction mixture was concentrated under reduced pressure, diluted with methylenechloride (40 ml), washed thrice with $H_2O$, dried over anhydrous $MgSO_4$ and evaporated under reduced pressure. The resultant residue (800 mg) was chromatographed on silica gel (Wako Gel C-200) with n-hexane/ethyl acetate (from 5:1 to 3:1) as eluent to give the title compound as a slightly yellow viscous oil; yield: 370 mg.

$^1$HNMR δppm ($CDCl_3$): 1.13 (12H, t, J=7Hz, N-$CH_2C\underline{H}_3$ x 4), 3.05 (8H, bs, -$C\underline{H}_2C\underline{H}_2$CO- x 2), 3.23 (8H, q, J=7Hz, N-$C\underline{H}_2CH_3$ x 4), 7.34 (4H, d, J=8Hz, (Ar2-H, 6-H) x 2), 7.73 (4H, d, J=8Hz, (Ar3-H, 5-H) x 2).

IR (Neat) $\nu cm^{-1}$: 2120, 1650.

UV ($CH_3CN$) $\lambda_{max}$ nm (log ε): 234.4 (4.58).

Anal. calcd. for $C_{27}H_{36}N_4O_6S_2$: C%, 56.23; H%, 6.29; N%, 9.71.

Found: C%, 56.40; H%, 6.42; N%, 9.60.

Example 20

Synthesis of 4-diazo-1,7-bis(4-sulfophenyl)-3,5-heptanedione

(Compound [IV]; $Y^1=Y^2=H$, $X^1=X^2=-SO_3H$, m=n=2)

1,7-Bis(4-chlorosulfonylphenyl)-4-diazo-3,5-heptanedione (2.0 g, 4.0 mmol) obtained in Example 17, (3) was stirred in a solution of acetonitrile (70 ml) and $H_2O$ (50 ml) for 25 h at 30-35°C. The reaction mixture was washed with dichloromethane (30 ml x 3) and then the aqueous layer was evaporated to dryness under reduced pressure to give the title compound as a pale yellow viscous oil; yield: 1.6 g.

$^1$HNMR δppm (DMSO-$d_6$-$CDCl_3$): 2.85 (4H, t, J=7Hz, -$CH_2C\underline{H}_2$CO- x 2), 3.08 (4H, t, J=7Hz, Ar-$C\underline{H}_2CH_2$CO- x 2), 5.32 (2H, bs, $SO_3\underline{H}$ x 2), 7.21 (4H, d, J=8Hz, (Ar3-H, 5-H) x 2), 7.54 (4H, d, J=8Hz, (Ar2-H, 6-H) x 2).

IR (Neat) $\nu cm^{-1}$: 2110, 1635.

Anal. calcd. for $C_{19}H_{18}N_2O_8S_2$: C%, 48.92; H%, 3.89; N%, 6.01.

Found: C%, 49.20; H%, 4.15; N%, 5.79.

Example 21

Synthesis of 4-diazo-1,7-bis(ammonium 4-sulfonatophenyl)-3,5-heptanedione

(Compound [IV]; $Y^1=Y^2=H$, $X^1=X^2=-SO_3^{\ominus}N^{\oplus}H_4$, m=n=2)

To a solution of 4-diazo-1,7-bis(4-sulfophenyl)-3,5-heptanedione (700 mg, 1.5 mmol) obtained in Example 20 in acetonitrile (20 ml), 28% aqueous ammonia (400 mg) was added dropwise at 0-5°C, and stirring was

continued for 3 h at the same temperature. Then the mixture was evaporated under reduced pressure, the residue was washed with acetonitrile for several times and evaporated to dryness under reduced pressure to give the title compound as a slightly yellow viscous oil; yield: 650 mg.

$^1$HNMR δppm ($CDCl_3$-DMSO-$d_6$): 2.84 (4H, t, J=7Hz, $-C\underline{H}_2CO-$ x 2), 3.08 (4H, t, J=7Hz, Ar-$C\underline{H}_2$ x 2), 4.04 (8H, bs, $^{\oplus}N\underline{H}_4$ x 2), 7.18 (4H, d, J=8Hz, (Ar2-H, 6-H) x 2), 7.52 (4H, d, J=8Hz, (Ar3-H, 5-H) x 2).

IR (Neat) $\nu cm^{-1}$: 3300, 2110, 1620.

Example 22

Synthesis of 4-diazo-1,7-bis(triethylammonium 4-sulfonatophenyl)-3,5-heptanedione

(Compound [IV]; $Y^1$=$Y^2$=H, $X^1$=$X^2$=$SO_3^{\ominus}N^{\oplus}H(C_2H_5)_3$, m=n=2)

To a solutoin of 4-diazo-1,7-bis(4-sulfophenyl)-3,5-heptanedione (700 mg, 1.5 mmol) obtained in Example 20 in acetonitrile (20 ml), triethylamine (600 mg) was added dropwise at 0-5°C, and stirring was continued for 3 h at the same temperature. Then the mixture was evaporated under reduced pressure, the resultant residue was washed with acetonitrile for several times and evaporated to dryness under reduced presence to give the title compound as a pale yellow viscous oil; yield: 900 mg.

$^1$HNMR δppm ($CDCl_3$-DMSO-$d_6$): 1.16 (18H, t, J=7Hz, N-$CH_2C\underline{H}_3$ x 6), 2.84 (4H, t, J=7Hz, $-C\underline{H}_2CO-$ x 2), 3.04-3.12 (16H, m, N-$C\underline{H}_2CH_3$ x 6 and Ar-$C\underline{H}_2$ x 2), 3.49 (2H, bs, $-^{\oplus}N\underline{H}$ x 2), 7.18(4H, d, J=8Hz, (Ar2-H, 6-H) x 2), 7.51 (4H, d, J=8Hz, (Ar3-H, 5-H) x 2).

IR (Neat) $\nu cm^{-1}$: 2110, 1650.

Example 23

Synthesis of 1,9-bis(4-chlorosulfonylphenyl)-5-diazo-4,6-nonanedione

(Compound [IV]; $Y^1$=$Y^2$=H, $X^1$=$X^2$=$-SO_2Cl$, m=n=3)

(1) Synthesis of 1,9-diphenyl-4,6-nonanedione

Using 2,4-pentandione (93.8 g, 0.94 mol) and β-phenethyl bromide (405 g), the reaction was carried out in the same manner as described in Example 17, (1), and the crude product (350 g) was purified by column chromatography on silica gel (Wako Gel C-200, manufactured by Wako Pure Chemical Industries, Ltd.) with benzene as eluent to give the title compound as a pale yellow oil which was an ca 1:5 mixture of Keto/Enol as seen by the methylene singlet at δ3.43 ppm and the methine singlet at δ5.42 ppm in the $^1$HNMR spectrum; yield: 66.9 g.

$^1$HNMR δppm ($CDCl_3$): 1.85-1.97 (4H, m, $-C\underline{H}_2CH_2-CO-$ x 2), 2.27 (4H, t, J=8Hz, $-CH_2CH_2-CO$ x 2), 2.62 (4H, t, J=8Hz, Ar-$C\underline{H}_2CH_2-$ x 2) 3.43 (2H, s, $-COC\underline{H}_2CO-$), 5.42 (1H, s, $\overset{-C:}{|}=C\underline{H}-CO-$), 7.10-7.33 (10H, m, Ar-H x 2), 15.53 (1H, bs, -O$\underline{H}$).

IR (Neat) $\nu cm^{-1}$: 1725, 1700, 1610

(2) Synthesis of 5-diazo-1,9-diphenyl-4,6-nonanedione

To a solution of 1,9-diphenyl-4,6-nonanedione (60 g, 0.195 mol) obtained in above (1) and triethylamine (19.8 g, 0.195 mol) in dichloromethane (270 ml), p-toluenesulfonylazide (40.1 g, 0.203 mol) obtained in Example 1 was added dropwise at 0-5°C, and stirring was continued for 2 h at the same temperature. The reaction mixture was washed with dilute aqueous potassium hydroxide and $H_2O$, dried over anhydrous $MgSO_4$ and evaporated under reduced pressure. The residual solid (68 g) was recrystallized from ethanol to afford the title compound as a slightly yellow leaflets; yield: 42.0 g; mp 54.5-56.5°C.

$^1$HNMR δppm ($CDCl_3$): 1.93-2.04 (4H, m, $-C\underline{H}_2CH_2-CO-$ x 2), 2.65-2.75 (8H, m, Ar-$C\underline{H}_2CH_2C\underline{H}_2CO-$ x 2), 7.14-7.31 (10H, m, Ar-H x 2).

IR (KBr) $\nu cm^{-1}$: 2080, 1640.

(3) Synthesis of 1,9-bis(4-chlorosulfonylphenyl)-5-diazo-4,6-nonanedione

(Compound [IV]; $Y^1$=$Y^2$=H, $X^1$=$X^2$=-$SO_2Cl$, m=n=3)

Using 5-diazo-1,9-diphenyl-4,6-nonanedione (21.5 g, 64.3 mmol) obtained in above (2), the reaction was carried out in the same manner as described in Example 17, (3), and the residue (10 g) was chromatographed on silica gel (Wako Gel C-200) with n-hexane/dichloromethane (from 1:1 to 1:4) to give the title compound as pale yellow viscous oil; yield; 6.8 g.

$^1$HNMR δppm ($CDCl_3$): 1.98-2.13 (4H, m, -C<u>H</u>$_2$CH$_2$-CO- x 2), 2.76-2.89 (8H, m, ArC<u>H</u>$_2$CH$_2$C<u>H</u>$_2$-CO- x 2), 7.49 (4H, d, J=8Hz, (Ar2-H, 6-H) x 2), 7.97 (4H, d, J=8Hz, (Ar3-H, 5-H) x 2).

IR (Neat) $\nu cm^{-1}$: 2110, 1650.

UV ($CH_3CN$) $\lambda_{max}$ nm (log ε): 244.2 (4.52).

Anal. calcd. for $C_{21}H_{20}Cl_2N_2O_6S_2$: C%, 47.46; H%, 3.79; N%, 5.27.

Found: C%, 47.39; H%, 3.96; N%, 5.33.

Example 24

Synthesis of 5-diazo-1,9-bis(4-methoxysulfonylphenyl)-4,6-nonanedione

(Compound [IV]; $Y^1$=$Y^2$=H, $X^1$=$X^2$=-$SO_3CH_3$, m=n=3)

Using 1,9-bis(4-chlorosulfonylphenyl)-5-diazo-4,6-nonanedione (800 mg, 1.5 mmol) obtained in Example 23, (3), and methanol (16 ml), the reaction was carried out in the same manner as described in Example 18. The resultant residue (700 mg) was chromatographed on silica gel (Wako Gel C-200) with n-hexane/ethyl acetate (from 5:1 to 3:1) to give the title compound as a slightly yellow viscous oil; yield: 350 mg.

$^1$HNMR δppm ($CDCl_3$): 2.02 (4H, q, J=8Hz, -C<u>H</u>$_2$CH$_2$-CO- x 2), 2.77 (8H, t, J=8Hz, Ar-C<u>H</u>$_2$CH$_2$C<u>H</u>$_2$-CO-X2), 3.75 (6H, s, $SO_3CH_3$ x 2), 7.39 (4H, d, J=8Hz, (Ar2-H, 6-H) x 2), 7.83 (4H, d, J=8Hz, (Ar3-H, 5-H) x 2).

IR (Neat) $\nu cm^{-1}$: 2110, 1645.

UV ($CH_3CN$) $\lambda_{max}$ nm (log ε): 228.3 (4.59).

Anal. calcd. for $C_{23}H_{26}N_2O_8S_2$: C%, 52.86; H%, 5.01; N%, 5.36.

Found: C%, 52.73; H%, 5.19; N%, 5.22.

Example 25

Synthesis of 5-diazo-1,9-bis(4-sulfophenyl)-4,6-nonanedione

(Compound [IV]; $Y^1$=$Y^2$=H, $X^1$=$X^2$=-$SO_3H$, m=n=3)

Using 1,9-bis(4-chlorosulfonylphenyl)-5-diazo-4,6-nonanedione (3.0 g, 5.64 mmol) obtained in Example 23, (3), the reaction was carried out in the same procedure as described in Example 20 to affort the title compound as a pale yellow viscous oil; yield: 2.0 g.

$^1$HNMR δppm (DMSO-$d_6$) : 1.78-1.89 (4H, m, -CH$_2$C<u>H</u>$_2$CH$_2$CO- x 2), 2.61 (4H, t, J=7.5Hz, -C<u>H</u>$_2$CO- x 2), 2.76 (4H, t, J=7.5 Hz, Ar-C<u>H</u>$_2$CH$_2$- x 2), 4.10 (2H, bs, SO$_3$<u>H</u> x 2), 7.18 (4H, d, J=7.5 Hz, (Ar3-H, 5-H) x 2), 7.54 (4H, d, J=7.5Hz, (Ar2-H, 6-H) x 2).

IR (Neat) $\nu cm^{-1}$: 2150, 1640.

Anal. calcd. for $C_{21}H_{22}N_2O_8S_2$ : C%, 51.00; H%, 4.48; N%, 5.66.

Found: C%, 50.91; H%, 4.63; N%, 5.79.

Example 26

Synthesis of 5-diazo-1,9-bis(triethylammonium 4-sulfonatophenyl)-4,6-nonanedione

(Compound [IV]; $Y^1$=$Y^2$=H, $X^1$=$X^2$=-$SO_3^{\ominus}N^{\oplus}H(C_2H_5)_3$, m=n=3)

Using 5-diazo-1,9-bis(4-sulfophenyl)-4,6-nonanedione (1.75 g, 3.54 mmol) obtained in Example 25, the reaction was carried out in the same manner as described in Example 22 to afford the title compound as a slightly yellow viscous oil; yield: 2.23 g.

$^1$HNMR δppm (DMSO-$d_6$): 1.17 (18H, t, J=7.5Hz, N-$CH_2C\underline{H}_3$ x 6), 1.76-1.88 (4H, m, -$C\underline{H}_2CH_2CO$- x 2), 2.60 (4H, t, J=7.7Hz, -$C\underline{H}_2CO$- x 2), 2.74 (4H, t, J=7Hz, Ar-$C\underline{H}_2CH_2$ x 2), 3.08 (12H, q, J=7.5Hz, N-$C\underline{H}_2CH_2$ x 6), 3.37 (2H, bs, N$\underline{H}$ x 2), 7.15 (4H, d, J=8Hz, (Ar2-H, 6-H) x 2), 7.52 (4H, d, J=8Hz, (Ar3-H, 5-H) x 2).

IR (Neat) νcm$^{-1}$: 2700, 2150, 1700, 1640.

Anal. calcd. for $C_{33}H_{52}N_4O_8S_2$: C%, 56.87; H%, 7.52; N%, 8.04.

Found: C%, 56.61; H%, 7.83 ; N%, 8.29.

Example 27

Synthesis of 1,7-bis(3-chlorosulfonyl-4-methylphenyl)-4-diazo-3,5-heptanedione

(Compound [IV]; $Y^1$=$Y^2$=-$CH^3$, $X^1$=$X^2$=-$SO_2Cl$, m=n=2)

(1) Synthesis of 1,7-bis(4-methylphenyl)-3,5-heptanedione

Using 2,4-pentandione (195.2 g, 1.95 moles) and 4-methylbenzylchloride (635.4 g), the reaction was carried out in the same manner as described in Example 17, (1), and the residue (568 g) was recrystallized from ethanol to afford the title compound as a white needles which was an ca 1:9 mixture of Keto/Enol as seen by the methylene singlet at δ3.50 ppm and the methine singlet at δ5.43 ppm in the $^1$HNMR spectrum; yield: 155 g; mp 74.8-75.7°C.

$^1$HNMR δppm ($CDCl_3$): 2.31 (6H, s, Ar-$C\underline{H}_3$ x 2), 2.55 (4H, t, J=8Hz, -$CH_2C\underline{H}_2$-CO- x 2), 2.88 (4H, t, J=8Hz, Ar$C\underline{H}_2CH_2$-CO- x 2), 3.50 (2H, s, -$COC\underline{H}_2CO$-), 5.43(1H, s, 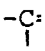 =C$\underline{H}$-CO-), 7.04-7.11 (8H, m, ArH x 2), 15.43 (1H, bs, -O$\underline{H}$).

IR (KBr) νcm$^{-1}$: 1620.

(2) Synthesis of 1,7-bis(4-methylphenyl)-4-diazo-3,5-heptanedione

Using 1,7-bis(4-methylphenyl)-3,5-heptanedione (112.8 g, 0.366 mol) obtained in above (1), the reaction was carried out in the same manner as described in Example 17, (2), and the residue (129 g) was recrystallized from ethanol to afford the title compound as a slightly yellow prisms; yield: 103 g; mp 45.6-46.8°C.

$^1$HNMR δppm ($CDCl_3$): 2.31 (6H, s, Ar-$C\underline{H}_3$x 2), 2.88-3.05 (8H, m, Ar-$C\underline{H}_2C\underline{H}_2CO$- x 2), 7.10 (8H, s, ArH x 2).

IR (KBr) νcm$^{-1}$: 2100, 1660.

(3) Synthesis of 1,7-bis(3-chlorosulfonyl-4-methylphenol)-4-diazo-3,5-heptanedione

(Compound [IV]; $Y^1$=$Y^2$=-$CH_3$, $X^1$=$X^2$=-$SO_2Cl$, m=n=2)

Using 1,7-bis(4-methylphenyl)-4-diazo-3,5-heptanedione (90 g, 0.269 mol) obtained in above (2), the reaction was carried out in the same manner as described in Example 17, (3), and the residue was chromatographed on silica gel (Wako Gel C-200) with n-hexane/ethyl acetate (1:1) as eluent to give the title compound as a pale yellow viscous oil; yield: 30 g.

$^1$HNMR δppm ($CDCl_3$): 2.74 (6H, s, Ar-$C\underline{H}_3$ x 2), 3.04-3.08 (8H, m, Ar-$C\underline{H}_2C\underline{H}_2$-CO- x 2), 7.34 (2H, d, J=8Hz, Ar5-H x 2), 7.49 (2H, dd, J=2Hz and J=8Hz, Ar6-H x 2), 7.90 (2H, d, J=2Hz, Ar2-H x 2).

IR (Neat) νcm$^{-1}$: 2110, 1650.

UV ($CH_3CN$) $\lambda_{max}$ nm (log ε): 233.1 (4.44).

Anal. calcd, for $C_{21}H_{20}Cl_2N_2O_6S_2$: C%, 47.46; H%, 3.79; N%, 5.27.

Found: C%, 47.54; H%, 3,99; N%, 5.21.

Example 28

Synthesis of 4-diazo-1,7-bis(3-methoxysulfonyl-4-methylphenyl)-3,5-heptanedione

(Compound [IV]; $Y^1$=$Y^2$=-$CH_3$, $X^1$=$X^2$=-$SO_3CH_3$, m=n=2)

Using 1,7-bis(3-chlorosulfonyl-4-methylphenyl)-4-diazo-3,5-heptanedione (3.1 g, 5.8 mmol) obtained in Example 27, (3) and methanol (25 ml), the reaction was carried out in the same manner as described in Example

18, and the residue (2.9 g) was chromatographed on silica gel (Wako Gel C-200, manufactured by Wako Pure Chemical Industries, Ltd.) with n-hexane/ethyl acetate (5:1 → 3:1 → 2:1) as eluent to give the title compound as a slightly yellow viscous oil; yield: 1.9 g.

$^1$HNMR δppm ($CDCl_3$): 2.60 (6H, s, Ar-C$\underline{H}_3$ x 2), 3.01-3.05 (8H, m, Ar-C$\underline{H}_2$C$\underline{H}_2$-CO- x 2), 3.74 (6H, s, $SO_3C\underline{H}_3$ x 2), 7.28 (2H, d, J=8Hz, Ar5-H x 2), 7.40 (2H, dd, J=8Hz and J=2Hz, Ar6-H x 2), 7.82 (2H, d, J=2Hz, Ar2-H x 2).

IR (Neat) $\nu cm^{-1}$: 2120, 1640.

UV ($CH_3CN$) $\lambda_{max}$ nm (log ε): 225.4 (4.46).

Anal. calcd. for $C_{23}H_{26}N_2O_8S_2$: C%, 52.86, H%, 5.01; N%, 5.36.

Found: C%, 52.74, H%, 5.24, N%, 5.26.

Example 29

Synthesis of 4-diazo-1,7-bis(3-ethoxysulfonyl-4-methylphenyl)-3,5-heptanedione

(Compound [IV]; $Y^1$=$Y^2$=-$CH_3$, $X^1$=$X^2$=-$SO_3C_2H_5$, m=n=2)

Using 1,7-bis(3-chlorosulfonyl-4-methylphenyl)-4-diazo-3,5-heptanedione (700 mg, 1.3 mmol) obtained in Example 27, (3) and ethanol (20 ml), the reaction was carried out in the same manner as described in Example 18, and the residue (700 mg) was chromatographed on silica gel (Wako Gel C-200) with n-hexane/ethyl acetate (from 5:1 to 3:1) as eluent to give the title compound as a slightly yellow viscous oil; yield: 450 mg.

$^1$HNMR δppm ($CDCl_3$): 1.32 (6H, t, J=7Hz, $SO_3CH_2C\underline{H}_3$ x 2), 2.61 (6H, s, Ar-C$\underline{H}_3$ x 2), 3.01-3.08 (8H, m, Ar-C$\underline{H}_2$C$\underline{H}_2$-CO- x 2), 4.10 (4H, q, J=7Hz, $SO_3C\underline{H}_2CH_2$ x 2), 4.27 (2H, d, J=8Hz, Ar5-H x 2), 7.39 (2H, dd, J=2Hz and J=8Hz, Ar6-H x 2), 7.82 (2H, d, J=2Hz, Ar2-H x 2).

IR (Neat) $\nu cm^{-1}$: 2110, 1645.

UV ($CH_3CN$) $\lambda_{max}$ nm (log ε): 225.4 (4.49).

Anal. calcd. for $C_{25}H_{30}N_2O_8S_2$: C%, 54,53; H%, 5.49; N%, 5.09.

Found: C%, 54.46; H%, 5.56; N%, 5.14

Example 30

Synthesis of 4-diazo-1,7-bis(3-N,N-diethylaminosulfonyl-4-methylphenyl)-3,5-heptanedione

(Compound [IV]; $Y^1$=$Y^2$=-$CH_3$, $X^1$=$X^2$=-$SO_2N(C_2H_5)_2$, m=n=2)

Using 1,7-bis(3-chlorosulfonyl-4-methylphenyl)-4-diazo-3,5-heptanedione (700 mg, 1.3 mmol) obtained in Example 27, (3) and N,N-diethylamine (1 g), the reaction was carried out in the same manner as described in Example 19,,and the resultant oil (800 mg) was chromatographed on silica gel (Wako Gel C-200) with n-hexane/ethyl acetate (from 5:1 to 2:1) as eluent to give the title compound as a pale yellow viscous oil; yield: 380 mg.

$^1$HNMR δppm ($CDCl_3$): 1.21 (12H, t, J=7Hz, N-$CH_2C\underline{H}_3$ x 4), 2.55 (6H, s, Ar-C$\underline{H}_3$ x 2), 2.96-3.08 (8H, m, -C$\underline{H}_2$C$\underline{H}_2$CO- x 2), 3.30 (8H, q J=7Hz, N-C$\underline{H}_2$$CH_3$ x 4), 7.20 (2H, d, J=8Hz, Ar5-H x 2), 7.28 (2H, d, J=8Hz, Ar6-H x 2), 7.76 (2H, s, Ar2-H x 2).

IR (Neat) $\nu cm^{-1}$: 2120, 1650.

UV ($CH_3CN$) $\lambda_{max}$ nm (log ε): 227.2 (4.49).

Anal. cacd. for $C_{29}H_{40}N_4O_6S_2$: C%, 57.59; H%, 6.67; N%, 9.26.

Found: C%, 57.71; H%, 6.42; N%, 9.50.

Example 31

Synthesis of 4-diazo-1,7-bis(4-methyl-3-sulfophenyl)-3,5-heptanedione

(Compound [IV]; $Y^1$=$Y^2$=-$CH_3$, $X^1$=$X^2$=-$SO_3H$, m=n=2)

To a solution of 1,7-bis(3-chlorosulfonyl-4-methylphenyl)-4-diazo-3,5-heptanedione (1.4 g, 2.6 mmol) obtained in Example 27, (3) in tetrahydrofuran (40 ml), $H_2O$ (40 ml) was added, and stirring was continued for 48 h at room temperature. The reaction mixture was washed with dichloromethane for several times and evaporated to dryness under reduced pressure to afford the title compound as a pale yellow viscous oil; yield: 1.2 g.

$^1$HNMR δppm ($CDCl_3$-DMSO-$d_6$): 2.48 (6H, s, $C\underline{H}_3$ x 2), 2.80 (4H, t, J=7Hz, -$C\underline{H}_2$CO- x 2), 3.04 (4H, t, J=7Hz, A4-$C\underline{H}_2$- x 2), 4.98 (2H, bs, -$SO_3\underline{H}$ x 2), 7.02-7.14 (4H, m, (Ar5-H, 6-H) x 2), 7.60 (2H, s, Ar2-H x 2).

IR (Neat) $\nu cm^{-1}$: 2110, 1630.

Anal. calcd. for $C_{21}H_{22}N_2O_8S_2$: C%, 51.00; H%, 4.48; N%, 5.66.

Found: C%, 50.82; H%, 4.73; N%, 5.93.

Example 32

Synthesis of 4-diazo-1,7-bis(ammonium 4-methyl-3-sulfonatophenyl)-3,5-heptanedione

(Compound [IV], $Y^1$=$Y^2$=-$CH_3$, $X^1$=$X^2$=-$SO_3^{\ominus}N^{\oplus}H_4$, m=n=2)

Using 4-diazo-1,7-bis(4-methyl-3-sulfophenyl)-3,5-heptanedione (1.0 g, 2 mmol) obtained in Example 31, the reaction was carried out in the same procedure as described in Example 21 to give the title compound as a pale yellow viscous oil; yield: 950 mg.

$^1$HNMR δppm ($CDCl_3$-DMSO-$d_6$): 2.60 (6H, s, $C\underline{H}_3$ x 2), 2.83 (4H, t, J=7Hz, -$C\underline{H}_2$CO- x 2), 3.06 (4H, t, J=7Hz, Ar-$C\underline{H}_2$- x 2), 4.74 (8H, bs, $^{\oplus}N\underline{H}_4$ x 2), 7.07 (4H, s, (Ar5-H, 6-H) x 2), 7.06 (2H, s, Ar2-H x 2).

IR (Neat) $\nu cm^{-1}$: 3300, 2090, 1620.

Anal. calcd. for $C_{21}H_{28}N_4O_8S_2$: C%, 47.72; H%, 5.34; N%, 10.60.

Found: C%, 47.50; H%, 5.51; N%, 10.91.

Example 33

Synthesis of 1,7-bis(4-chlorosulfonyl-1-naphthyl) 4-diazo-3,5-heptanedione

(Compound [VI]; $Y^1$=$Y^2$=H, $X^1$=$X^2$=-$SO_2Cl$, m=n=2)

(1) Synthesis of 1,7-di(1-naphthyl)-3,5-heptanedione

Using 2,4-pentanedione (59.6 g, 0.595 mol) and 1-chloromethylnaphthalene (245 g), the reaction was carried out in the same manner as described in Example 17, (1), and the residual oil (200 g) was chromatographed on silica gel (Wako Gel C-200) with n-hexane/benzene (1:4) as eluent to give the title compound as a yellow oil which was an ca 1:6 mixture of Keto/Enol as seen by the methylene singlet at δ3.33 ppm and the methine singlet at δ5.27 ppm in the $^1$HNMR spectrum; yield: 71 g.

$^1$HNMR δppm ($CDCl_3$): 2.56 (4H, t, J=8Hz, -$CH_2C\underline{H}_2$-CO- x 2), 3.24 (4H, t, J=8Hz, -$C\underline{H}_2CH_2$CO- x 2), 3.33 (2H, s, -CO$C\underline{H}_2$CO-), 5.27 (1H, s, $\underset{|}{-C}$=C$\underline{H}$-CO-), 7.17-7.91 (14H, m, ArH x 2), 15.43 (1H, bs, -O$\underline{H}$).

IR (Neat) $\nu cm^{-1}$: 1720, 1600.

(2) Synthesis of 4-diazo-1,7-di(1-naphthyl)-3,5-heptanedione

Using 1,7-di(1-naphthyl)-3,5-heptanedione (30 g, 79 mmol) obtained in above (1), the reaction was carried out in the same manner as described in Example 17, (2), and the residue (35 g) was chromatographed on silica gel (Wako Gel C-200) with n-hexane/dichloromethane (from 4:1 to 2:1) as eluent to give the title compound as a pale yellow viscous oil; yield: 22.7 g.

$^1$HNMR δppm ($CDCl_3$): 3.14 (4H, t, J=8Hz, -$CH_2C\underline{H}_2$-CO x 2), 3.42 (4H, t, J=8Hz, -$C\underline{H}_2CH_2$-CO- x 2), 7.34-8.03 (4H, m, ArH x 2).

IR (Neat) $\nu cm^{-1}$: 2120, 1650.

(3) Synthesis of 1,7-bis(4-chlorosulfonyl-1-naphthyl)-4-diazo-3,5-heptanedione

(Compound [VI]; $Y^1$=$Y^2$=H, $X^1$=$X^2$=-$SO_2Cl$, m=n=2)

Using 4-diazo-1,7-di(1-naphthyl)-3,5-heptanedione (11.0 g, 27 mmol) obtained in above (2), the reaction was carried out in the same manner as described in Example 17, (3), and the residual oil (5 g) was purified by column chromatography on silica gel (Wako Gel C-200, manufactured by Wako Pure Chemical Industries, Ltd.) with n-hexane/dichloromethane (1:1 → 1:4 → 1:9) as eluent to give the title compound as a slightly yellow

viscous oil; yield: 2.6 g.

$^1$HNMR δppm ($CDCl_3$): 3.22 (4H, t, -$CH_2C\underline{H}_2CO$- x 2), 3.55 (4H, t, -$C\underline{H}_2CH_2CO$- x 2), 7.52 (2H, d, J=8Hz, Ar2-H x 2), 7.71-7.88 (4H, m, (Ar6-H, 7-H) x 2), 8.20 (2H, d, J=8Hz, Ar8-H x 2), 8.29 (2H, d, J-8Hz, Ar3-H x 2), 8.85 (2H, d, J=8Hz, Ar5-H x 2).

IR (Neat) $\nu cm^{-1}$: 2100, 1645.

UV ($CH_3CN$) $\lambda_{max}$ nm (log ε): 239.4 (4.81).

Anal. calcd. for $C_{27}H_{20}Cl_2N_2O_6S_2$: C%, 53.74; H%, 3.34; N%, 4.64.

Found: C%, 53.59; H%, 3.51; N%, 4.52.

Example 34

Synthesis of 1,7-bis(4-chlorosulfonyl-1-naphthyl)-4-diazo-3,5-heptanedione.

(Compound [VI], $Y^1$=$Y^2$=H, $X^1$=$X^2$=-$SO_2Cl$, m=n=2)

(1) Synthesis of 1,7-bis(potassium 4-sulfonate-1-naphthyl)-3,5-heptanedione

To a suspension of 1,7-di-(1-naphthyl)-3,5-heptanedione (16 g, 40 mmol) obtained in Example 33, (1), and tantalum powder (100 mg) in carbon tetrachloride (35 ml), chlorosulfonic acid (9.4 g, 80 mmol) was added dropwise at -5~0°C, and stirring was continued for 30 min at the same temperature. After reaction, supernatant solvent was decanted, the residue was treated with cold $H_2O$ (80 ml) and filtered to removed inorganic materials. The filtrate was neutralized with dilute aqueous potassion hydroxide and then the precipitate was filtered off. The resultant filtrate was evaporated to dryness under reduced pressure to give the title compound as a pale yellow solid; yield: 17.9 g.

$^1$HNMR δppm (DMSO-$d_6$): 2.68-2.75 (4H, m, -$CH_2C\underline{H}_2CO$- x 2), 3.23-3.40 (4H, m, -$C\underline{H}_2CH_2CO$- x 2), 7.30-8.15 (10H, m, (Ar2-H, 3-H, 6-H, 7-H, 8-H) x 2), 8.77 (2H, d, J=8Hz, Ar5-H x 2).

IR (KBr) $\nu cm^{-1}$: 1720, 1700, 1600.

(2) Synthesis of 4-diazo-1,7-bis(potassium 4-sulfonato-1-naphthyl)-3,5-heptanedione.

To a solution of 1,7-bis(potassium 4-sulfonato-1-naphthyl)-3,5-heptanedione (8.9 g, 14.3 mmol) obtained in above (1) and triethylamine (1.6 g, 15.9 mmol) in N,N-dimethylformamide (50 ml) and dichloromethane (10 ml), p-toluenesulfonylazide (3.1 g, 15.5 mmol) obtained in Example 1 was added dropwise at 0-5°C, and stirring was continued for 4 h at the same temperature. Then the reaction mixture was taken up in dichloromethane (200 ml), the resultant precipitate was filtered, washed thrice with dichloromethane and dried in vacuo to give the title compound as a slightly yellow solid: yield: 6.9 g.

$^1$HNMR δppm (DMSO-$d_6$): 3.17-3.24 (4H, m, -$CH_2C\underline{H}_2CO$- x 2), 3.33-3.52 (4H, m, -$C\underline{H}_2CH_2CO$- x 2), 7.31-8.04 (10H, m, (Ar2-H, 3-H, 6-H, 7-H, 8-H) x 2), 8.92 (2H, d, J=8Hz, Ar5-H x 2).

IR (KBr) $\nu cm^{-1}$: 2100, 1655.

(3) Synthesis of 1,7-bis(4-chlorosulfonyl-1-naphthyl)-4-diazo-3,5-heptanedione

(Compound [VI]; $Y^1$=$Y^2$=H, $X^1$=$X^2$=-$SO_2Cl$, m=n=2)

To a solution of 4-diazo-1,7-bis(potassium 4-sulfonato-1-naphthyl)-3,5-heptanedione (6.0 g, 9.3 mmol) obtained in above (2) in N,N-dimethylformamide (20 ml), thionylchloride (3.4 g, 28 mmol) was added dropwise at 10-15°C, and stirring was continued for 2 h at 10-15°C. The reaction mixture was poured into cold $H_2O$ (200 ml), extracted with chloroform, the organic extract was washed thrice with $H_2O$, dried over anhydrous $MgSO_4$ and evaporated in vacuo. The resultant residue (1.7 g) was chromatographed on silica gel (Wako Gel C-200) with n-hexane/dichloromethane (1:1 → 1:4 → 1:9) as eluent to give the title compound as a slightly yellow viscous oil; yield: 800 mg.

$^1$HNMR, IR and UV spectra of this product were identical with those of the product as described in Example 33, (3).

Example 35

Synthesis of 4-diazo-1,7-bis(4-methoxysulfonyl-1-naphthyl)-3,5-heptanedione

(Compound [VI]; $Y^1$=$Y^2$=H, $X^1$=$X^2$=-$SO_3CH_3$, m=n=2)

Using 1,7-bis(4-chlorosulfonyl-1-naphthyl)4-diazo-3,5-heptanedione (4.0 g, 6.6 mmol) obtained in Example 33 and methanol (30 ml), the reaction was carried out in the same manner as described in Example 18, and the resultant residue (3.7 g) was chromatographed on silica gel (Wako Gel C-200, manufactured by Wako Pure Chemical Industries, Ltd.) with n-hexane/ethyl acetate (from 5:1 to 2:1) as eluent to give the title compound as a white amorphous solid; yield: 1.9 g.

$^1$HNMR δppm ($CDCl_3$): 3.20 (4H, t, -$CH_2$CO- x 2), 3.51 (4H, t, Ar-$CH_2$- x 2), 3.72 (6H, s, -$SO_3CH_3$ x 2), 7.49 (2H, d, J=8Hz, Ar2-H x 2), 7.64-7.74 (4H, m, (Ar6-H, 7-H) x 2), 8.15 (2H, dd, J=8Hz and J=2Hz, Ar8-H x 2), 8.21 (2H, d, J=8Hz, Ar3-H x 2), 8.65 (2H, dd, J=8Hz and J=2Hz, Ar5-H x 2).

IR (KBr) $\nu cm^{-1}$: 2120, 1640.

UV ($CH_3CN$) $\lambda_{max}$ nm (log ε): 228.8 nm (5.01).

Example 36

Synthesis of 1,13-bis(4-chlorosulfonyl)-7-diazo-6,8-tridecanedione

(Compound [IV]; $Y^1$=$Y^2$=H, $X^1$=$X^2$=-$SO_2Cl$, m=n=5)

(1) Synthesis of 1,13-diphenyl-6,8-tridecanedione

Using 2,4-pentanedione (26.7 g, 0.267 mol) and 1-chloro-4-phenylbutane (110 g, 0.65 mol) obtained in Example 13, (2), the reaction was carried out in the same manner as described in Example 17, (1), and the residue was chromatographed on silica gel (Wako Gel C-200) with benzene/n-hexane (from 4:1 to 10:1) as eluent to afford the title compound as a pale yellow oil which was an ca 1:4 mixture of Keto/Enol as seen by the methylene singlet at δ3.52 ppm and the methine singlet at δ5.44 ppm in the $^1$HNMR spectrum; yield: 2.5 g.

$^1$HNMR δppm ($CDCl_3$): 1.26-1.42 (4H, m, -$CH_2CH_2CH_2$CO- x 2), 1.56-1.69 (8H, m, -$CH_2$-$CH_2CH_2CH_2$CO- x 2), 2.26 (4H, t, J=8Hz, -$CH_2CH_2$CO- $CH_2$ x 2), 2.61 (4H, t, J=8Hz, Ar-$CH_2$- x 2), 3.52 (2H, s, -CO$CH_2$CO-), 5.44 (1H, s, $\overset{-C}{|}$=CH-CO-), 7.15-7.30 (10H, m, Ar-H x 2), 15.52 (1H, bs, -OH).

IR (Neat) $\nu cm^{-1}$: 1700, 1600.

(2) Synthesis of 7-diazo-1,13-diphenyl-6,8-tridecanedione

Using 1,13-diphenyl-6,8-tridecanedione (2.0 g, 5.5 mmol) obtained in above (1), the reaction was carried out in the same manner as described in Example 17, (2), and the residue (2.8 g) was chromatographed on silica gel (Wako Gel C-200) with n-hexane/ethyl acetate (4:1) as eluent to give the title compound as a pale yellow viscous oil; yield: 1.9 g.

$^1$HNMR δppm ($CDCl_3$): 1.34-1.43 (4H, m, -$CH_2CH_2CH_2$CO- x 2), 1.58-1.73 (8H, m, $CH_2CH_2CH_2CH_2$CO- x 2), 2.57-2.64 (4H, m, -$CH_2CH_2$CO- x 2), 2.67-2.74 (4H, m, Ar-$CH_2$- x 2), 7.14-7.30 (10H, m, Ar-H x 2).

IR (Neat) $\nu cm^{-1}$: 2100, 1650.

(3) Synthesis of 1,13-bis(4-chlorosulfonyl)-7-diazo-6,8-tridecanedione

(Compound [IV]; $Y^1$=$Y^2$=H, $X^1$=$X^2$=-$SO_2Cl$, m=n=5)

Using 7-diazo-1,13-diphenyl-6,8-tridecanedione (1.6 g, 4.0 mmol) obtained in above (2), the reaction was carried out in the same manner as described in Example 17, (3), and the residue (0.8 g) was chromatographed on silica gel (Wako Gel C-200) with n-hexane/ethyl acetate (from 2:1 to 1:1) as eluent to give the title compound as a slightly yellow viscous oil; yield: 300 mg.

$^1$HNMR δppm ($CDCl_3$): 1.19-1.35 (4H, m, -$CH_2CH_2CH_2$CO- x 2), 1.51-1.94 (12H, m, -$CH_2$- x 6), 2.70-2.77 (4H, m, Ar-$CH_2$- x 2), 7.41 (4H, d, J=8.5Hz, (Ar2-H, 6-H) x 2), 7.95 (4H, d, J=8.5 Hz, (Ar3-H, 5-H) x 2).

IR (Neat) $\nu cm^{-1}$: 2160, 1650, 1370.

Anal. calcd for $C_{25}H_{28}Cl_2N_2O_6S_2$: C%, 51.11; H%, 4.80; N%, 4.77.

Found: C%, 50.83; H%, 5.06; N%, 4.58.

Example 37

Synthesis of mixture of 1,7-bis(6-and 8-chlorosulfonyl-2-naphthyl)-4-diazo-3,5-heptanedione

Compound [VI]; $Y^1$=$Y^2$-H, $X^1$=$X^2$=-$SO_2Cl$, m=n=2)

(1) Synthesis of ethyl 2-naphthoate

To a solution of 2-naphthoic acid (121 g, 0.7 mol) in chloroform (1.5 $\ell$) and N,N-dimethylform amide (100 ml), thionyl chloride (167 g) was added dropwise under reflux and stirring was continued for 1 h under reflux. The reaction mixture was concentrated in vacuo and the resultant residue was added dropwise to a solution of triethylamine (106 g) and ethanol (150 ml) at 10°C or lower. The mixture was stirred for 1 h at room temperature, poured into $H_2O$ (1 $\ell$), extracted with chloroform, the organic extract was washed with $H_2O$, dried over anhydrous $MgSO_4$ and evaporated under reduced pressure. The residue was distilled under reduced pressure to give the title compound as a colorless oil which solidified in the refrigerator; yield: 112 g; bp 145-147°C/2 mm Hg; mp 35.0-36.5°C. (Lit. bp 146-147°C/1-2 mmHg, mp 35.5-36.2°C; C.C. Price. P.H. Michel, J.Am. Chem. Soc., 74, 3652 (1952).).

(2) Synthesis of 2-hydroxymethylnaphthalene

To a suspension of lithium aluminum hydride (19 g) in ethyl ether (500 ml), a solution of ethyl 2-naphthoate (111 g, 0.55 mol) obtained in above (1) in ethyl ether was added dropwise at 10°C or lower under nitrogen, and stirring was continued for 1 h at room temperature. To this reaction mixture, ethyl acetate (200 ml) was added dropwise to destroy excess $LiAlH_4$. The resultant mixture was poured into hydrochloric acid (200 ml) and $H_2O$ (300 ml), the organic layer was separated, washed with $H_2O$, dried over anhydrous $MgSO_4$ and evaporated under reduced pressure. The residual brown solid was recrystallized from ligroin to give the title compound as a white needles; yield: 74.0 g; mp 80.5-81.5C (Lit. mp 80.5-81C; C.R. Hauser, D.N. VanEenam, P.L. Bayless, J. Org. Chem., 23, 354 (1958).).

(3) Synthesis of 2-chloromethylnaphthalene

To a solution of 2-hydroxymethylnaphthalene (53.7 g, 0.34 mol) obtained in above (2) in dichloromethane (300 ml), thionyl chloride (60.7 g) was added dropwise at 30±5°C, and stirring was continued for 1 h under reflux. After cooling, the reaction mixture was washed twice with $H_2O$, dried over anhydrous $MgSO_4$ and evaporated in vacuo. The resultant oil (54 g) was distilled under reduced pressure to give the title compound as a pale yellow viscous oil which solidified in the refrigerator; yield: 44.2 g; bp 129-133°C/2 mmHg, mp 46.8-48.0°C (Lit. bp 125-132°C/2 mmHg, mp 47-48°C; C.R. Hauser, D.N. VanEenam. P.L. Bayless, J. Org. Chem., 23, 354 (1958).).

(4) Synthesis of 1,7-di-(2-naphthyl)-3,5-heptanedione

Using 2,4-pentanedione (10.0 g, 0.1 mol) and 2-chloromethylnaphthalene (35.3 g, 0.2 mol) obtained in above (3), the reaction was carried out in the same manner as described in Example 17, (1) and crude product (32 g) was chromatographed on silica gel (Wako Gel C-200, manufactured by Wako Pure Chemical Industries, Ltd.) with benzene as eluent to give the title compound as a pale yellow amorphous solid which was an ca 3:2 mixture of Keto/Enol as seen by the methylene singlet at δ3.53 ppm and the methine singlet at δ5.45 ppm in the $^1$HNMR spectrum; yield: 15.4 g; mp 123.0-125.0°C.

$^1$HNMR δppm ($CDCl_3$): 2.65 (4H, t, J=8Hz, -$C\underline{H}_2CO$- x 2), 3.06 (4H, t, J=8Hx, -$C\underline{H}_2CH_2CO$- x 2), 3.53 (2H, s, -$CO C\underline{H}_2 CO$-), 5.45 (1H, s, $-\overset{|}{\underset{|}{C}}$=C$\underline{H}$-CO-), 7.29 (2H, d, J=6.5Hz, Ar4-H x 2), 7.31-7.40 (4H, m, (Ar5-H, 7-H) x 2), 7.60 (2H, bs, Ar1-H x 2), 7.73-7.81 (6H, m, (Ar3-H, 6-H, 8-H) x 2), 15.46 (1H, bs, O$\underline{H}$).

IR (KBr) $\nu cm^{-1}$: 1600.

(5) Synthesis of 4-diazo-1,7-di-(2-naphthyl)-3,5-heptanedione

Using 1,7-di-(2-naphthyl)-3,5-heptanedione (6.85 g, 18 mmol) obtained in above (4), the reaction was carried out in the same procedure as described in Example 17, (2), and crude oil (9.8 g) was purified by column chromatography on silica gel (Wako Gel C-200) with n-hexane/dichloromethane (1:1) as eluent to give the title compound as a yellow viscous oil; yield: 6.8 g.

$^1$HNMR ppm ($CDCl_3$): 3.12 (8H, bs, -$C\underline{H}_2C\underline{H}_2$CO- x 2), 7.33 (2H, d, J=8Hz, Ar4-H x 2), 7.41-7.62 (4H, m, (Ar5-H, 7-H) x 2), 7.64 (2H, bs, Ar1-H x 2), 7.75-7.81 (6H, m, (Ar3-H, 6-H, 8-H) x 2).

IR (Neat) $\nu cm^{-1}$: 2100, 1650.

(6) Synthesis of mixture of 1,7-bis(6- and 8-chlorosulfonyl-2-naphthyl)-4-diazo-3,5-heptanedione

(Compound [VI]; $Y^1=Y^2$=H, $X^1=X^2$=-$SO_2Cl$, m=n=2)

Using 4-diazo-1,7-di-(2-naphthyl)-3,5-heptanedione (6.4 g, 15.7 mmol) obtained in above (5), the reaction was carried out in the same manner as described in Example 17, (3) and crude oil (1.1 g) was chromatographed on silica gel (Wako Gel C-200) with n-hexane/dichloromethane (1:2) as eluent to give the title compound as a slightly yellow viscous oil which was an ca 1:1 mixture of 6-isomer/8-isomer by the singlet at δ8.54 ppm and δ8.71 ppm in the $^1$HNMR spectrum; yield: 250 mg.

$^1$HNMR δppm ($CDCl_3$): 3.19-3.31 (8H, m, -$C\underline{H}_2C\underline{H}_2$- x 2), 7.52-8.36 (8H, m, ArH x 2), 8.54 (1H, bs, Ar5-H: 6-isomer), 8.71 (1H, d, J=9.0Hz, Ar7-H: 8-isomer).

IR (Neat) $\nu cm^{-1}$: 2110, 1650.

Anal. calcd. for $C_{27}H_{20}Cl_2N_2O_6S_2$: C%, 53.73; H%, 3.34; N%, 4.64.

Found: C%, 53.49; H%, 3.45; N%, 4.81.

Example 38

Synthesis of 1,7-bis(3-chlorosulfonyl-4-methylphenyl)-4-diazo-3,5-heptanedione

(Compound [IV]; $Y^1=Y^2$=-$CH_3$, $X^1=X^2$=-$SO_2Cl$, m=n=2)

(1) Synthesis of 1,7-bis(sodium 4-methyl-3-sulfonatophenyl)-3,5-heptanedione

To a suspension of 1,7-bis(4-methylphenyl)-3,5-heptanedione (41.6 g, 0.135 mol) obtained in Example 27, (1) and tantalum powder (300 mg) in carbon tetrachloride (100 ml), chlrosulfonic acid (31.5 g, 0.27 mol) was added dropwise at -5~0°C, and stirring was continued for 1 h at room temperature. After reaction, supernatant solvent was decanted, the residue was treated with cold $H_2O$ (250 ml) and filtered to remove any inorganic materials. The filtrate was neutralized with dilute aqueous sodium hydroxide and then the precipitate was filtered off. The resultant filtrate was evaporated to dryness under reduced pressure and the residue was recrystallized from ethanol to give the title compound as a white prisms; yield: 40.0 g.

$^1$HNMR δppm ($CDCl_3$-DMSO-$d_6$): 2.41 (4H, 5, J=7Hz, -$CH_2C\underline{H}_2$CO- x 2), 2.59 (6H, s, Ar-$C\underline{H}_3$ x 2), 2.82 (4H, t, J=7Hz, Ar$C\underline{H}_2CH_2$ x 2), 7.05 (4H, bs, (Ar5-H, 6-H) x 2), 7.75 (2H, s, Ar2-H x 2).

IR (KBr) $\nu cm^{-1}$: 1610.

(2) Synthesis of 4-diazo-1,7-bis(sodium 4-methyl-3-sulfonatophenyl)-3,5-heptanedione

To a solution of 1,7-bis(sodium 4-methyl-3-sulfonatophenyl)-3,5-heptanedione (35.9 g, 70 mmol) obtained in above (1) and triethylamine (7.9 g, 78 mmol) in N,N-dimethylformamide (200 ml) and dichloromethane (40 ml), p-toluenesulfonylazide (15.0 g, 76 mmol) obtained in Example 1 was added dropwise at 0-5°C, and stirring was continued for 4 h at the same temperature. Then the reaction mixture was taken up in dichloromethane (800 ml), the resultant precipitate was filtered, washed with dichloromethane and dried in vacuo to afford the title compound as a pale yellow solid; yield: 21.5 g

$^1$HNMR δppm ($CDCl_3$-DMSO-$d_6$): 2.59 (6H, s, Ar-$C\underline{H}_3$ x 2), 2.86 (4H, t, J=7Hz, -$CH_2C\underline{H}_2$CO-x 2), 3.02 (4H, t, J=7Hz, Ar-$CH_2$- x 2), 7.08 (4H, bs, (Ar5-H, 6-H) x 2), 7.77 (2H, s, Ar6-H x 2).

IR (KBr) $\nu cm^{-1}$: 2150, 1640.

(3) Synthesis of 1,7-bis(3-chlorosulfonyl-4-methylphenyl)-4-diazo-3,5-heptanedione

(compound [IV]; $Y^1=Y^2$=-$CH_3$, $X^1=X^2$=-$SO_2Cl$, m=n=2)

To a solution of 4-diazo-1,7-bis(sodium 4-methyl-3-sulfonatophenyl)-3,5-heptanedione (20.6 g, 40 mmol)

obtained in above (2) in N,N-dimethylformamide (60 ml), thionyl chloride (14.3 g, 120 mmol) was added dropwise at 10-15°C, and stirring was continued for 2 h at room temperature. The reaction mixture was poured into cold $H_2O$ (1 ℓ), extracted with chloroform, the organic extract was washed thrice with $H_2O$, dried over anhydrous $MgSO_4$ and evaporated under reduced pressure. The resultant residue (7 g) was chromatographed on silica gel (Wako Gel C-200, manufactured by Wako Pure Chemical Industries, Ltd.) with n-hexane/ethyl acetate (1:1) as eluent to give the title compound as a pale yellow viscous oil; yield: 4.8 g.

$^1$HNMR, IR and UV spectra of this product were identical with those of the product as described in Example 27, (3).

Example 39

Synthesis of 13-(4-chlorosulfonylphenyl)-3-diazo-2,4-tridecanedione

(Compound [II]; $R^{13}$=-$CH_3$, $X^1$=-$SO_2Cl$, $Y^1$=H, m=9)

(1) Synthesis of 9-carbomethoxynonanoyl chloride

Thionyl chloride (360 g) was added dropwise to methyl hydrogen sebacate (432.6 g, 2 moles) at 60°C with vigorous stirring and stirring was continued for 2 h under reflux. The reaction mixture was evaporated and the residue was distilled under reduced pressure to give the title compound as a colorless oil; yield: 400 g; bp 145-150°C/4 mmHg. (Lit. bp 166-168°C/14 mmHg; T.D. Heyes, J.C. Roberts, J. Chem. Soc., 1952, 4935).

(2) Synthesis of methyl 9-benzoylnonanoate

To a solution of 9-carbomethoxynonanoyl chloride (213.6 g, 0.91 mol) obtained in above (1) in benzene (900 ml), aluminum chloride (153 g) was added in small portions at 5°C or lower with vigorous stirring. The mixture was allowed to warm to reflux, stirring was continued for 1 h under reflux and poured into cold $H_2O$ (1 ℓ). The organic layer was separated, washed thrice with $H_2O$ (500 ml), dried over anhydrous $MgSO_4$ and evaporated under reduced pressure. The residual dark orange oil (200 g) was chromatographed on silica gel (Wako Gel C-200, mfd. by Wako Pure Chemical Industries, Ltd.) with n-hexane/ethyl acetate (4:1) as eluent to give the title compound as a colorless viscous oil, which solidified in the refrigerator; yield: 73.8 g, mp 37.0-38.0°C.

$^1$HNMR δppm ($CDCl_3$): 1.26-1.40 (8H, m, -$C\underline{H}_2$- x 4), 1.59-1.76 (4H, m, -$COCH_2C\underline{H}_2$- and -$C\underline{H}_2CH_2COOCH_3$), 2, 2.30 (2H, t, J=7.3Hz, -$C\underline{H}_2COOCH_3$), 2.96 (2H, t, J=7.3Hz, Ar-$COC\underline{H}_2$-), 3.67 (3H, s, -$COOC\underline{H}_3$), 7.42-7.58 (3H, m, Ar3-H, 4-H, 5-H), 7.96 (2H, d, J=7.3Hz, Ar2-H, 6-H).

IR (KBr) $\nu cm^{-1}$: 2900, 2850, 1740, 1680.

(3) Synthesis of 10-phenyldecanoic acid

To a solution of methyl 9-benzoylnonanoate (18.3 g, 66 mmol) obtained in above (2) in diethyleneglycol (100 ml), 80% hydrazine hydrate (11.6 g) and potassium hydroxide (14.9 g) were added and stirring was continued for 2 h under reflux. The reaction mixture was concentrated, poured into $H_2O$ (200 ml), acidified with dilute hydrochloric acid and extracted with ethyl acetate. The organic extract was washed with $H_2O$, dried over anhydrous $MgSO_4$ and evaporated under reduced pressure. The residue was recrystallized from ligroin to give the title compound as a white leaflets; yield: 14.5 g; mp 44.5-46.5°C. (Lit. mp 46.4-46.6°C; R.Goto, A.Ishizawa, M.Yamamura, Nippon Kagaku Zasshi, 88(6), 678 (1967).).

$^1$HNMR δppm ($CDCl_3$): 1.24-1.31 (10H, m, -$C\underline{H}_2$ x 10), 1.58-1.63 (4H, m, $ArCH_2C\underline{H}_2$ and -$C\underline{H}_2CH_2COOH$), 2.34 (2H, t, J=7.3Hz, -$C\underline{H}_2COOH$), 2.60 (2H, t, J=7.7Hz, Ar-$C\underline{H}_2$-), 7.14-7.19 (3H, m, Ar2-H, 4-H, 6-H), 7.25-7.34 (2H, m, Ar3-H, 5-H).

IR (KBr) $\nu cm^{-1}$: 1670.

(4) Synthesis of methyl 10-phenyldecanoate

To a solution of 10-phenyldecanoic acid (13.7 g, 55 mmol) obtained in above (3) in dichloromethane (100 ml), thionyl chloride (13.1 g) was added dropwise at 40°C, and stirring was continued for 1 h under reflux. The reaction mixture was concentrated, the resultant residue was added dropwise to a solution of triethylamine (11.2 g) and methanol (20 ml) at 20°C or lower, and stirring was continued for 2 h at 20±5°C. The mixture was taken up in $H_2O$ (100 ml), extracted with dichloromethane, the organic layer was washed thrice with $H_2O$ (100 ml), dried over anhydrous $MgSO_4$ and evaporated under reduced pressure. The residue (15.4 g) was purified

by column chromatography on silica gel (Wako Gel C-200) with dichloromethane as eluent to give the title compound as a pale yellow oil; yield: 10.1 g.

$^1$HNMR δppm ($CDCl_3$): 1.24-1.32 (10H, m, $-C\underline{H}_2$ x 5), 1.56-1.61 (4H, m, $ArCH_2C\underline{H}_2$- and $-C\underline{H}_2CH_2COO$-), 2.29 (2H, t, J=7.3Hz, $-C\underline{H}_2COO$-), 2.59 (2H, t, J=7.3Hz, $Ar-C\underline{H}_2$-), 7.16-7.18 (3H, m, Ar2-H, 4-H, 6-H), 7.24-7.30 (2H, m, Ar3-H, 5-H).

IR (Neat) $\nu cm^{-1}$: 1720.

(5) Synthesis of 13-phenyl-2,4-tridecanedione

Using methyl 10-phenyldecanoate (9.2 g, 35 mmol) obtained in above (4) and acetone (2.4 g, 42 mmol), the reaction was carried out in the same manner as described in Example 2, (1), and the crude oil (10.8 g) was chromatographed on silica gel (Wako Gel C-200) with n-hexane/ethyl acetate (10:1) as eluent to give the title compound as a yellow oil which was an ca 3:7 mixture of Keto/Enol as seen by the methylene singlet at δ3.56 ppm and the methine singlet at δ5.48 ppm in the $^1$HNMR spectrum; yield: 1.2 g.

$^1$HNMR δppm ($CDCl_3$): 1.29 (10H, bs, $-C\underline{H}_2$- x 5), 1.59 (4H, bs, $Ar-CH_2C\underline{H}_2$ and $-C\underline{H}_2CH_2COCH_2$-), 2.05 (3H, s, $-COC\underline{H}_3$), 2.26 (2H, t, J=6.5Hz, $-C\underline{H}_2COCH_2COCH_3$), 2.60 (2H, t, J=7.2Hz, $Ar-C\underline{H}_2$-), 3.56 (2H, s, $-COC\underline{H}_2CO$-), 5.48 (1H, s, $\overset{-C}{|}{=}C\underline{H}CO-$), 7.14-7.19 (3H, m, Ar2-H, 4-H, 6-H), 7.25-7.30 (2H, m, Ar3-H, 5-H), 15.51 (1H, s, O$\underline{H}$).

IR (Neat) $\nu cm^{-1}$: 1600.

(6) Synthesis of 3-diazo-13-phenyl-2,4-tridecanedione

Using 13-phenyl-2,4-tridecanedione (1.1 g, 4 mmol) obtained in above (5), the reaction was carried out in the same manner as described in Example 2, (2) and the crude oil (1.4 g) was purified by column chromatography on silica gel (Wako Gel C-200) with dichloromethane as eluent to give the title compound as a yellow viscous oil which solidified when kept overnignt in the refrigerator; yield: 1.0 g; mp 29.0-31.0°C.

$^1$HNMR δppm ($CDCl_3$): 1.29 (10H, bs, $-C\underline{H}_2$- x 5), 1.59-1.64 (4H, m, $Ar-CH_2C\underline{H}_2$- and $-C\underline{H}_2CH_2CO$-), 2.44 (3H, s, $-COC\underline{H}_3$), 2.59 (2H, t, J=7.7Hz, $-C\underline{H}_2CO$-), 2.69 (2H, t, J=7.5Hz, $Ar-C\underline{H}_2$-), 7.16-7.18 (3H, m, Ar2-H, 4-H, 6-H), 7.24-7.30 (2H, m, Ar3-H, 5-H).

IR (Neat) $\nu cm^{-1}$: 2090, 1640.

(7) Synthesis of 13-(4-chlorosulfonylphenyl)-3-diazo-2,4-tridecanedione

(Compound [II]; $R^{13}$=$-CH_3$, $X^1$=$-SO_2Cl$, $Y^1$=H, m=9)

Using 3-diazo-13-phenyl-2,4-tridecanedione (0.88 g, 2.8 mmol) obtained in above (6), the reaction was carried out in the same manner as described in Example 2, (3) and the crude product (300 mg) was purified by column chromatography on silica gel (Wako Gel C-200, manufactured by Wako Pure Chemical Industries, Ltd.) with dichloromethane as eluent to afford the title compound as a pale yellow viscous oil which solidified when kept overnight in the refrigerator; yield: 150 mg; mp 48.5-50.0°C.

$^1$HNMR δppm ($CDCl_3$): 1.30 (10H, bs, $-C\underline{H}_2$- x 5), 1.59-1.64 (4H, m, $Ar-CH_2C\underline{H}_2$- and $-C\underline{H}_2CH_2CO$-), 2.45 (3H, s, $-COC\underline{H}_3$), 2.68-2.75 (4H, m, $ArC\underline{H}_2$ and $-C\underline{H}_2CO$-), 7.41 (2H, d, J=6.6Hz, Ar2-H, 6-H), 7.94 (2H, d, J=6.6Hz, Ar3-H, 5-H).

IR (Neat) $\nu cm^{-1}$: 2090, 1630.

UV ($CH_3CN$) $\lambda_{max}$ nm (log ε): 241.3 (4.41)

Anal. calcd. for $C_{19}H_{25}ClN_2O_4S$: C%, 55.26; H%, 6.10; N%, 6.78.

Found: C%, 55.38; H%, 6.17; N%, 6.69.

Example 40

Synthesis of 16-(4-chlorosulfonylphenyl)-3-diazo-2,4-hexadecanedione

(Compound [II]; $R^{13}$=$-CH_3$, $X^1$=$-SO_2Cl$, $Y^1$=H, m=12)

(1) Synthesis of methyl 10-oxo-13-phenyltridecanoate

To a suspention of magnesium (turnings, 17.1 g) in ethyl ether (300 ml), a solution of β-phenethyl bromide (140 g, 0.7 mol) in ethyl ether was added dropwise under mild reflux with vigorous stirring under nitrogen, and stirring was continued for 1 h under reflux. After cooling, the mixture (Grignard reagent) was added dropwise to a solution of 9-carbomethoxynonanoyl chloride (150.1 g, 0.64 mol) in ethyl ether (500 ml) at -50~-30°C. The mixture was allowed to warm to room temperature, stirred for 2 h at the same temperature and poured into $H_2O$ (500 ml). The organic layer was separated, washed with $H_2O$, dried over anhydrous $MgSO_4$ and evaporated in vacuo. The residue (203 g) was chromatographed on silica gel (Wako Gel C-200, manufactured by Wako Pure Chemical Industries, Ltd.) with n-hexane/ethyl acetate (4:1) as eluent to afford the title compound as a colorless oil; yield: 99.7 g.

$^1$HNMR δppm ($CDCl_3$): 1.23-1.30 (8H, m, -C$\underline{H_2}$- x 4), 1.51-1.70 (4H, m, $-CH_2COCH_2C\underline{H_2}$ and $-C\underline{H_2}CH_2COO-$), 1.81-2.01 (2H, m, Ar-$CH_2C\underline{H_2}$-), 2.30 (2H, t, J=7.3Hz, $-C\underline{H_2}COOCH_3$), 2.36-2.43 (4H, m, $-C\underline{H_2}$ $COC\underline{H_2}-$), 2.53-2.72 (2H, m, Ar-$C\underline{H_2}$-), 3.66 (3H, s, $-COOC\underline{H_3}$), 7.13-7.31 (5H, m, ArH).

IR (Neat) $\nu cm^{-1}$: 2950, 2800, 1730, 1710.

(2) Synthesis of 13-phenyltridecanoic acid

To a solution of methyl 10-oxo-13-phenyltridecanoate (56.8 g, 0.178 mol) obtained in above (1) in diethylene glycol (600 ml), 80% hydrazine hydrate (50 ml) and potassium hydroxide (70 g) were added and stirring was continued for 2 h under reflux. The reaction mixture was concentrated, poured into $H_2O$ (740 ml), acidified with dilute hydrochloric acid and extracted with chloroform. The organic extract was washed with $H_2O$, dried over anhydrous MgSO4 and evaporated in vacuo. The residue was recrystallized from ligroin to afford the title compound as a white leaflets; yield: 44.4 g; mp 51.5-52.5°C. (Lit. mp 52.2-52.5°C; A. Ishizawa, M.Yamamura, R. Goto, Nippon Kagaku Zasshi, 89(8), 815 (1968).).

$^1$HNMR δppm (DMSO-$d_6$): 1.24-1.33 (16H, m, $-C\underline{H_2}$ x 8), 1.47-1.58 (4H, m, $ArCH_2C\underline{H_2}$- and $-C\underline{H_2}CH_2$ COOH), 2.18 (2H, t, J=7.3Hz, $-C\underline{H_2}COOH$), 2.48-2.58 (2H, m, $ArC\underline{H_2}$-), 7.11-7.28 (5H, m, ArH), 11.89 (1H, bs, -COO$\underline{H}$).

IR (KBr) $\nu cm^{-1}$: 2900, 2850, 1680.

(3) Synthesis of methyl 13-phenyltridecanoate

13-Phenyltridecanoic acid (22.4 g, 77 mmol) was reacted for 1 h in methanol (60 ml) and sulfuric acid (2.2 g) under reflux. The reaction mixture was concentrated, poured into $H_2O$ (100 ml) and extracted with dichloromethane. The organic layer was washed with $H_2O$, dried over anhydrous $MgSO_4$ and evaporated under reduced pressure. The residue (33 g) was chromatographed on silica gel (Wako Gel C-200) with dichloromethane as eluent to give the title compound as a white solid; yield: 17.6 g; mp 30.5-31.5°C.

$^1$HNMR δppm ($CDCl_3$): 1.25-1.29 (16H, m, $-C\underline{H_2}$ x 8), 1.56-1.63 (4H, m, $-C\underline{H_2}CH_2COOH_3$ and Ar-$CH_2C\underline{H_2}$-), 2.30 (2H, t, J=7.5 Hz, $-C\underline{H_2}COOCH_3$), 2.60 (2H, t, J=7.8Hz, Ar-$C\underline{H_2}$-), 3.66 (3H, s, $-COOC\underline{H_3}$), 7.16-7.18 (3H, m, Ar3-H, 4-H, 5-H), 7.24-7.29 (2H, m, Ar2-H, 6-H).

IR (KBr) $\nu cm^{-1}$: 2900, 2850, 1730.

(4) Synthesis of 16-phenyl-2,4-hexadecanedione

Using methyl 13-phenyltridecanoate (15.9 g, 55 mmol) obtained in above (3) and acetone (3.8 g, 66 mmol), the reaction was carried out in the same procedure as described in Example 2, (1) and the crude oil was purified by column chromatography on silica gel (Wako Gel C-200) with n-hexane/ethyl acetate (10:1) as eluent to give the title compound as a yellow oil which was an ca 3:7 mixture of Keto/Enol as seen by the methylene singlet at δ3.55 ppm and the methine singlet at δ5.47 ppm in the $^1$HNMR spectrum; yield: 1.8 g.

$^1$HNMR δppm ($CDCl_3$): 1.29 (16H, bs, $-C\underline{H_2}$- x 8), 1.56-1.63 (4H, m, $-C\underline{H_2}CH_2CO$- and Ar-$CH_2C\underline{H_2}$-), 2.05 (3H, s, $-COC\underline{H_3}$), 2.26 (2H, t, J=6.5Hz, $-C\underline{H_2}COCH_2COCH_3$), 2.60 (2H, t, J=7.2Hz, Ar-$C\underline{H_2}$-), 3.55 (2H, s, $-COC\underline{H_2}CO-$),

5.47 (1H, s, $\overset{-C}{|}$=C$\underline{H}$CO-), 7.14-7.19 (3H, m, Ar2-H, 4-H, 6-H), 7.25-7.30 (2H, m, Ar3-H, 5-H), 15.51 (1H, s, O$\underline{H}$).

IR (Neat) $\nu cm^{-1}$: 1605 $cm^{-1}$ (C=O).

(5) Synthesis of 3-diazo-16-phenyl-2,4-hexadecanedione

Using 16-phenyl-2,4-hexadecanedione (1.5 g, 4.5 mmol) obtained in above (4), the reaction was carried out in the same procedure as described in Example 2, (2) and the crude oil was chromatographed on silica gel (Wako Gel C-200) with dichloromethane as eluent to give the title compound as a yellow viscous oil; yield: 1.3 g.

$^1$HNMR $\delta$ppm ($CDCl_3$): 1.29 (16H, bs, $-C\underline{H}_2-$ x 8), 1.59-1.63 (4H, m, $ArCH_2C\underline{H}_2-$ and $-C\underline{H}_2CH_2CO-$), 2.44 (3H, s, $-COC\underline{H}_3$), 2.59 (2H, t, J=7.5Hz, $-C\underline{H}_2CO-$), 2.70 (2H, t, J=7.5Hz, $ArC\underline{H}_2-$), 7.16-7.18 (3H, m, Ar2-H, 4-H, 6-H), 7.24-7.30 (2H, m, Ar3-H, 5-H).

IR (Neat) $\nu cm^{-1}$: 2090, 1640.

(6) Synthesis of 16-(4-chlorosulfonylphenyl)-3-diazo-2,4-hexadecadione

(Compound [II]; $R^{13}$=$-CH_3$, $X^1$=$-SO_2Cl$, $Y^1$=H, m=12)

Using 3-diazo-16-phenyl-2,4-hexadecanedione (1.1 g, 3 mmol) obtained in above (5), the reaction was carried out in the same procedure as described in Example 2,(3) and the crude oil (300 mg) was chromatographed on silica gel (Wako Gel C-200, manufactured by Wako Pure Chemical Industries, Ltd.) with dichloromethane as eluent to afford the title compound as a pale yellow viscous oil; yield: 140 mg.

$^1$HNMR $\delta$ppm ($CDCl_3$): 1.30 (16H, bs, $-C\underline{H}_2$ x 8), 1.59-1.63 (4H, m, $Ar-CH_2C\underline{H}_2-$ and $-C\underline{H}_2CH_2CO-$), 2.45 (3H, s, $-COC\underline{H}_3$), 2.68-2.75 (4H, m, $Ar-C\underline{H}_2-$ and $-C\underline{H}_2CO-$), 7.41 (2H, d, J=6.6Hz, Ar2-H, 6-H), 7.95 (2H, d, J=6.6Hz, Ar3-H, 5-H).

IR (Neat) $\nu cm^{-1}$: 2090, 1635.

Example 41

Synthesis of 5-(4-chlorosulfonylphenyl)-1-cyclohexyl-2-diazo-1,3-pentanedione

(Compound [II]; $R^{13}$=Cyclohexyl, $X^1$=$-SO_2Cl$, $Y^1$=H, m=2)

(1) Synthesis of ethyl cyclohexanecarboxylate

To a solution of ethanol (45 ml) and triethylamine (78 g) in toluene (120 ml), cyclohexane carbonyl chloride (75 g, 0.51 mol) was added dropwise at 15°C or lower, and stirring was continued for 3 h at room temperature. The reaction mixture was poured into cold $H_2O$ (350 ml), acidified with dilute hydrochloric acid and extracted with ethyl acetate. The organic extract was washed thrice with $H_2O$ (150 ml), dried over anhydrous $Na_2SO_4$ and the solvent was removed in vacuo. The residue (93 g) was distilled under reduced pressure to give the title compound as a colorless oil; yield: 64 g; bp 101-102°C/35 mmHg (Lit: bp 194°C/~760 torr: G.A. Olah, T. Keumi, D. Meider, Synthesis, 1978, 929.).

$^1$HNMR $\delta$ppm ($CDCl_3$): 1.13-1.92 (13H, m, cyclohexyl 2-H, 2′-H, 3-H, 3′-H, 4-H, 4′-H, 5-H, 5′-H, 6-H, 6′-H and $-COOCH_2C\underline{H}_3$), 2.22-2.35 (1H, m, cyclohexyl 1-H), 4.11 (2H, g, J=7Hz, $-COOC\underline{H}_2CH_3$).

IR (Neat) $\nu cm^{-1}$: 1725

(2) Synthesis of 1-cyclohexyl-5-phenyl-1,3-pentanedione

Using ethyl cyclohexane carboxylate (63 g, 0.4 mol) and 4-phenyl-2-butanone (20 g, 0.13 mol), the reaction was carried out in the same manner as described in Example 2, (1) and crude dark orange oil (23 g) was chromatographed on silica gel (Wako Gel C-200) eluting with n-hexane/ethyl acetate (100:1 $\rightarrow$ 100:2 $\rightarrow$ 100:3) to afford the title compound as a yellow oil which was an ca 3:17 mixture of Keto/Enol by the methylene singlet at $\delta$3.55 ppm and the methine singlet at $\delta$5.45 ppm in the $^1$HNMR spectrum; yield: 2.5 g.

$^1$HNMR $\delta$ppm ($CDCl_3$): 1.05-1.89 (10H, m, cyclohexyl 2-H, 2′-H, 3-H, 3′-H, 4-H, 4′-H, 5-H, 5′-H, 6-H, 6′-H), 2.20-2.37 (1H, m, cyclohexyl 1-H), 2.65 (2H, t, J=7Hz, $-CH_2C\underline{H}_2CO-$), 2.90 (2H, t, J=7Hz, $Ar-C\underline{H}_2-$), 3.55

(2H, s, $-COC\underline{H}_2CO-$), 5.45 (1H, s, $\overset{-C}{|}=C\underline{H}CH-$), 7.15-7.27 (5H, m, ArH), 15.58 (1H, bs, $O\underline{H}$).

IR (Neat) $\nu cm^{-1}$: 1710.

(3) Synthesis of 1-cyclohexyl-2-diazo-5-phenyl-1,3-pentanedione

Using 1-cyclohexyl-5-phenyl-1,3-pentanedione (2.4 g, 9.3 mmol) obtained in above (2), the reaction was carried out in the same manner as described in Example 2, (2) and the crude yellow oil g) was purified by column chromatography on silica gel (Wako Gel C-200) with n-hexane/ethyl acetate (from 100:1 to 100:2) as eluent to give the title compound as a pale yellow viscous oil; yield: 1.9 g).

$^1$HNMR $\delta$ppm ($CDCl_3$): 1.18-1.53 (6H, m, cyclohexyl 3-H, 3′-H, 4-H, 4′-H, 5-H, 5′-H), 1.68-1.82 (4H, m, cyclohexyl 2-H, 2′-H, 6-H, 6′-H), 2.83-3.00 (3H, m, cyclohexyl 1-H and $-CH_2C\underline{H}_2CO-$), 3.05-3.11 (2H, m, Ar-$C\underline{H}_2$-), 7.17-7.31 (5H, m, ArH).

IR (Neat) $\nu cm^{-1}$: 2080, 1630.

(4) Synthesis of 5-(4-chlorosulfonylphenyl)-1-cyclohexyl-2-diazo-1,3-pentanedione

(Compound [II]; $R^{13}$=cyclohexyl, $X^1$=$-SO_2Cl$, $Y^1$=H, m=2)

Using 1-cyclohexyl-2-diazo-5-phenyl-1,3-pentanedione (1.7 g, 6 mmol) obtained in above (3), the reaction was carried out in the same manner as described in Example 2, (3) and the crude oil (400 mg) was chromatographed on silica gel (Wako Gel C-200, manufactured by Wako Pure Chemical Industries, Ltd.) eluting with n-hexane/ethyl acetate (3:1) to afford the title compound as a pale yellow viscous oil; yield: 200 mg.

$^1$HMMR $\delta$ppm ($CDCl_3$): 1.18-1.64 (6H, m, cyclohexyl 3-H, 3′-H, 4-H, 4′-H, 5-H, 5′-H), 1.67-1.90 (4H, m, cyclohexyl 2-H, 2′-H, 6-H, 6′-H), 2.65-2.78 (1H, m, cyclohexyl 1-H), 3.09 (2H, t, J=7Hz, $-CH_2C\underline{H}_2CO-$), 3.20 (2H, t, J=7Hz, $ArC\underline{H}_2-$), 7.49 (2H, d, J=8Hz, Ar2-H, 6-H), 7.95 (2H, d, J=8Hz, Ar3-H, 5-H).

IR (Neat) $\nu cm^{-1}$: 2100, 1640.

Example 42

Synthesis of 2-[2-(4-chlorosulfonylphenyl)ethyl]-5-diazo-2-methyl-1,3-dioxane-4,6-dione

(Compound [VII]; $R^9$=$-CH_3$, $X^3$=$-SO_2Cl$, $Y^3$=H, p=2)

(1) Synthesis of 2-methyl-2-(2-phenylethyl)-1,3-dioxane-4,6-dione

To a solution of malonic acid (56.7 g, 0.55 mol) and acetic anhydride (73 ml), sulfuric acid (2 ml) was added dropwise at 5-10°C and stirring was continued for 20 min at the same temperature. To this mixture, 4-phenyl-2-butanone (105 g, 0.7 mol) was added dropwise at 15-20°C and stirring was continued for 15 h at the same temperature. The reaction mixture was taken up into $H_2O$, extracted with chloroform, then the organic extract was washed with $H_2O$, dried over anhydrous $MgSO_4$ and evaporated. The resultant solid was recrystallized from aqueous acetone to give the title compound as a white amorphous solid; yield: 68.0 g; mp. 63.0-65.0°C.

$^1$HNMR $\delta$ppm ($CDCl_3$): 1.74 (3H, s, $C\underline{H}_3$), 2,03-2.44 (2H, m, Ar-$CH_2C\underline{H}_2$-), 2.60-3.02 (2H, m, Ar-$C\underline{H}_2CH_2$-), 3.58 (2H, s, $-COC\underline{H}_2CO-$), 7.16 (5H, s, Ar-H).

IR (KBr) $\nu cm^{-1}$: 1750.

(2) Synthesis of 5-diazo-2-methyl-2-(2-phenylethyl)-1,3-dioxane-4,6-dione

To a solution of 2-methyl-2-(2-phenylethyl)-1,3-dioxane-4,6-dione (67 g, 0.29 mol) obtained in above (1) and triethylamine (32 g) in ethanol (150 ml), p-toluenesulfonylazide (61 g, 0.3 mol) obtained in Example 1 was added dropwise at -15~-10°C, and stirring was continued for 45 min at the same temperature. The reaction mixture was extracted with dichloromethane, the organic layer was washed with $H_2O$, dried over anhydrous $MgSO_4$ and evaporated under reduced pressure. The resultant residue was chromatographed on silica gel (Wako Gel C-200) with n-hexane/ethyl acetate (10:1 $\rightarrow$ 5:1 $\rightarrow$ 3:1) as eluent to give the title compound as a yellow crystals; yield: 44.0 g; mp. 50.5-51.0°C.

$^1$HNMR $\delta$ppm ($CDCl_3$): 1.74 (3H, s, $C\underline{H}_3$), 2.04-2.47 (2H, m, Ar-$CH_2C\underline{H}_2$-), 2.60-3.02 (2H, m, Ar-$C\underline{H}_2CH_2$ ), 7.16 (5H, s, Ar-H).

IR (KBr) $\nu cm^{-1}$: 2180, 1695.

(3) Synthesis of 2-[2-(4-chlorosulfonylphenyl)ethyl]-5-diazo-2-methyl-1,3-dioxane-4,6-dione

Compound [VII]; $R^9$=$-CH_3$, $X^3$=$-SO_2Cl$, $Y^3$=H, p=2)

To a solution of 5-diazo-2-methyl-2-(2-phenylethyl)-1,3-dioxane-4,6-dione (8.0 g, 31 mmol) obtained in above (2) in chloroform (35 ml), chlorosulfonic acid (21.5 g) was added dropwise at -20~-10°C. The mixture was stirred for 45 min at the same temperature, poured into cold $H_2O$ and extracted with chloroform. The organic extract was washed with $H_2O$, dried over anhydrous $MgSO_4$ and evaporated. The residue was chromatographed on silica gel (Wako Gel C-200, manufactured by Wako Pure Chemical Industries, Ltd.) with n-hexane/ethyl acetate (10:1 → 4:1 → 1:1) as eluent to give the title compound as a white amorphous solid; yield: 1.6 g; mp 153.5-156.5°C.

$^1$HNMR δppm ($CDCl_3$): 1.82 (3H, s, $C\underline{H_3}$), 2.13-2.56 (2H, m, Ar-$CH_2C\underline{H_2}$-), 2.73-3.19 (2H, m, Ar$C\underline{H_2}CH_2$ ), 7.44 (2H, d, J=9Hz, Ar2-H, 6-H), 7.97 (2H, d, J=9Hz, Ar3-H, 5-H).

IR (KBr) $\nu cm^{-1}$: 2170, 1710.

Anal. calcd. for $C_{13}H_{11}ClN_2O_6S$: C%, 43.52; H%, 3.09; N%, 7.81.

Found: C%, 43.66; H%, 3.01; N%, 7.69.

## Example 43

Synthesis of 1,7-bis(4-chloro-3-chlorosulfonylphenyl)-4-diazo-3,5-heptanedione

(Compound [IV], $Y^1$=$Y^2$=- Cl, $X^1$=$X^2$= - $SO_2Cl$, m=2, n=2)

(1) Synthesis of 1,7-bis(4-chlorophenyl)-3,5-heptanedione

Using 2,4-pentanedione (20.0 g, 0.2 mol) and 4-chlorobenzyl chloride (74.7 g), the reaction was carried out in the same manner as described in Example 17, (1), and the residual orange red semisolid (69 g) was recrystallized from methanol to give the title compound as s white prisms which was an ca 3:17 mixture of Keto/Enol as seen by the methylene singlet at δ3.51 ppm and the methine singlet at δ5.37 ppm in the $^1$HNMR spectrun; yield: 24.7 g; mp 74.6-76.1°C.

$^1$HNMR δppm ($CDCl_3$): 2.55 (4H, t, J=7.3Hz, Ar-$CH_2C\underline{H_2}$CO- x 2). 2.88 (4H, t, J=7.3Hz, Ar-$C\underline{H_2}$ x 2), 3.51 (2H, s, -CO$C\underline{H_2}$CO-), 5.37 (1H, s, $\overset{-C}{|}=C\underline{H}-CO-$), 7.07-7.11 (4H, m, (Ar2-H, 6-H) x 2), 7.23-7.26 (4H, m, (Ar3-H, 5-H) x 2), 15.35 (1H, s, -O$\underline{H}$).

IR (KBr) $\nu cm^{-1}$: 3300, 1640, 1600.

(2) Synthesis of 1,7-bis(4-chlorophenyl)-4-diazo-3,5-heptanedione

Using 1,7-bis(4-chlorophenyl)-3,5-heptanedione (12.0 g, 34.4 mmol) obtained in above (1), the reaction was carried out in the same manner as described in Example 17, (2), and the residue (15 g) was purified by column chromatography on silica gel (Wako Gel C-200) with dichloromethane as eluent to give the title compound as a pale yellow crystals; yield: 11.2 g; mp 83°C (dec.).

$^1$HNMR δppm ($CDCl_3$); 2.93-3.01 (8H, m, Ar-$C\underline{H_2}C\underline{H_2}$CO-x 2), 7.12-7.16 (4H, m, (Ar2-H, 6-H) x 2), 7.24-7.27 (4H, m, (Ar3-H, 5-H) x 2).

IR (KBr) $\nu cm^{-1}$: 2100, 1650.

UV ($CH_3CN$) $\lambda_{max}$ nm (log ε): 225.0 (4.50)

(3) Synthesis of 1,7-bis(4-chloro-3-chlorosulfonylphenyl)-4-diazo-3,5-heptanedione

(Compound [IV], $Y^1$=$Y^2$=-Cl, $X^1$=$X^2$=$-SO_2Cl$, m=2, n=2)

Using 1,7-bis(4-chlorophenyl)-4-diazo-3,5-heptanedione (3.98 g, 10.6 mmol) obtained in above (2), the reaction was carried out in the same manner as described in Example 17, (3), and the residue (1.3 g) was chromatographed on silica gel (Wako Gel C-200, manufactured by Wako Pure Chemical Industries, Ltd.) with dichloromethane as eluent to give the title compound as a pale yellow viscous oil; yield: 1.1 g.

$^1$HNMR δppm ($CDCl_3$): 3.08 (8H, s, -C$\underline{H}_2$- x 4), 7.55 (4H, s, (Ar5-H, 6-H) x 2), 7.99 (2H, s, Ar2-H x 2).
IR (Neat) $\nu cm^{-1}$: 2110, 1655.
UV ($CH_3CN$) $\lambda_{max}$ nm (log ε): 227.0 (4.53), 287.0 (3.75).
Anal. calcd. for $C_{19}H_{14}Cl_4N_2O_6S_2$: C%, 39.88; H%, 2.47; N%, 4.90.
Found: C%, 40.09; H%, 2.31; N%, 4.82.

Example 44

Synthesis of 1,7-bis(3-chlorosulfonyl-4-methoxyphenyl)-4-diazo-3,5-heptanedione

(Compound [IV], $Y^1$=$Y^2$-$CH_3O$, $X^1$=$X^2$=-$SO_2Cl$, m=2, n=2)

(1) Synthesis of 1,7-bis(4-methoxyphenyl)-3,5-heptanedione

Using 2,4-pentanedione (37.3 g, 0.37 mol) and 4-methoxybenzyl chloride (133 g), the reaction was carried out in the same manner as described in Example 17, (1), and the solid residue was purified by recrystalization from ethanol to give the title compound as pale yellow microneedles which were not shown any methylene singlet at δ3.0-4.0 ppm in the $^1$HNMR spectrum; yield: 95.1 g; mp 73.5-76.2°C.

$^1$HNMR δppm ($CDCl_3$): 2.64 (4H, t, J=8Hz, Ar-$CH_2$C$\underline{H}_2$CO- x 2), 2.86 (4H, t, J=8Hz, Ar-C$\underline{H}_2$- x 2), 3.78 (6H, s, C$\underline{H}_3$CO- x 2), 5.41 (1H, s, $\overset{-C}{|}$=CH-CO-), 6.82 (4H, d, J=9Hz, (Ar2-H, 6-H) x 2), 7.09 (4H, d, J=9Hz, (Ar3-H, 5-H) x 2), 15.43 (1H, bs, -O$\underline{H}$).
IR (KBr) $\nu cm^{-1}$: 1600.

(2) Synthesis of 1,7-bis(4-methoxyphenyl)-4-diazo-3,5-heptanedione

Using 1,7-bis(4-methoxyphenyl)-3,5-heptanedione (4.5 g, 13.2 mmol) obtained in above (1), the reaction was carried out in the same procedure as described in Example 17, (2), and the dark brown oily residue (5.6 g) was purified by silica gel column chromatograpy (Wako Gel C-200) with dichloromethane as eluent to afford the title compound as a yellow viscous oil; yield: 2.4 g.

$^1$HNMR δppm ($CDCl_3$): 2.87-3.03 (8H, m, -C$\underline{H}_2$- x 4), 3.78 (6H, s, C$\underline{H}_2$O- x 2), 6.82 (4H, d, J=8.6Hz, (Ar3-H, 5-H) x 2), 7.13 (4H, d, J=8.6Hz, (Ar2-H, 6-H) x 2).
IR (Neat) $\nu cm^{-1}$: 2900, 2100, 1650.

(3) Synthesis of 1,7-bis(3-chlorosulfonyl-4-methoxyphenyl)-4-diazo-3,5-heptanedione

(Compound [IV], $Y^1$=$Y^2$=-$CH_3O$, $X^1$=$X^2$=-$SO_2Cl$, m=2, n=2)

Using 1,7-bis(4-methoxyphenyl)-4-diazo-3,5-heptanedione (2.2 g, 6 mmol) obtained in above (2), the reaction was carried out in the same manner as described in Example 17, (3), and the residue (1.8 g) was purified by column chromatography on silica gel (Wako Gel C-200, manufactured by Wako Pure Chemical Industries, Ltd.) with n-hexane/ethyl acetate (1:1) as eluent to give the title compound as a pale yellow amorphous solid; yield: 1.7 g; mp 51.0-52.0°C.

$^1$HNMR δppm ($CDCl_3$): 2.99-3.05 (8H, m, -C$\underline{H}_2$- x 4), 4.03 (6H, s, C$\underline{H}_3$O- x 2), 7.06 (2H, d, J=8.8Hz, Ar5-H x 2), 7.57 (2H, dd. J=8.8Hz and 2.2Hz, Ar6-H x 2), 7.79 (2H, d, J=2.2Hz, Ar2-H x 2).
IR (KBr) $\nu cm^{-1}$: 2100, 1650.
UV ($CH_3CN$) $\lambda_{max}$ nm (log ε): 228.0 (4.54), 306.0 (3.88).
Anal. calcd. for $C_{21}H_{20}Cl_2N_2O_8S_2$: C%, 44.77; H%, 3.58; N%, 4.97.
Found: C%, 44.80; H%, 3.51; N%, 5.07.

Example 45

A photosensitive composition having the following composition was prepared:

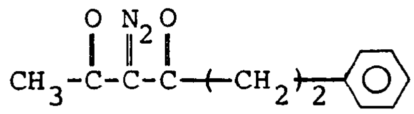 1.5 g

Poly(p-vinylphenyl) (m.v. 7000) 8.5 g

Diethylene glycol dimethyl ether 30 g

The photosensitive composition was spin coated on a semiconductor substrate (Si substrate) in 1.0 μm thickness and prebaked at 90°C using a hot plate for 2 minutes. Then, a dissolving rate in an alkali developing solution [a 2.38% aqueous solution of tetramethylammonium hydroxide (TMAH)] was measured. The dissolving rate was 400 nm/min, which value was 1/10 of that of poly(p-vinylphenol)(4000 nm/min.). This means that the photosensitive compound mentioned above has a function of lowering the dissolving rate in the alkali developing solution.

A pattern was formed using the photosensitive composition as shown in Fig. 1. A layer 2 of the photosensitive composition was formed by spin coating on a substrate 1 such as a semiconductor in 1.0 μm thickness [Fig. 1(a)]. The substrate 1 may have thereon an oxidizing film, an insulating film, and an electroconductive film. The layer 2 was selectively exposed to KrF excimer laser 4 via a mask 5 [Fig. 1(b)]. Finally, the layer 2 was dipped in a 2.38% TMAH aqueous solution for 1 minutes to form a pattern 2a by dissolving and removing the exposed portions [Fig. 1(c)]. The resulting pattern 2a had a film erosion of about 40%, an aspect ratio of 70 degree and resolution of 0.4 μm lines and spaces.

Example 46

Photosensitive compositions comprising a photosensitive compound of the formula (I) as listed in Table 1, poly(p-vinylphenol) as a polymer and diethylene glycol dimethyl ether as a solvent, in amounts as listed in Table 1 were prepared.

Each photosensitive composition was subjected to the same dissolving test and pattern formation as in Example 45.

The dissolving rate, the film erosion, the aspect ratio and the resolution were measured in the same manner as in Example 45 and listed in Table 1.

Table 1

| Run No. | Compound (I) | Mixing ratio (parts) Compound (I) | Polymer | Solvent | Dissolving rate (nm/min) | Film erosion (%) | Aspect ratio (degree) | Resolution (lines and spaces) (μm) |
|---|---|---|---|---|---|---|---|---|
| 1 | 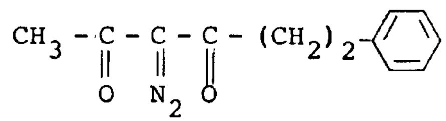 | 15 | 85 | 300 | 400 | 40 | 70 | 0.4 |
| 2 | 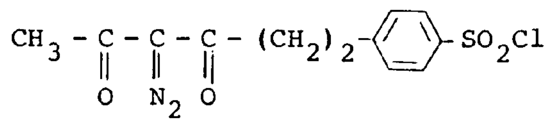 | " | " | " | 150 | 15 | 82 | 0.4 |
| 3 | 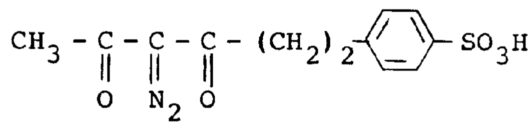 | " | " | " | 200 | 30 | 80 | 0.4 |
| 4 | 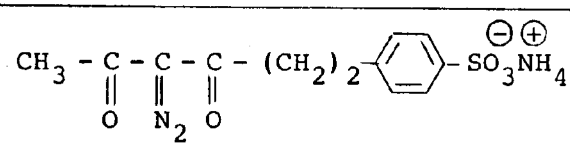 | " | " | " | 200 | 30 | 80 | 0.4 |
| 5 | 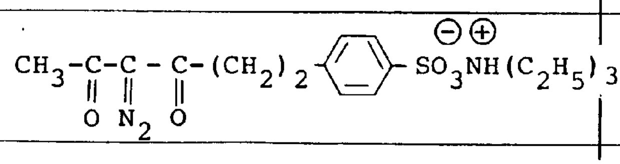 | " | " | " | 200 | 30 | 80 | 0.4 |

- Cont'd -

Table 1 (Cont'd)

| | | | | | | | | |
|---|---|---|---|---|---|---|---|---|
| 6 | 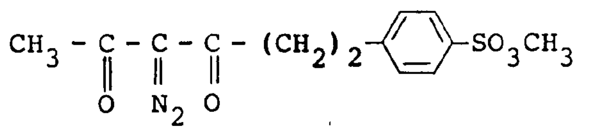 | 15 | 85 | 300 | 200 | 30 | 80 | 0.4 |
| 7 | 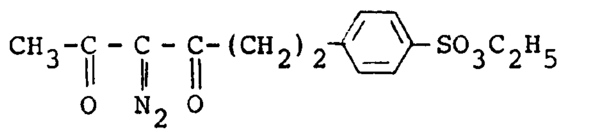 | " | " | " | 170 | 25 | 81 | 0.4 |
| 8 | 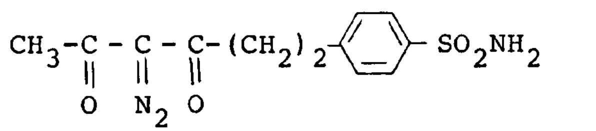 | " | " | " | 200 | 25 | 80 | 0.4 |
| 9 | 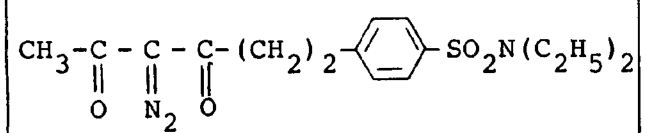 | " | " | " | 180 | 20 | 81 | 0.4 |
| 10 | 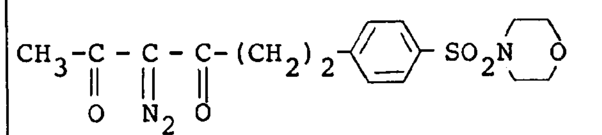 | " | " | " | 200 | 25 | 80 | 0.4 |
| 11 | 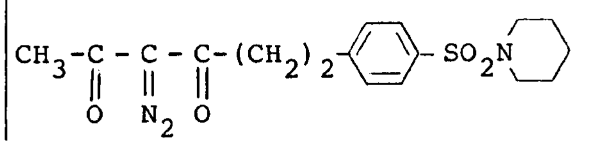 | " | " | " | 200 | 25 | 80 | 0.4 |

- Cont'd -

Table 1 (Cont'd)

| | | | | | | | | |
|---|---|---|---|---|---|---|---|---|
| 12 | 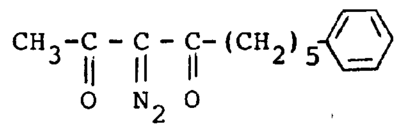 | 15 | 85 | 300 | 120 | 12 | 83 | 0.4 |
| 13 | 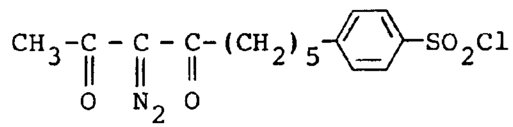 | " | " | " | 80 | 10 | 85 | 0.4 |
| 14 | 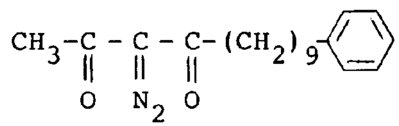 | 12 | 88 | 300 | 100 | 10 | 82 | 0.4 |
| 15 | 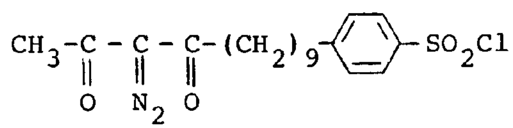 | " | " | " | 80 | 8 | 85 | 0.4 |
| 16 | 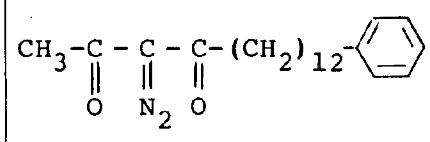 | 10 | 90 | 300 | 80 | 8 | 85 | 0.4 |
| 17 | 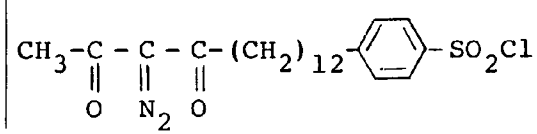 | " | " | " | 50 | 5 | 88 | 0.4 |

- Cont'd -

Table 1 (Cont'd)

| | | | | | | | | |
|---|---|---|---|---|---|---|---|---|
| 18 | 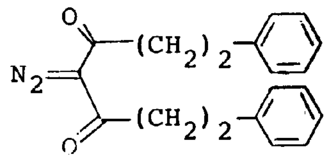 | 15 | 85 | 300 | 300 | 30 | 75 | 0.4 |
| 19 | 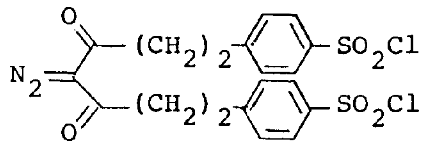 | " | " | " | ≦10 | 0 | 86 | 0.4 |
| 20 | 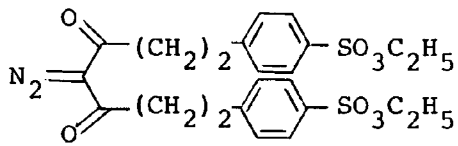 | " | " | " | 150 | 15 | 82 | 0.4 |
| 21 | 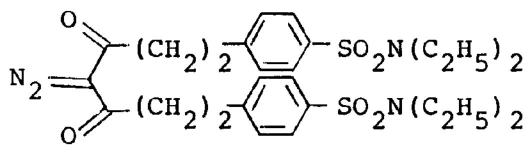 | " | " | " | 150 | 15 | 82 | 0.4 |

- Cont'd -

Table 1 (Cont'd)

| | | | | | | | | |
|---|---|---|---|---|---|---|---|---|
| 22 | 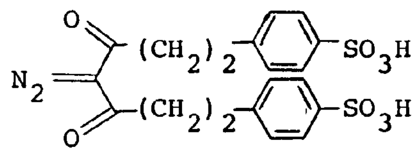 | 15 | 85 | 300 | 150 | 15 | 82 | 0.4 |
| 23 | 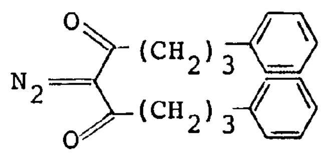 | 12 | 88 | 300 | 150 | 15 | 82 | 0.4 |
| 24 | 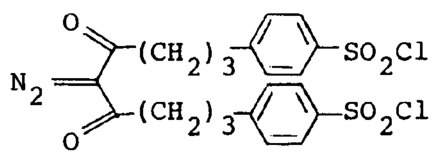 | " | " | " | ≤10 | 0 | 88 | 0.4 |
| 25 | 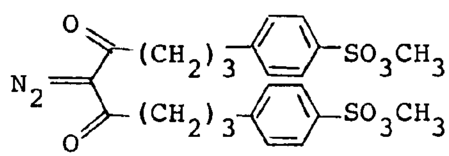 | " | " | " | 90 | 10 | 84 | 0.4 |

- Cont'd -

Table 1 (Cont'd)

| | | | | | | | | |
|---|---|---|---|---|---|---|---|---|
| 26 | 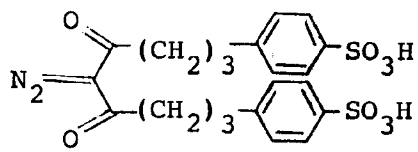 | 12 | 88 | 300 | 90 | 10 | 84 | 0.4 |
| 27 | 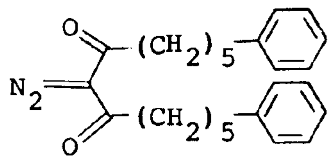 | 10 | 90 | 300 | 100 | 10 | 80 | 0.4 |
| 28 | 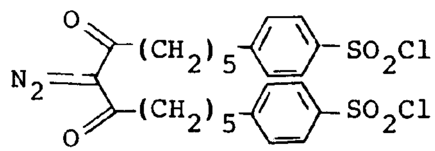 | " | " | " | ≦10 | 0 | 90 | 0.4 |
| 29 | 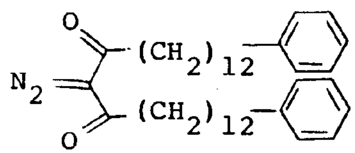 | " | " | " | ≦10 | 0 | 90 | 0.4 |

- Cont'd -

Table 1 (Cont'd)

| | | | | | | | | |
|---|---|---|---|---|---|---|---|---|
| 30 | 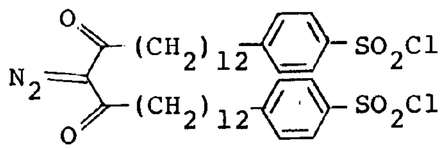 | 6 | 94 | 300 | ≦10 | 0 | 90 | 0.4 |
| 31 | 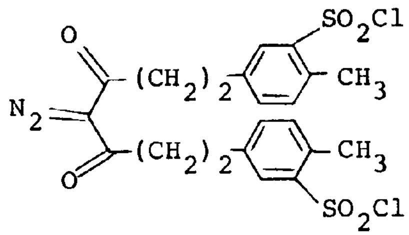 | 15 | 85 | 300 | ≦10 | 0 | 90 | 0.4 |
| 32 | 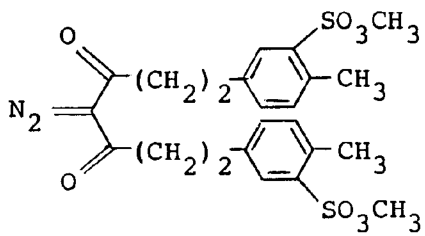 | " | " | " | 100 | 10 | 85 | 0.4 |
| 33 | 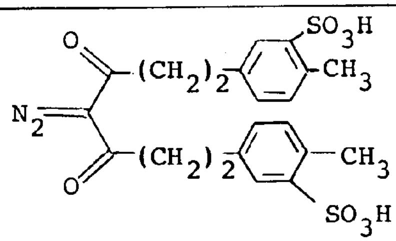 | " | " | " | 100 | 10 | 85 | 0.4 |

- Cont'd -

Table 1 (Cont'd)

| | | | | | | | | |
|---|---|---|---|---|---|---|---|---|
| 34 | 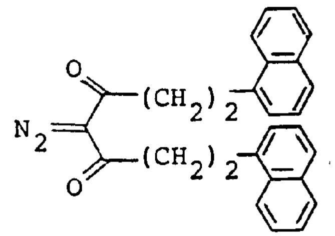 | 10 | 90 | 300 | 80 | 10 | 83 | 0.4 |
| 35 | 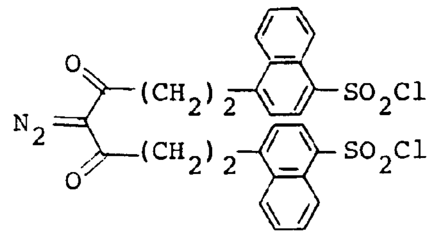 | " | " | " | ≦10 | 0 | 90 | 0.4 |
| 36 | 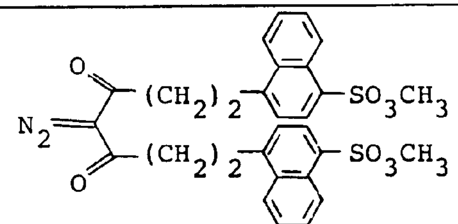 | " | " | " | 50 | 5 | 84 | 0.4 |
| 37 | 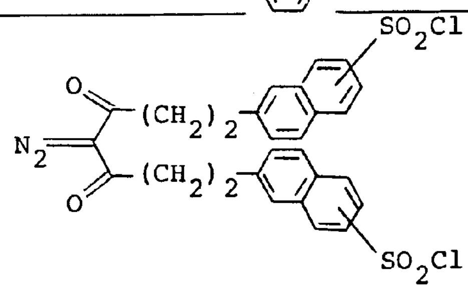 | " | " | " | ≦10 | 0 | 90 | 0.4 |

- Cont'd -

Table 1 (Cont'd)

| | | | | | | | | |
|---|---|---|---|---|---|---|---|---|
| 38 | 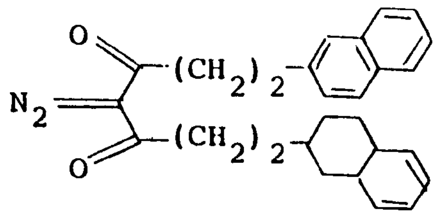 | 10 | 90 | 300 | 80 | 10 | 83 | 0.4 |
| 39 | 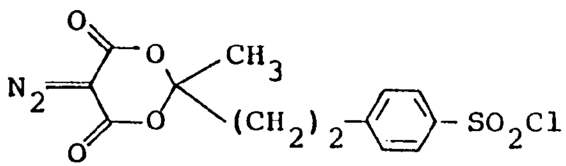 | " | " | " | 50 | 5 | 82 | 0.4 |
| 40 | 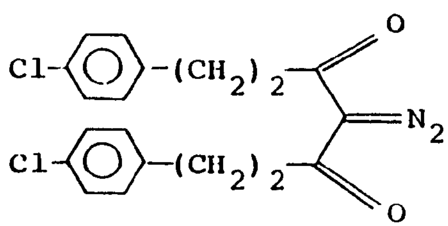 | " | " | " | ≦10 | 0 | 86 | 0.4 |

- Cont'd -

Table 1 (Cont'd)

| | | | | | | | | |
|---|---|---|---|---|---|---|---|---|
| 41 | 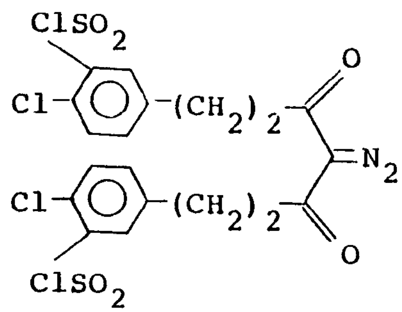 | 15 | 85 | 300 | <10 | 0 | 90 | 0.4 |
| 42 | 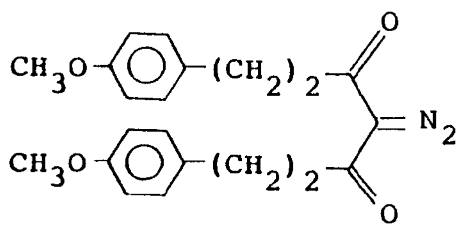 | " | " | " | 150 | 15 | 82 | 0.4 |
| 43 | 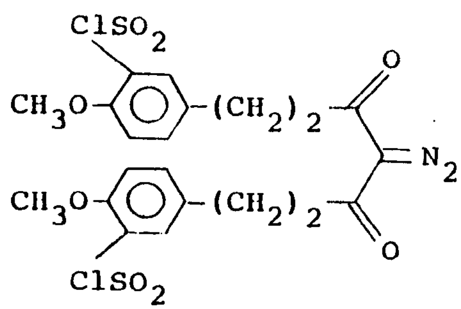 | " | " | " | <10 | 0 | 90 | 0.4 |

As is clear from Table 1, influences of the methylene chain length are shown in Run Nos. 2, 13, 15 and 17, Run Nos. 1, 12, 14 and 16, Run Nos. 18, 23, 27 and 29, and Run Nos. 19, 24, 28 and 30, wherein longer methylene chains provide good results with smaller using amounts, that is, the sensitivity as the photosensitive compound is increased.

Further, influences of substituents are shown in Run Nos. 1 and 2 to 11, wherein Run No. 2 is the best; in Run Nos. 18 and 19 to 22, wherein Run No. 19 is the best; and in Run Nos. 23 and 24 to 26, wherein Run No. 24 is the best.

## Claims

1. A photosensitive compound represented by the formula:

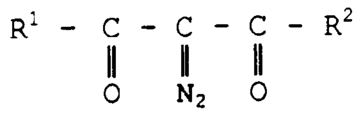

(I)

wherein $R^1$ is

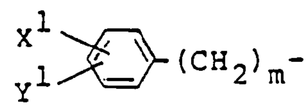

or

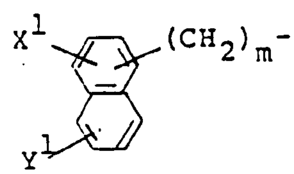

;

$X^1$ and $Y^1$ are independently a hydrogen atom, a halogen atom, a lower alkyl group having 1 to 5 carbon atoms, a lower alkoxy group having 1 to 5 carbon atoms, $-SO_2Cl$, $-SO_2Br$,

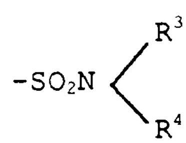

,

$-SO_3H$ or $-SO_3R^5$; $R^3$ and $R^4$ are independently a hydrogen atom or a lower alkyl group having 1 to 5 carbon atoms which may have one or more substituents, or $R^3$, $R^4$ and N taken together may form a heterocyclic ring; $R^5$ is a lower alkyl group having 1 to 5 carbon atoms; the $-SO_2Cl$ or $-SO_2Br$ group may include a quaternary salt thereof; the $-SO_3H$ group may include an ammonium salt thereof, an organic base salt thereof and a quaternary salt thereof; m is an integer of 1 to 20; $R^2$ is an alkyl group, a cycloalkyl group, a hydroxyalkyl group, an alkoxyalkyl group,

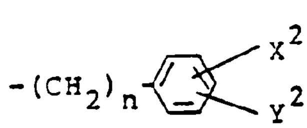

or

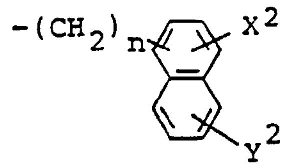

$X^2$ and $Y^2$ are independently a hydrogen atom, a halogen atom, a lower alkyl group having 1 to 5 carbon atoms, a lower alkoxy group having 1 to 5 carbon atoms, $-SO_2Cl$, $-SO_2Br$,

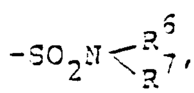

$-SO_3H$ or $-SO_3R^8$; $R^6$ and $R^7$ are independently a hydrogen atom or a lower alkyl group having 1 to 5 carbon atoms which may have one or more substituents, or $R^6$, $R^7$ and N taken together may form a heterocyclic ring; $R^8$ is a lower alkyl group having 1 to 5 carbon atoms; the $-SO_2Cl$ or $-SO_2Br$ group may include a quaternary salt thereof; the $-SO_3H$ group may include an ammonium salt thereof, an organic base salt thereof and a quaternary salt thereof; n is an integer of 1 to 20; or $R^1$ and $R^2$ taken together may form a group of the formula:

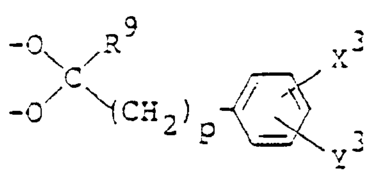

wherein $R^9$ is an alkyl group, an aralkyl group, a hydroxyalkyl group or an alkoxyalkyl group; $X^3$ and $Y^3$ are independently a hydrogen atom, a halogen atom, a lower alkyl group having 1 to 5 carbon atoms, a lower alkoxy group having 1 to 5 carbon atoms, $-SO_2Cl$, $-SO_2Br$,

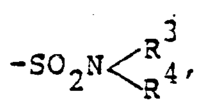

$-SO_3H$ or $-SO_3R^5$; $R^3$, $R^4$ and $R^5$ are as defined above; the $-SO_2Cl$ or $-SO_2Br$ group may include a quaternary salt thereof; the $-SO_3H$ group may include an ammonium salt thereof, an organic base salt thereof and a quaternary salt thereof; and p is an integer of 1 to 20, provided that $X^3$ and $Y^3$ cannot both together be a hydrogen atom.

2. A photosensitive compound according to Claim 1, which is a compound of the formula:

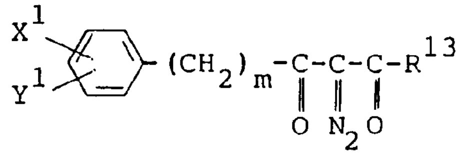

(II)

wherein $X^1$, $Y^1$ and m are as defined in Claim 1; and $R^{13}$ is an alkyl group, a cycloalkyl group, a hydroxyalkyl group or an alkoxyalkyl group,

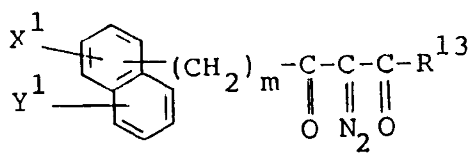

(III)

wherein $X^1$, $Y^1$, $R^{13}$ and m are as defined above,

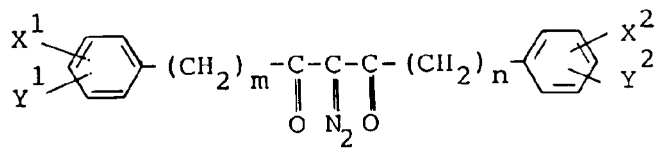

(IV)

wherein $X^1$, $Y^1$, $X^2$, $Y^2$, m and n are as defined in claim 1,

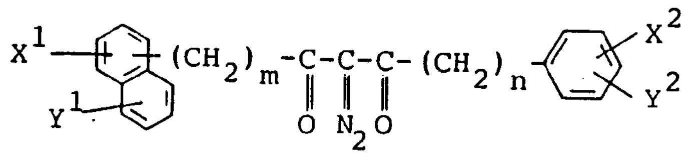

(V)

wherein $X^1$, $Y^1$, $X^2$, $Y^2$, m and n are as defined above,

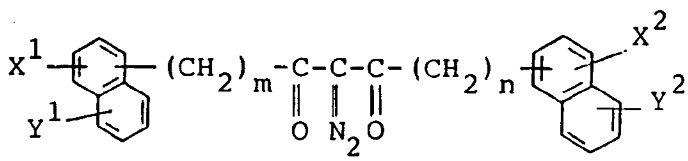

(VI)

wherein $X^1$, $Y^1$, $X^2$, $Y^2$, m and n are as defined above, or

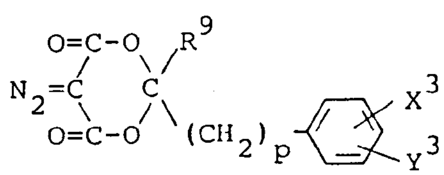

(VII)

wherein $X^3$, $Y^3$, $R^9$ and p are as defined in Claim 1.

3. A process for producing a compound of the formula:

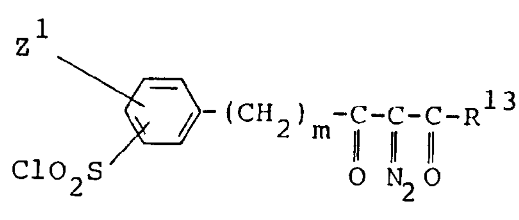

(II')

wherein $Z^1$ is a hydrogen atom, a halogen atom, a lower alkyl group or a lower alkoxy group; $R^{13}$ is an alkyl group, a cycloalkyl group, a hydroxyalkyl group or an alkoxyalkyl group; and m is an integer of 1 to 20, which comprises

reacting a compound of the formula:

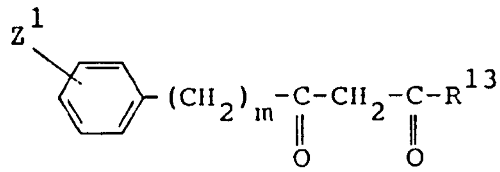

(II-1)

wherein $Z^1$, $R^{13}$ and m are as defined above, with a diazotizing agent in the presence of an organic base to form a compound of the formula:

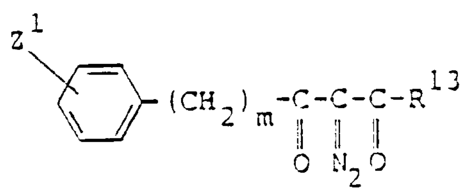

(II-4)

wherein $Z^1$, $R^{13}$ and m are as defined above, and reacting the compound of the formula (II-4) with chlorosulfonic acid.

**4.** A process for producing the compound of the formula (II′) of claim 3 which comprises
reacting the compound of the formula (ii-1) of claim 3 with a sulfonating agent, followed by neutralization to yield a compound of the formula:

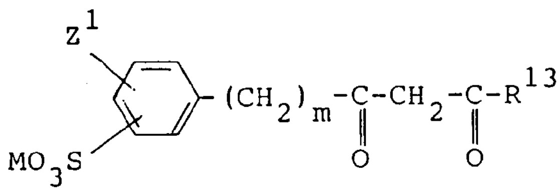

(II-2)

wherein M is an alkali metal atom, an ammonium group or a residue of an organic base; $Z^1$, $R^{13}$ and m are as defined above,
reacting the compound of the formula (II-2) with a diazotizing agent in the presence of an organic base to yield a compound of the formula:

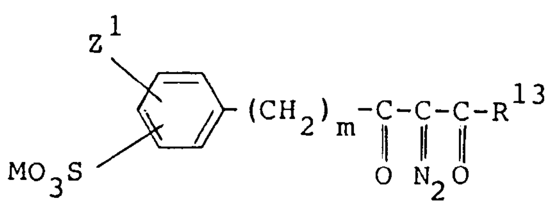

(II-3)

wherein M, $Z^1$, $R^{13}$ and m are as defined above, followed by reaction with a chlorinating agent.

**5.** A process for producing a compound of the formula:

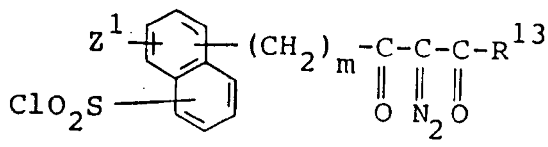

(III')

wherein $Z^1$ is a hydrogen atom, a halogen atom, a lower alkyl group or a lower alkoxy group; $R^{13}$ is an alkyl group, a cycloalkyl group, a hydroxyalkyl group, or an alkoxyalkyl group; and m is an integer of 1 to 20, which comprises

reacting a compound of the formula:

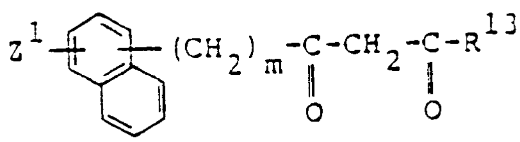

(III-1)

wherein $Z^1$, $R^{13}$ and m are as defined above, with a diazotizing agent in the presence of an organic base to yield a compound of the formula:

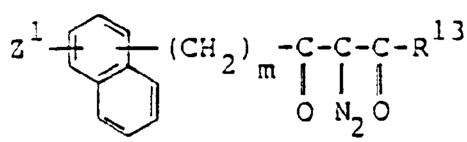

(III-4)

wherein $Z^1$, $R^{13}$ and m are as defined above, followed by a reaction with chlorosulfonic acid.

6. A process for producing the compound of the formula (III') of claim 5 which comprises

reacting the compound of the formula (III-1) of claim 5 with a sulfonating agent, followed by neutralization to yield a compound of the formula:

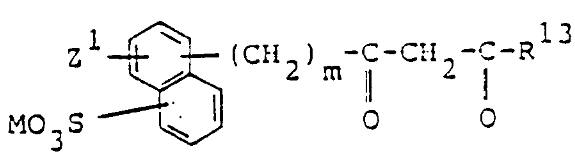

(III-2)

wherein M is an alkali metal atom, an ammonium group or a residue of an organic base; $Z^1$, $R^{13}$, and m are as defined above,

reacting the compound of the formula (III-2) with a diazotizing agent in the presence of a base to yield a compound of the formula:

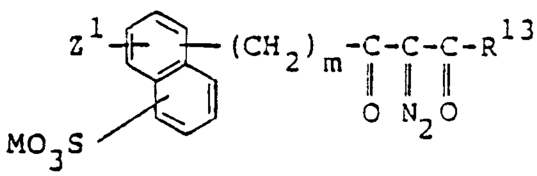

(III-3)

wherein M, $Z^1$, $R^{13}$ and m are as defined above, followed by reaction with a chlorinating agent.

7. A process for producing a compound of the formula:

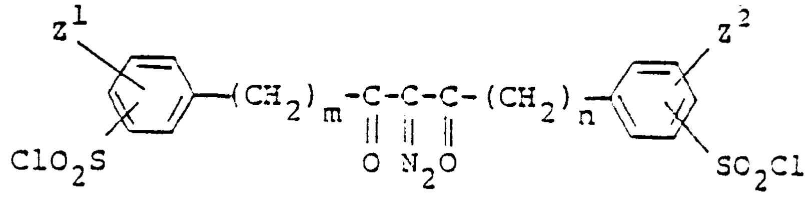

(IV')

wherein $Z^1$ and $Z^2$ are independently a hydrogen atom, a halogen atom, a lower alkyl group or a lower alkoxy group; m is an integer of 1 to 20; and n is an integer of 1 to 20, which comprises

reacting a compound of the formula:

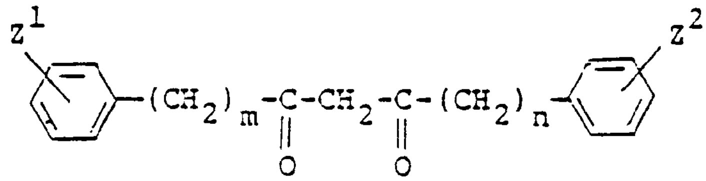

(IV-1)

wherein $Z^1$, $Z^2$, m and n are as defined above, with a diazotizing agent in the presence of an organic base to yield a compound of the formula:

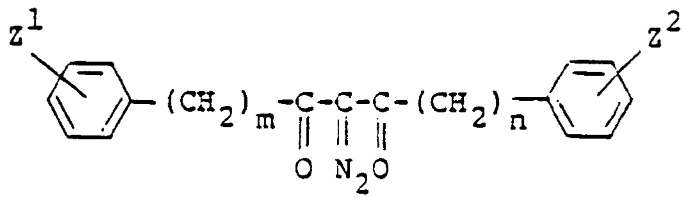

(IV-4)

wherein $Z^1$, $Z^2$, m and n are as defined above, followed by a reaction with chlorosulfonic acid.

**8.** A process for producing a compound of the formula (IV') of claim 7 which comprises
reacting the compound of the formula (IV-1) of claim 7 with a sulfonating agent, followed by neutralization to yield a compound of the formula:

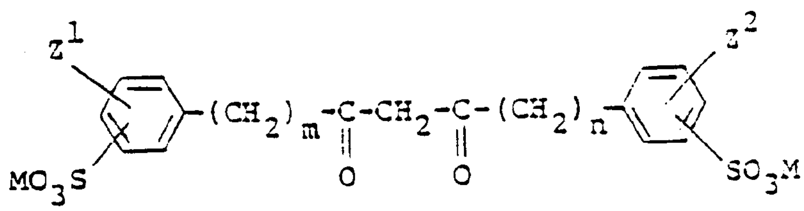

(IV-2)

wherein M is an alkali metal atom, an ammonium group or a residue of an organic base; $Z^1$, $Z^2$, m and n are as defined above, reacting the compound of the formula (IV-2) with a diazotizing agent in the presence of an organic base to yield a compound of the formula:

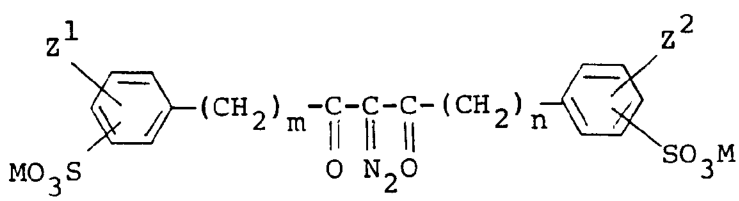

(IV-3)

wherein $Z^1$, $Z^2$, M, m and n are as defined above, followed by reaction with a chlorinating agent.

**9.** A process for producing a compound of the formula:

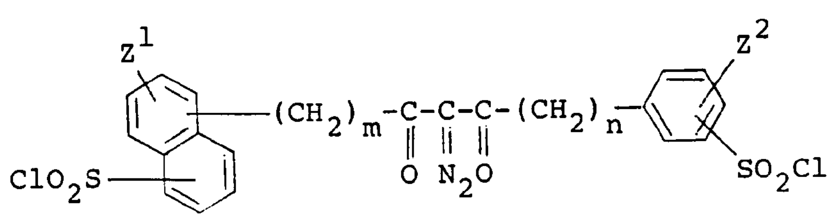

(V')

wherein $Z^1$ and $Z^2$ are independently a hydrogen atom, a halogen atom, a lower alkyl group or a lower alkoxy group; m is an integer of 1 to 20; and n is an integer of 1 to 20, which comprises reacting a compound of the formula:

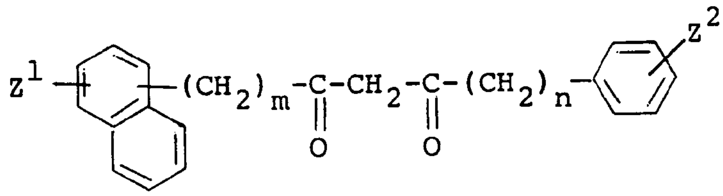

(V-1)

wherein $Z^1$, $Z^2$, m and n are as defined above, with a diazotizing agent in the presence of an organic base to yield a compound of the formula:

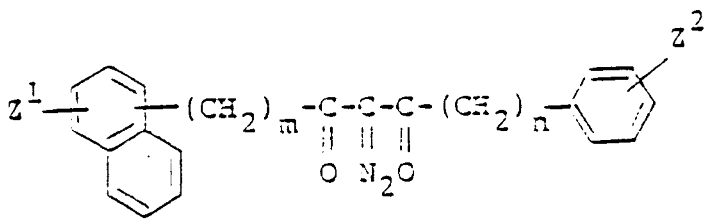

(V-4)

wherein $Z^1$, $Z^2$, m and n are as defined above, followed by a reaction with chlorosulfonic acid.

**10.** A process for producing a compound of the formula (V') of claim 9 which comprises reacting the compound of the formula (V-1) of claim 9 with a sulfonating agent, followed by neutralization to yield a compound of the formula:

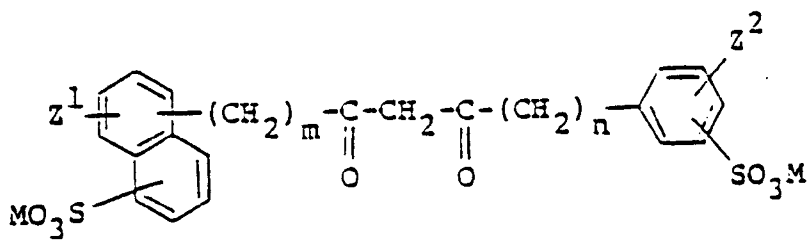

(V-2)

wherein M is an alkali metal atom, an ammonium group or a residue of an organic base; $Z^1$, $Z^2$, m and n are as defined above,

reacting the compound of the formula (V-2) with a diazotizing agent in the presence of an organic base to yield a compound of the formula:

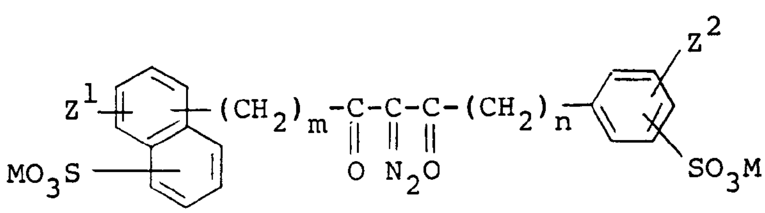

(V-3)

wherein $Z^1$, $Z^2$, M, m and n are as defined above, followed by reaction with a halogenating agent.

**11.** A process for producing a compound of the formula:

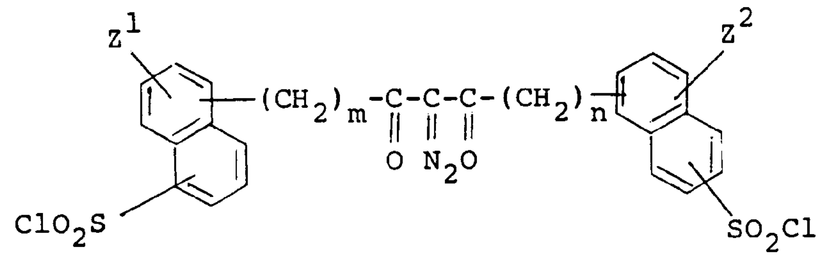

(VI')

wherein $Z^1$ and $Z^2$ are independently a hydrogen atom, a halogen atom, a lower alkyl group or a lower alkoxy group; m is an integer of 1 to 20; and n is an integer of 1 to 20, which comprises

reacting a compound of the formula:

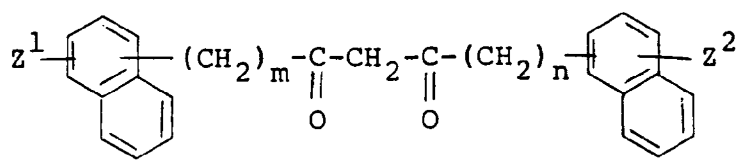

(VI-1)

wherein $Z^1$, $Z^2$, m and n are as defined above, with a diazotizing agent in the presence of an organic base to yield a compound of the formula:

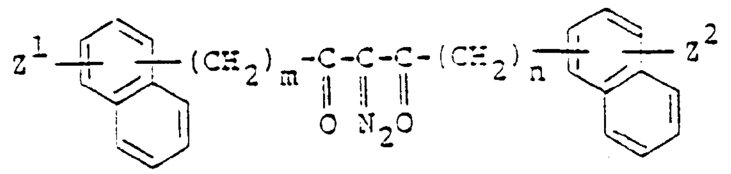

(VI-4)

wherein $Z^1$, $Z^2$, m and n are as defined above, followed by reaction with chlorosulfonic acid.

**12.** A process for producing a compound of the formula (VI') of claim 11 which comprises

reacting the compound of the formula (VI-1) of claim 11 with a sulfonating agent, followed by neutralization to yield a compound of the formula:

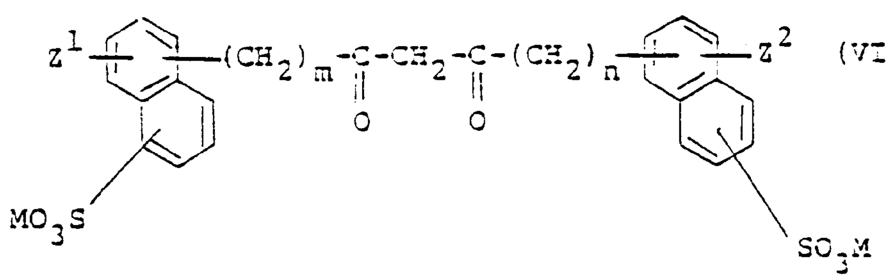

(VI-2)

wherein M is an alkali metal atom, an ammonium group or a residue of an organic base; $Z^1$, $Z^2$, m and n are as defined above,

reacting the compound of the formula (VI-2) with a diazotizing agent in the presence of a base to yield a compound of the formula:

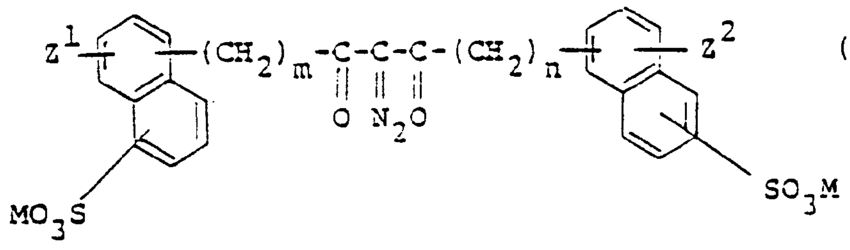

(VI-3)

wherein M, $Z^1$, $Z^2$, m and n are as defined above, followed by reaction with a chlorinating agent.

**13.** A process for producing a compound of the formula:

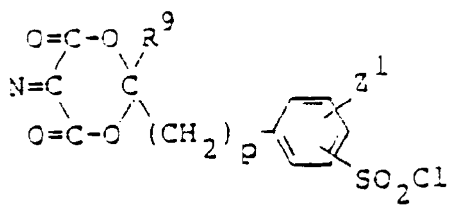

(VII')

wherein $Z^1$ is a hydrogen atom, a halogen atom, a lower alkyl group or a lower alkoxy group; $R^9$ is an alkyl group, an aralkyl group, a hydroxyalkyl group, or an alkoxyalkyl group; and p is an integer of 1 to 20, which comprises

reacting a compound of the formula:

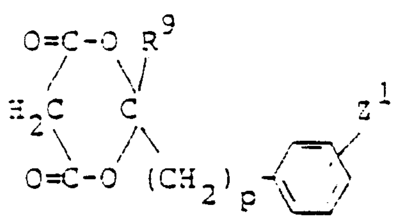

(VII-1)

wherein $Z^1$, $R^9$ and p are as defined above, with a diazotizing agent in the presence of an organic base to yield a compound of the formula:

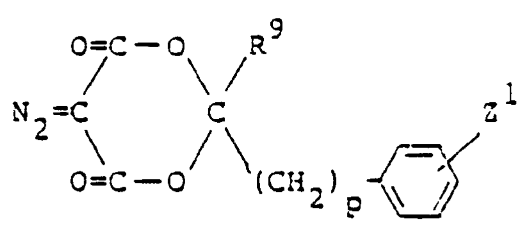

(VII-4)

wherein $Z^1$, $R^9$ and are as defined above, followed by a reaction with chlorosulfonic acid.

**14.** A process for producing the compound of the formula (VII') of claim 13 which comprises

reacting the compound of the formula (VII-1) of claim 13 with a sulfonating agent, followed by neutralization to yield a compound of the formula:

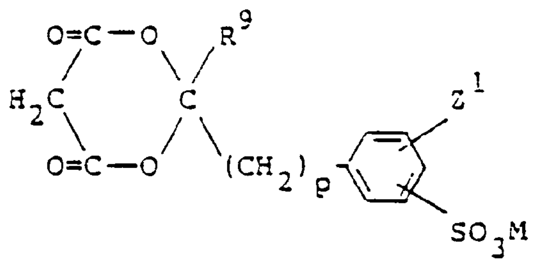

(VII-2)

wherein M is an alkali metal atom, an ammonium group or a residue of an organic base; $Z^1$, $R^9$ and p are as defined above,

reacting the compound of the formula (VII-2) with a diazotizing agent in the presence of an organic base to yield a compound of the formula:

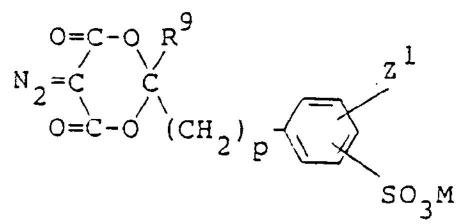

(VII-3)

wherein M, $Z^1$, $R^9$ and p are as defined above, followed by reaction with a chlorinating agent.

**15.** A process of production of photoengraved plates employing a photosensitive agent wherein the agent is a compound according to claim 1.

**Patentansprüche**

**1.** Photoempfindliche Verbindung dargestellt durch die Formel:

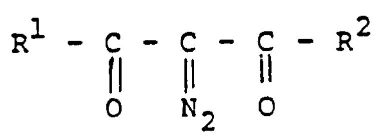

(I)

worin $R^1$ entweder

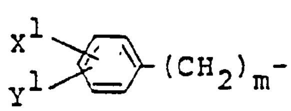

oder

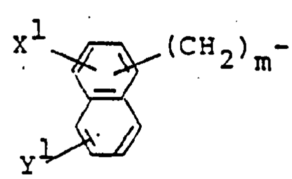

ist;

mit $X^1$ und $Y^1$ unabhängig ein Wasserstoffatom, ein Halogenatom, eine niedere Alkyl-Gruppe mit 1 bis 5 Kohlenstoffatomen, eine niedere Alkoxy-Gruppe mit 1 bis 5 Kohlenstoffatomen, $-SO_2Cl$, $-SO_2Br$,

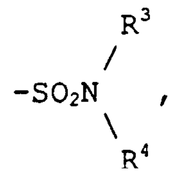

$-SO_3H$ oder $-SO_3R^5$; wobei $R^3$ und $R^4$ unabhängig ein Wasserstoffatom oder eine niedere Alkyl-Gruppe mit 1 bis 5 Kohlenstoffatomen sind, die einen oder mehrere Substituenten haben können, oder $R^3$, $R^4$ und N zusammengenommen einen heterocyclischen Ring bilden können; und wobei $R^5$ eine niedere Alkyl-Gruppe mit 1 bis 5 Kohlenstoffatomen ist, die Gruppen $-SO_2Cl$ oder $-SO_2Br$ ein quartäres Salz davon umfassen können und wobei die Gruppe $-SO_3H$ ein Ammoniumsalz davon, ein organisches basisches Salz davon und ein quartäres Salz davon umfassen kann;
und mit m eine ganze Zahl von 1 bis 20;

und worin $R^2$ eine Alkyl-Gruppe, eine Cycloalkyl-Gruppe, eine Hydroxyalkyl-Gruppe, eine Alkoxyalkyl-Gruppe,

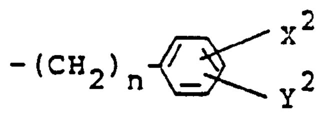

oder

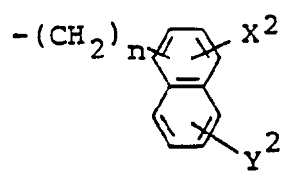

ist;
mit $X^2$ und $Y^2$ unabhängig ein Wasserstoffatom, ein Halogenatom, eine niedere Alkyl-Gruppe mit 1 bis 5 Kohlenstoffatomen, eine niedere Alkoxy-Gruppe mit 1 bis 5 Kohlenstoffatomen, $-SO_2Cl$, $-SO_2Br$,

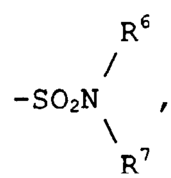

$-SO_3H$ oder $-SO_3R^8$; wobei $R^6$ und $R^7$ unabhängig ein Wasserstoffatom oder eine niedere Alkyl-Gruppe mit 1 bis 5 Kohlenstoffatome sind, die einen oder mehrere Substituenten haben können, oder $R^6$, $R^7$ und N können zusammengenommen einen heterocyclischen Ring bilden; und wobei $R^8$ eine niedere Alkyl-Gruppe mit 1 bis 5 Kohlenstoffatomen ist, die Gruppen $-SO_2Cl$ oder $-SO_2Br$ ein quartäres Salz davon umfassen können und die Gruppe $-SO_3H$ ein Ammoniumsalz davon, ein organisches basisches Salz davon und ein quartäres Salz davon umfassen kann;
und mit n eine ganze Zahl von 1 bis 20;

oder worin $R^1$ und $R^2$ zusammengenommen eine Gruppe der Formel

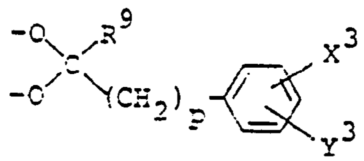

bilden können,
wobei $R^9$ eine Alkyl-Gruppe, eine Aralkyl-Gruppe, eine Hydroxyalkyl-Gruppe oder eine Alkoxyalkyl-Gruppe ist, $X^3$ und $Y^3$ unabhängig ein Wasserstoffatom, ein Halogenatom, eine niedere Alkyl-Gruppe mit 1 bis 5 Kohlenstoffatomen, eine niedere Alkoxy-Gruppe mit 1 bis 5 Kohlenstoffatomen, $-SO_2Cl$, $-SO_2Br$,

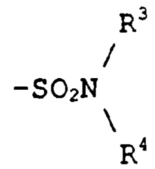 ,

$-SO_3H$ oder $-SO_3R^5$ sind; wobei $R^3$, $R^4$ und $R^5$ wie vorstehend festgelegt sind, die Gruppen $-SO_2Cl$ oder $-SO_2Br$ ein quartäres Salz davon umfassen können; die Gruppe $-SO_3H$ ein Ammoniumsalz davon, ein organisches basisches Salz davon und ein quartäres Salz davon umfassen kann; und wobei p eine ganze Zahl von 1 bis 20 unter der Voraussetzung ist, daß $X^3$ und $Y^3$ nicht beide gemeinsam ein Wasserstoffatom sind.

2. Photoempfindliche Verbindung gemäß Anspruch 1, welche eine Verbindung der Formel ist:

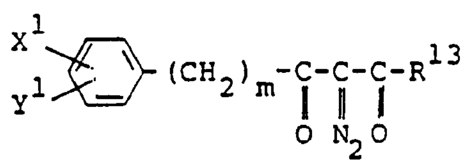 (II)

worin $X^1$, $Y^1$ und m wie in Anspruch 1 festgelegt sind; und $R^{13}$ eine Alkyl-Gruppe, eine Cycloalkyl-Gruppe, eine Hydroxyalkyl-Gruppe oder eine Alkoxyalkyl-Gruppe ist,

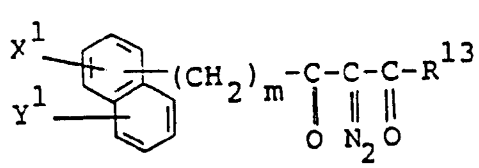 (III)

worin $X^1$, $Y^1$, $R^{13}$ und m wie vorstehend festgelegt sind,

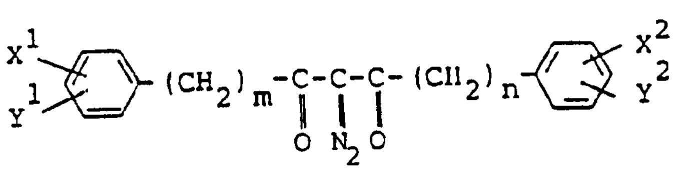 (IV)

worin $X^1$, $Y^1$, $X^2$, $Y^2$, m und n wie in Anspruch 1 festgelegt sind,

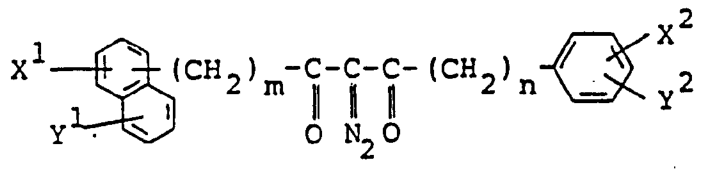

(V)

worin $X^1$, $Y^1$, $X^2$, $Y^2$, m und n wie vorstehend festgelegt sind,

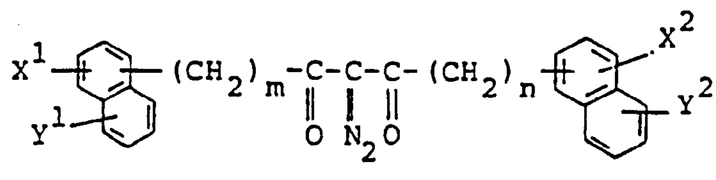

(VI)

worin $X^1$, $Y^1$, $X^2$, $Y^2$, m und n wie vorstehend festgelegt sind oder

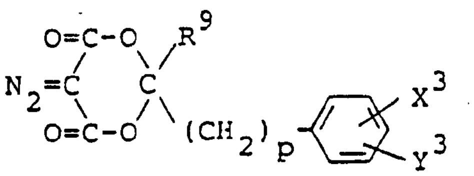

(VII)

worin $X^3$, $Y^3$, $R^9$ und p wie in Anspruch 1 festgelegt sind.

3. Verfahren zur Herstellung einer Verbindung der Formel:

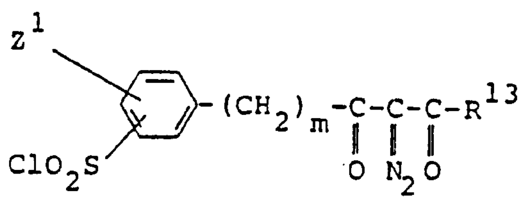

(II')

worin $Z^1$ ein Wasserstoffatom, ein Halogenatom, eine niedere Alkyl-Gruppe oder eine niedere Alkoxy-Gruppe ist; $R^{13}$ eine Alkyl-Gruppe, eine Cycloalkyl-Gruppe, eine Hydroxyalkyl-Gruppe oder eine Alkoxyalkyl-Gruppe ist und m eine ganze Zahl von 1 bis 20 ist, welches Verfahren umfaßt:

Umsetzen einer Verbindung der Formel:

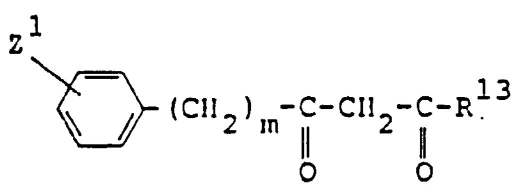

(II-1)

worin $Z^1$, $R^{13}$ und m wie vorstehend festgelegt sind, mit einem Diazotierungsmittel in Gegenwart einer organischen Base, um eine Verbindung der Formel zu bilden:

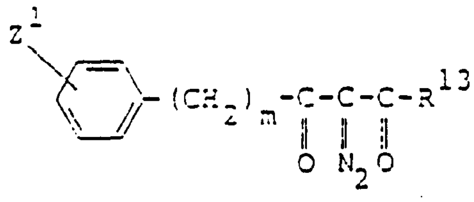

(II-4)

worin $Z^1$, $R^{13}$ und m wie vorstehend festgelegt sind, und Umsetzen der Verbindung der Formel (II-4) mit Chlorsulfonsäure.

4. Verfahren zur Herstellung der Verbindung der Formel (II') von Anspruch 3, umfassend das Umsetzen der Verbindung der Formel (II-1) von Anspruch 3 mit einem Sulfonierungsmittel, gefolgt durch Neutralisieren, um eine Verbindung der Formel:

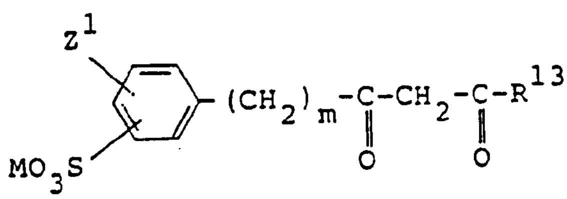

(II-2)

zu ergeben, worin M ein Alkalimetallatom, eine Ammonium-Gruppe oder ein Rest einer organischen Base ist; $Z^1$, $R^{13}$ und m wie vorstehend festgelegt sind,

Umsetzen der Verbindung der Formel (II-2) mit einem Diazotierungsmittel in Gegenwart einer organischen Base, um eine Verbindung der Formel:

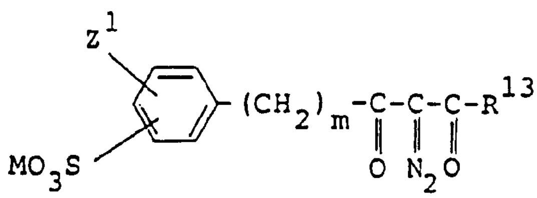

(II-3)

zu ergeben, worin M, $Z^1$, $R^{13}$ und m wie vorstehend festgelegt sind, gefolgt durch Neutralisieren mit einem Chlorierungsmittel.

5. Verfahren zur Herstellung einer Verbindung der Formel:

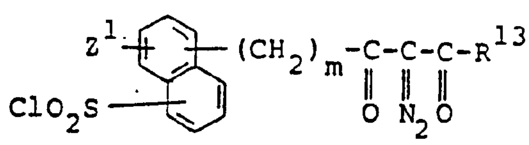

(III')

worin $Z^1$ ein Wasserstoffatom, ein Halogenatom, eine niedere Alkyl-Gruppe oder eine niedere Alkoxy-Gruppe ist; $R^{13}$ eine Alkyl-Gruppe, eine Cycloalkyl-Gruppe, eine Hydroxyalkyl-Gruppe oder eine Alkoxyalkyl-Gruppe ist und m eine ganze Zahl von 1 bis 20, welches Verfahren umfaßt:

Umsetzen einer Verbindung der Formel:

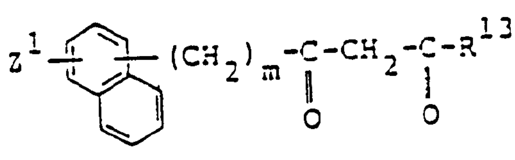

(III-1)

worin $Z^1$, $R^{13}$ und m wie vorstehend festgelegt sind, mit einem Diazotierungsmittel in Gegenwart einer organischen Base, um eine Verbindung der Formel:

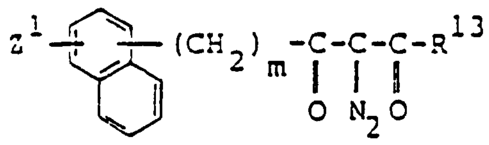

(III-4)

zu ergeben, worin $Z^1$, $R^{13}$ und m wie vorstehend festgelegt sind, gefolgt durch ein Umsetzen mit Chlorsulfonsäure.

6. Verfahren zur Herstellung der Verbindung der Formel (III') von Anspruch 5, umfassend das
Umsetzen der Verbindung der Formel (III-1) von Anspruch 5 mit einem Sulfonierungsmittel, gefolgt durch Neutralisieren, um eine Verbindung der Formel:

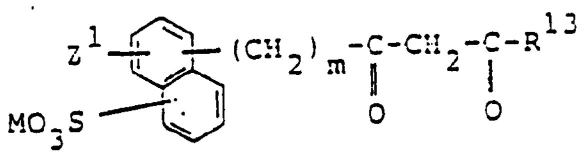

(III-2)

zu ergeben, worin M ein Alkalimetallatom, eine Ammonium-Gruppe oder ein Rest einer organischen Base ist; $Z^1$, $R^{13}$ und m wie vorstehend festgelegt sind,
Umsetzen der Verbindung der Formel (III-2) mit einem Diazotierungsmittel in Gegenwart einer Base, um eine Verbindung der Formel:

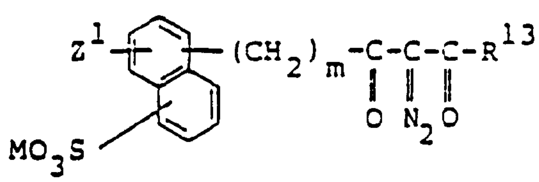

(III-3)

zu ergeben, worin M, $Z^1$, $R^{13}$ und m wie vorstehend festgelegt sind, gefolgt von einem Umsetzen mit einem Chlorierungsmittel.

7. Verfahren zur Herstellung einer Verbindung der Formel:

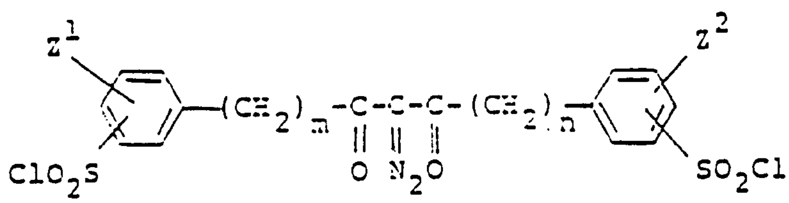

(IV')

worin $Z^1$ und $Z^2$ unabhängig ein Wasserstoffatom, ein Halogenatom, eine niedere Alkyl-Gruppe oder eine niedere Alkoxy-Gruppe sind; m eine ganze Zahl von 1 bis 20 und n eine ganze Zahl von 1 bis 20 sind, welches Verfahren umfaßt:
Umsetzen einer Verbindung der Formel:

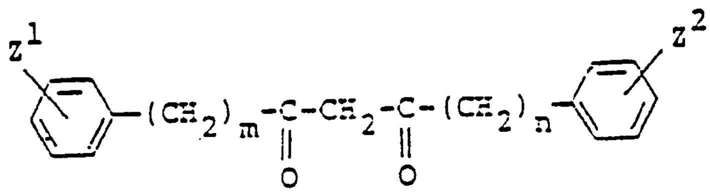

(IV-1)

worin $Z^1$, $Z^2$, m und n wie vorstehend festgelegt sind, mit einem Diazotierungsmittel in Gegenwart einer organischen Base, um eine Verbindung der Formel:

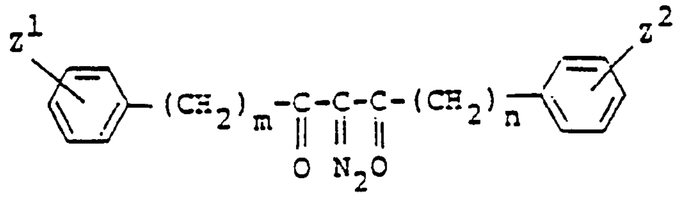

(IV-4)

zu ergeben, worin $Z^1$, $Z^2$, m und n wie vorstehend festgelegt sind, gefolgt von einem Umsetzen mit Chlorsulfonsäure.

**8.** Verfahren zur Herstellung einer Verbindung der Formel (IV′) von Anspruch 7, welches Verfahren umfaßt:
Umsetzen der Verbindung der Formel (IV-1) von Anspruch 7 mit einem Sulfonierungsmittel, gefolgt durch Neutralisieren, um eine Verbindung der Formel:

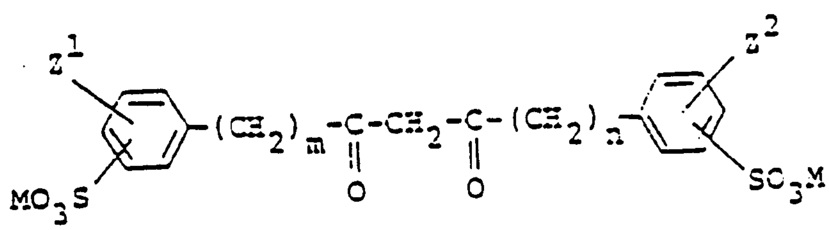

(IV-2)

zu ergeben, worin M ein Alkalimetallatom, eine Ammonium-Gruppe oder ein Rest einer organischen Base ist; $Z^1$, $Z^2$, m und n wie vorstehend festgelegt sind, Umsetzen der Verbindung der Formel (IV-2) mit einem Diazotierungsmittel in Gegenwart einer organischen Base, um eine Verbindung der Formel:

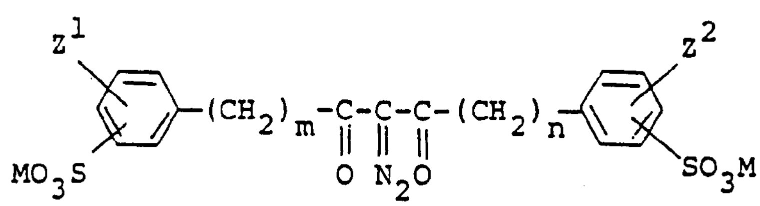

(IV-3)

zu ergeben, worin $Z^1$, $Z^2$, M, m und n wie vorstehend festgelegt sind, gefolgt von einem Umsetzen mit einem Chlorierungsmittel.

**9.** Verfahren zur Herstellung einer Verbindung der Formel:

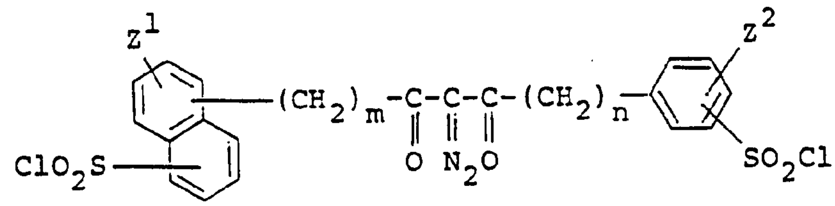

(V')

worin $Z^1$ und $Z^2$ unabhängig ein Wasserstoffatom, ein Halogenatom, eine niedere Alkyl-Gruppe oder eine niedere Alkoxy-Gruppe sind; m eine ganze Zahl von 1 bis 20 und n eine ganze Zahl von 1 bis 20 sind, welches Verfahren umfaßt:

Umsetzen einer Verbindung der Formel:

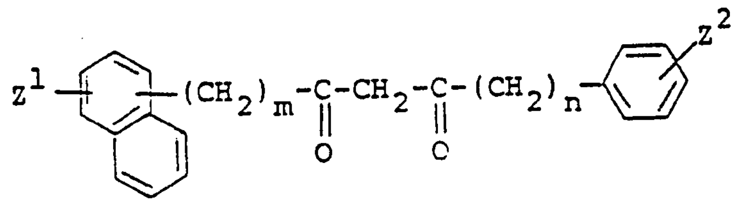

(V-1)

worin $Z^1$, $Z^2$, m und n wie vorstehend festgelegt sind, mit einem Diazotierungsmittel in Gegenwart einer organischen Base, um eine Verbindung der Formel:

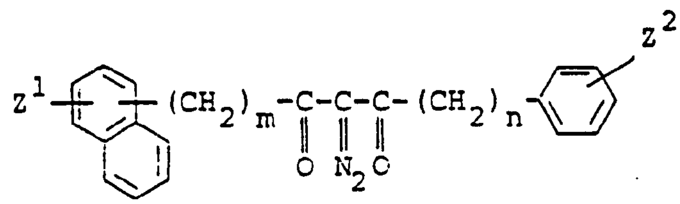

(V-4)

zu ergeben, worin $Z^1$, $Z^2$, m und n wie vorstehend festgelegt sind, gefolgt von einem Umsetzen mit Chlorsulfonsäure.

**10.** Verfahren zur Herstellung einer Verbindung der Formel (V') von Anspruch 9, welches Verfahren umfaßt:

Umsetzen der Verbindung der Formel (V-1) von Anspruch 9 mit einem Sulfonierungsmittel, gefolgt durch Neutralisieren, um eine Verbindung der Formel:

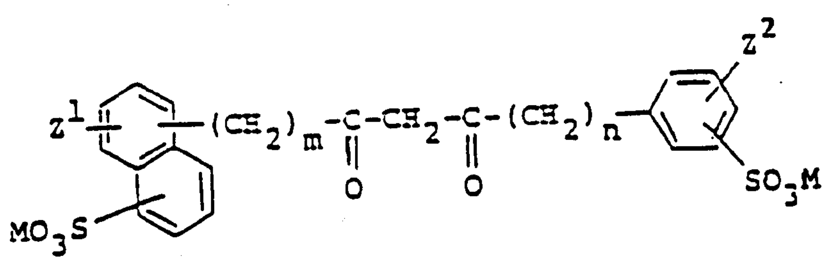

(V-2)

zu ergeben, worin M ein Alkalimetallatom, eine Ammonium-Gruppe oder ein Rest einer organischen Base ist; $Z^1$, $Z^2$, m und n wie vorstehend festgelegt sind,

Umsetzen der Verbindung der Formel (V-2) mit einem Diazotierungsmittel in Gegenwart einer organischen Base, um eine Verbindung der Formel:

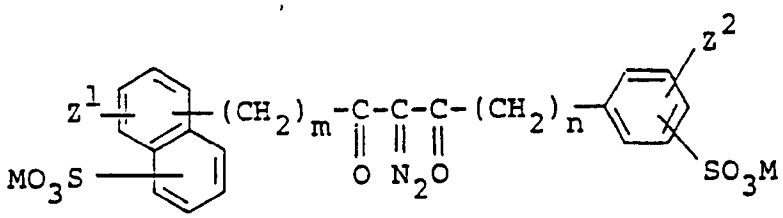

(V-3)

zu ergeben, worin $Z^1$, $Z^2$, M, m und n wie vorstehend festgelegt sind, gefolgt von einem Umsetzen mit einem Halogenierungsmittel.

**11.** Verfahren zur Herstellung einer Verbindung der Formel:

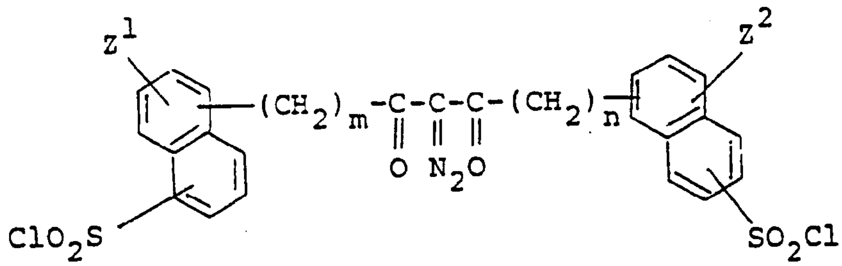

(VI')

worin $Z^1$ und $Z^2$ unabhängig ein Wasserstoffatom, ein Halogenatom, eine niedere Alkyl-Gruppe oder niedere Alkoxy-Gruppe sind, m eine ganze Zahl von 1 bis 20 und n eine ganze Zahl von 1 bis 20 sind, welches Verfahren umfaßt:

Umsetzen einer Verbindung der Formel:

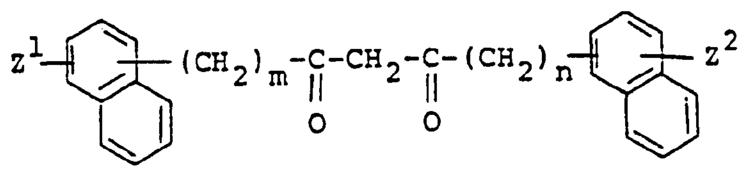

(VI-1)

worin $Z^1$, $Z^2$, m und n wie vorstehend festgelegt sind, mit einem Diazotierungsmittel in Gegenwart einer organischen Base, um eine Verbindung der Formel:

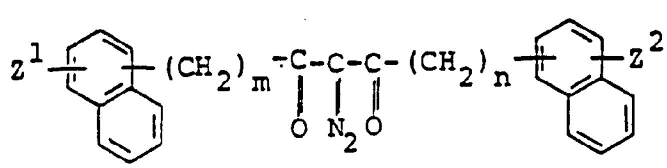

(VI-4)

zu ergeben, worin $Z^1$, $Z^2$, m und n wie vorstehend festgelegt sind, gefolgt von einem Umsetzen mit Chlorsulfonsäure.

**12.** Verfahren zu Herstellung einer Verbindung der Formel (VI') von Anspruch 11, welches Verfahren umfaßt:

Umsetzen der Verbindung der Formel (VI-1) von Anspruch 11 mit einem Sulfonierungsmittel, gefolgt durch Neutralisieren, um eine Verbindung der Formel:

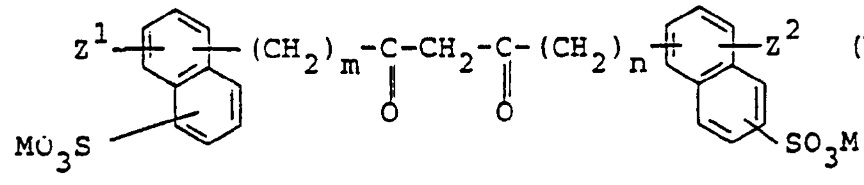

(VI-2)

zu ergeben, worin M ein Alkalimetallatom, eine Ammonium-Gruppe oder ein Rest einer organischen Base ist; $Z^1$, $Z^2$, m und n wie vorstehend festgelegt sind,

Umsetzen der Verbindung der Formel (VI-2) mit einem Diazotierungsmittel in Gegenwart einer Base, um eine Verbindung der Formel:

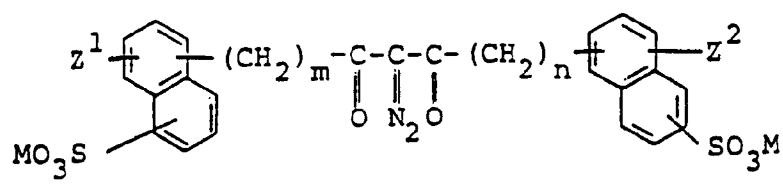

(VI-3)

zu ergeben, worin M, $Z^1$, $Z^2$, m und n wie vorstehend festgelegt sind, gefolgt von einem Umsetzen mit einem Chlorierungsmittel.

**13.** Verfahren zur Herstellung einer Verbindung der Formel:

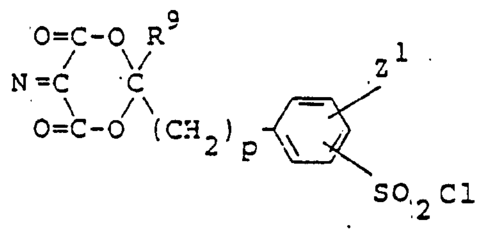

(VII')

worin $Z^1$ ein Wasserstoffatom, ein Halogenatom, eine niedere Alkyl-Gruppe oder eine niedere Alkoxy-Gruppe ist; $R^9$ eine Alkyl-Gruppe, eine Aralkyl-Gruppe, eine Hydroxyalkyl-Gruppe oder eine Alkoxyalkyl-Gruppe ist und p eine ganze Zahl von 1 bis 20 ist, welches Verfahren umfaßt:

Umsetzen einer Verbindung der Formel:

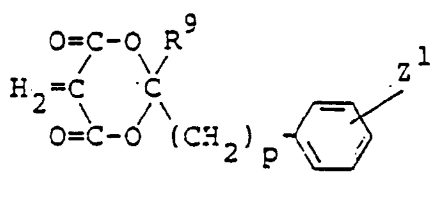

(VII-1)

worin $Z^1$, $R^9$ und p wie vorstehend festgelegt sind, mit einem Diazotierungsmittel in Gegenwart einer organischen Base, um eine Verbindung der Formel:

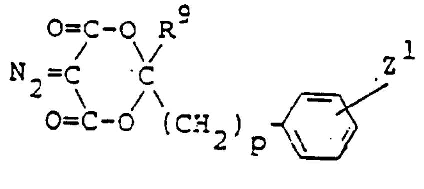

(VII-4)

zu ergeben, worin $Z^1$, $R^9$ und p wie vorstehend festgelegt sind, gefolgt von einem Umsetzen mit Chlorsulfonsäure.

**14.** Verfahren zur Herstellung der Verbindung der Formel (VII') von Anspruch 13, welches Verfahren umfaßt:

Umsetzen der Verbindung der Formel (VII-1) von Anspruch 13 mit einem Sulfonierungsmittel, gefolgt durch Neutralisieren, um eine Verbindung der Formel:

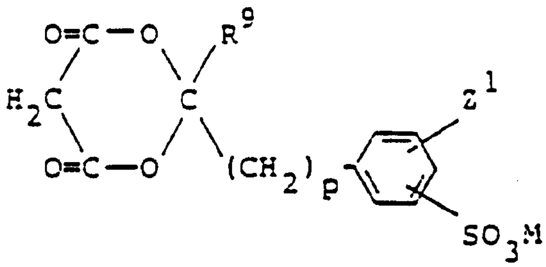

(VII-2)

zu ergeben, worin M ein Alkalimetallatom, eine Ammonium-Gruppe oder ein Rest einer organischen Base ist; $Z^1$, $R^9$ und p wie vorstehend festgelegt sind,

Umsetzen der Verbindung der Formel (VII-2) mit einem Diazotierungsmittel in Gegenwart einer organischen Base, um eine Verbindung der Formel:

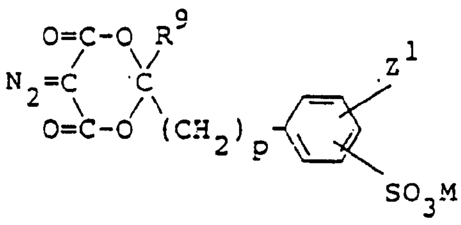

(VII-3)

zu ergeben, worin M, $Z^1$, $R^9$ und p wie vorstehend festgelegt sind, gefolgt von einem Umsetzen mit einem Chlorierungsmittel.

**15.** Verfahren zur Herstellung von Ätzplatten unter Einsatz eines photoempfindlichen Mittels, bei welchem das Mittel eine Verbindung nach Anspruch 1 ist.

## Revendications

**1.** Composé photosensible représenté par la formule

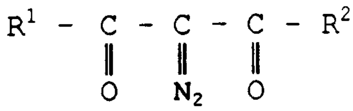

(I)

où $R^1$ est

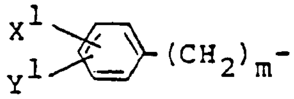

ou

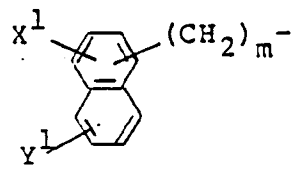

;

$X^1$ et $Y^1$ sont indépendamment un atome d'hydrogène, un atome d'halogène, un groupe alkyle inférieur ayant 1 à 5 atomes de carbone, un groupe alcoxy inférieur ayant 1 à 5 atomes de carbone, $-SO_2Cl$, $-SO_2Br$,

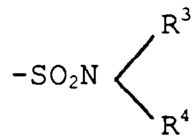 ,

$-SO_3H$ ou $-SO_3R^5$ ; $R^3$ et $R^4$ sont indépendamment un atome d'hydrogène ou un groupe alkyle inférieur ayant 1 à 5 atomes de carbone qui peut avoir un ou plusieurs substituants, ou $R^3$, $R^4$ et N pris ensemble peuvent former un cycle hétérocyclique ; $R^5$ est un groupe alkyle inférieur ayant 1 à 5 atomes de carbone ; le groupe $-SO_2Cl$ ou $-SO_2Br$ peut inclure un sel quaternaire de celui-ci ; le groupe $-SO_3H$ peut inclure un sel d'ammonium de celui-ci, un sel de base organique de celui-ci et un sel quaternaire de celui-ci ; m est un nombre entier de 1 à 20 ; $R^2$ est un groupe alkyle, un groupe cycloalkyle, un groupe hydroxyalkyle, un groupe alcoxyalkyle,

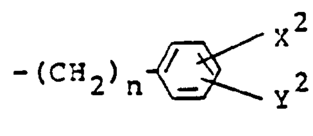 ou 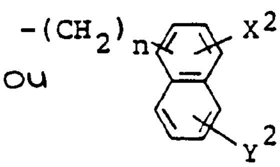 ;

$X^2$ et $Y^2$ sont indépendamment un atome d'hydrogène, un atome d'halogène, un groupe alkyle inférieur ayant 1 à 5 atomes de carbone, un groupe alcoxy inférieur ayant 1 à 5 atomes de carbone, $-SO_2Cl$, $-SO_2Br$,

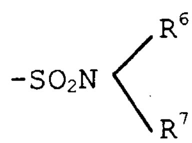 ,

$-SO_3H$ ou $-SO_3R^8$ ; $R^6$ et $R^7$ sont indépendamment un atome d'hydrogène ou un groupe alkyle inférieur ayant 1 à 5 atomes de carbone qui peut avoir un ou plusieurs substituants, ou $R^6$, $R^7$ et N pris ensemble peuvent former un cycle hétérocyclique ; $R^8$ est un groupe alkyle inférieur ayant 1 à 5 atomes de carbone ; le groupe $-SO_2Cl$ ou $-SO_2Br$ peut inclure un sel quaternaire de celui-ci ; le groupe $-SO_3H$ peut inclure un sel d'ammonium de celui-ci, un sel de base organique de celui-ci et un sel quaternaire de celui-ci ; n est un nombre entier de 1 à 20 ; ou $R^1$ et $R^2$ pris ensemble peuvent former un groupe de formule :

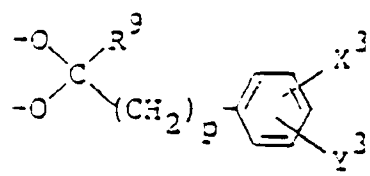

où $R^9$ est un groupe alkyle, un groupe aralkyle, un groupe hydroxyalkyle ou un groupe alcoxyalkyle ; $X^3$ et $Y^3$ sont indépendamment un atome d'hydrogène, un atome d'halogène, un groupe alkyle inférieur ayant 1 à 5 atomes de carbone, un groupe alcoxy inférieur ayant 1 à 5 atomes de carbone, $-SO_2Cl$, $-SO_2Br$,

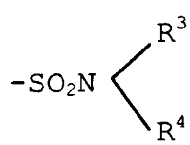 ,

$-SO_3H$ ou $-SO_3R^5$ ; $R^3$, $R^4$ et $R^5$ sont tels que définis ci-dessus ; le groupe $-SO_2Cl$ ou $-SO_2Br$ peut inclure un sel quaternaire de celui-ci ; le groupe $-SO_3H$ peut inclure un sel d'ammonium de celui-ci, un sel de base organique de celui-ci et un sel quaternaire de celui-ci et p est un nombre entier de 1 à 20, à condition que $X^3$ et $Y^3$ ne puissent pas être tous les deux ensemble un atome d'hydrogène.

2. Composé photosensible selon la revendication 1, qui est un composé de formule :

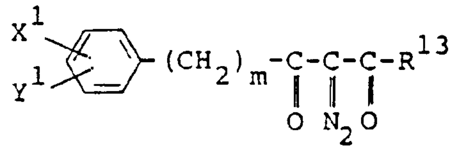

(II)

où $X^1$, $Y^1$ et m sont tels que définis dans la revendication 1 ; et $R^{13}$ est un groupe alkyle, un groupe cycloalkyle, un groupe hydroxyalkyle ou un groupe alcoxyalkyle,

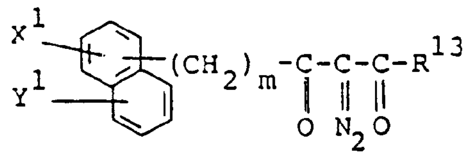

(III)

où $X^1$, $Y^1$, $R^{13}$ et m sont tels que définis ci-dessus,

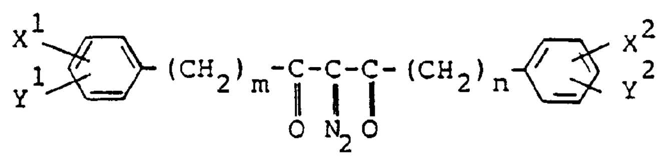

(IV)

où $X^1$, $Y^1$, $X^2$, $Y^2$, m et n sont tels que définis dans la revendication 1,

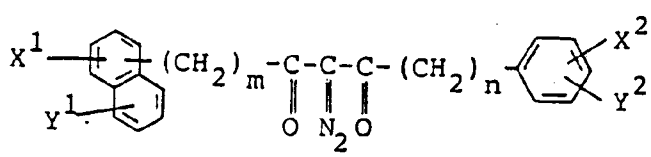

(V)

où $X^1$, $Y^1$, $X^2$, $Y^2$, m et n sont tels que définis ci-dessus

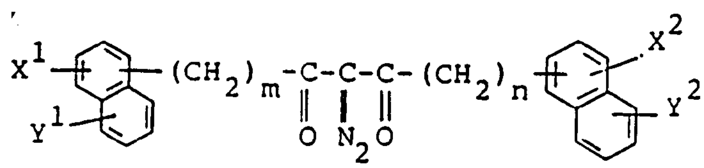

(VI)

où $X^1$, $Y^1$, $X^2$, $Y^2$, m et n sont tels que définis ci-dessus, ou

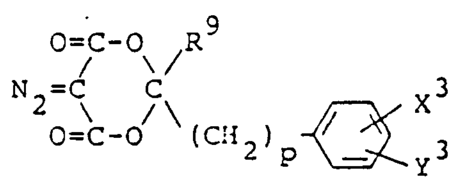

(VII)

où $X^3$, $Y^3$, $R^9$ et p sont tels que définis dans la revendication 1.

3. Procédé pour préparer un composé de formule :

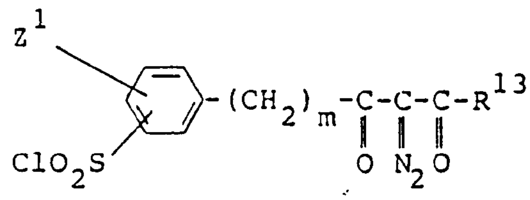

(II')

où $Z^1$ est un atome d'hydrogène, un atome d'halogène, un groupe alkyle inférieur ou un groupe alcoxy inférieur ; $R^{13}$ est un groupe alkyle, un groupe cycloalkyle, un groupe hydroxyalkyle ou un groupe alcoxyalkyle ; et m est un nombre entier de 1 à 20, qui comprend la réaction d'un composé de formule :

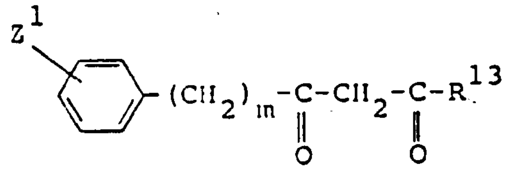

(II-1)

où $Z^1$, $R^{13}$ et m sont tels que définis ci-dessus, avec un agent diazotant en présence d'une base organique pour former un composé de formule :

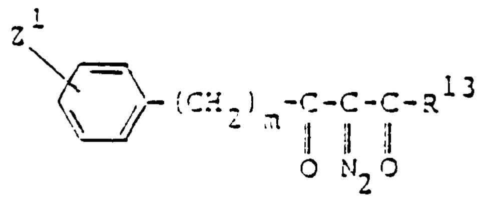

(II-4)

où $Z^1$, $R^{13}$ et m sont tels que définis ci-dessus, et la réaction du composé de formule (II-4) avec de l'acide chlorosulfonique.

4. Procédé pour préparer la composé de formule (II') de la revendication 3, qui comprend

la réaction du composé de formule (II-1) de la revendication 3 avec un agent sulfonant, suivie d'une neutralisation pour donner un composé de formule :

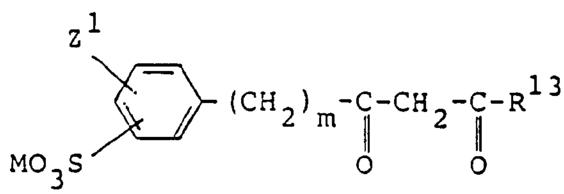

(II-2)

où M est un atome de métal alcalin, un groupe ammonium ou un résidu d'une base organique ; $Z^1$, $R^{13}$ et m sont tels que définis ci-dessus,

la réaction du composé de formule (II-2) avec un agent diazotant en présence d'une base organique pour donner un composé de formule :

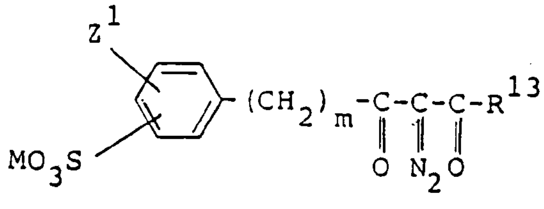

(II-3)

où M, $Z^1$, $R^{13}$ et m sont tels que définis ci-dessus, suivie d'une réaction avec un agent chlorant.

5. Procédé pour préparer un composé de formule :

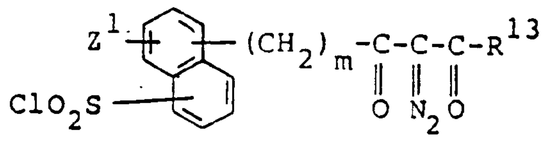

(III')

où $Z^1$ est un atome d'hydrogène, un atome d'halogène, un groupe alkyle inférieur ou un groupe alcoxy inférieur ; $R^{13}$ est un groupe alkyle, un groupe cycloalkyle, un groupe hydroxyalkyle, ou un groupe alcoxyalkyle ; et m est un nombre entier de 1 à 20, qui comprend la réaction d'un composé de formule :

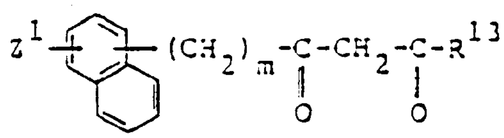

(III-1)

où $Z^1$, $R^{13}$ et m sont tels que définis ci-dessus, avec un agent diazotant en présence d'une base organique pour donner un composé de formule :

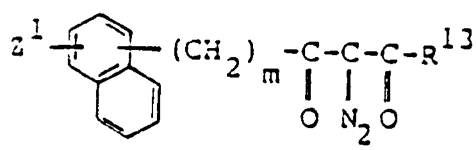

(III-4)

où $Z^1$, $R^{13}$ et m sont tels que définis ci-dessus, suivie d'une réaction avec de l'acide chlorosulfonique.

6. Procédé pour préparer le composé de formule (III') de la revendication 5, qui comprend

la réaction du composé de formule (III-1) de la revendication 5 avec un agent sulfonant, suivie d'une neutralisation pour donner un composé de formule :

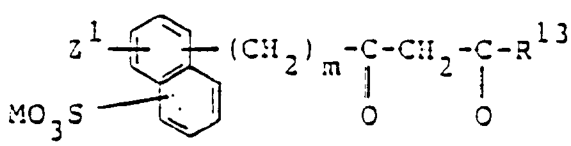

(III-2)

où M est un atome de métal alcalin, un groupe ammonium ou un résidu d'une base organique ; $Z^1$, $R^{13}$ et m sont tels que définis ci-dessus,

la réaction du composé de formule (III-2) avec un agent diazotant en présence d'une base organique pour donner un composé de formule :

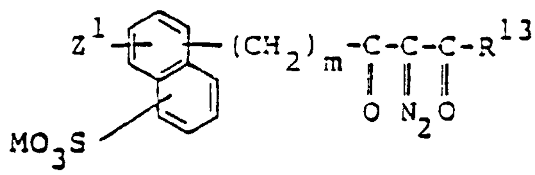

(III-3)

où M, $Z^1$, $R^{13}$ et m sont tels que définis ci-dessus, suivie d'une réaction avec un agent chlorant.

7. Procédé pour préparer un composé de formule :

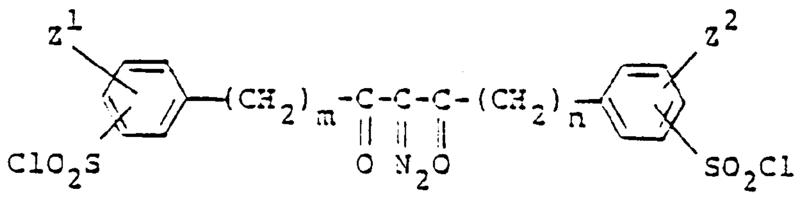

(IV')

où $Z^1$ et $Z^2$ sont indépendamment un atome d'hydrogène, un atome d'halogène, un groupe alkyle inférieur ou un groupe alcoxy inférieur ; m est un nombre entier de 1 à 20 ; et n est un nombre entier de 1 à 20, qui comprend la réaction d'un composé de formule :

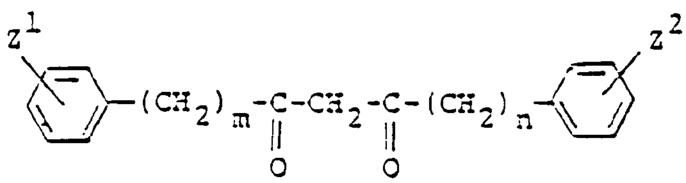

(IV-1)

où $Z^1$, $Z^2$, m et n sont tels que définis ci-dessus, avec un agent diazotant en présence d'une base organique pour donner un composé de formule :

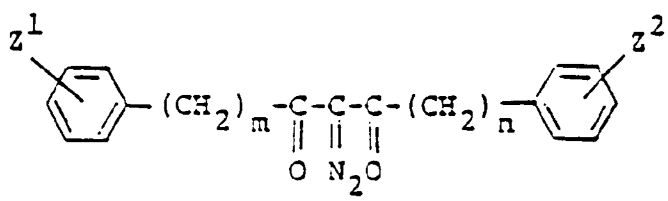

(IV-4)

où $Z^1$, $Z^2$, m et n sont tels que définis ci-dessus, suivie d'une réaction avec de l'acide chlorosulfonique.

8. Procédé pour préparer un composé de formule (IV') de la revendication 7, qui comprend

la réaction du composé de formule (IV-1) de la revendication 7 avec un agent sulfonant, suivie d'une neutralisation pour donner un composé de formule :

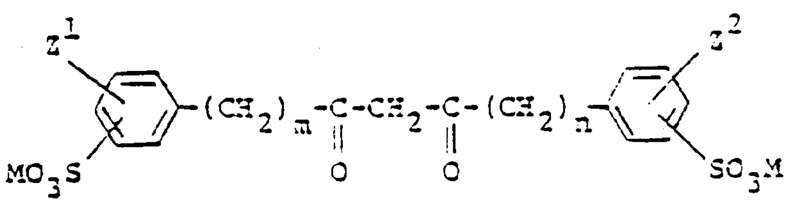

(IV-2)

où M est un atome de métal alcalin, un groupe ammonium ou un résidu d'une base organique ; $Z^1$, $Z^2$, m et n sont tels que définis ci-dessus,

la réaction du composé de formule (IV-2) avec un agent diazotant en présence d'une base organique pour donner un composé de formule :

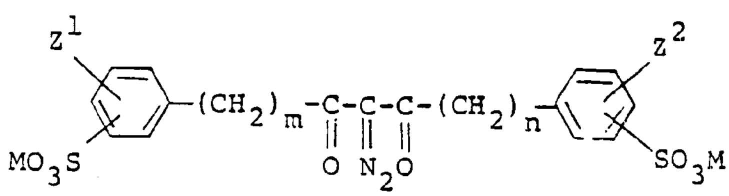

(IV-3)

où $Z^1$, $Z^2$, M, m et n sont tels que définis ci-dessus, suivie d'une réaction avec un agent chlorant.

**9.** Procédé pour préparer un composé de formule :

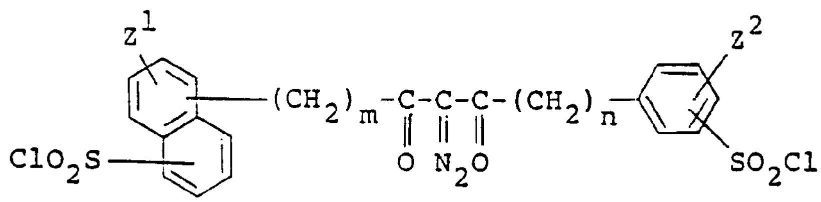

(V')

où $Z^1$ et $Z^2$ sont indépendamment un atome d'hydrogène, un atome d'halogène, un groupe alkyle inférieur ou un groupe alcoxy inférieur ; m est un nombre entier de 1 à 20 ; et n est un nombre entier de 1 à 20, qui comprend la réaction d'un composé de formule :

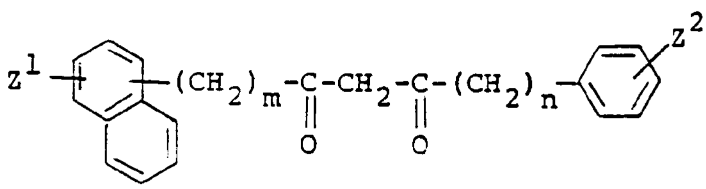

(V-1)

où $Z^1$, $Z^2$, m et n sont tels que définis ci-dessus, avec un agent diazotant en présence d'une base organique pour donner un composé de formule :

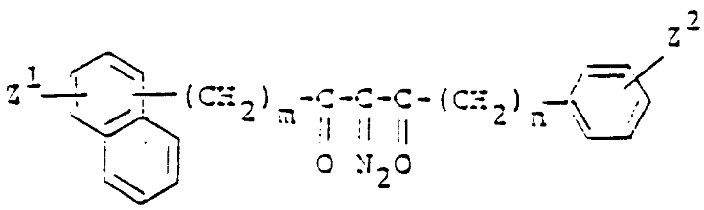

(V-4)

où $Z^1$, $Z^2$, m et n sont tels que définis ci-dessus, suivie d'une réaction avec de l'acide chlorosulfonique.

**10.** Procédé pour préparer un composé de formule (V') de la revendication 9, qui comprend

la réaction du composé de formule (V-1) de la revendication 9 avec un agent sulfonant, suivie d'une neutralisation pour donner un composé de formule :

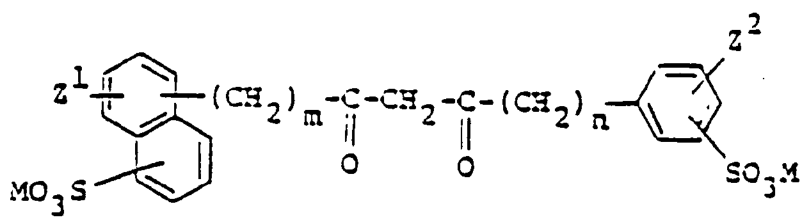

(V-2)

où M est un atome de métal alcalin, un groupe ammonium ou un résidu d'une base organique ; $Z^1$, $Z^2$, m et n sont tels que définis ci-dessus,

la réaction du composé de formule (V-2) avec un agent diazotant en présence d'une base organique pour donner un composé de formule :

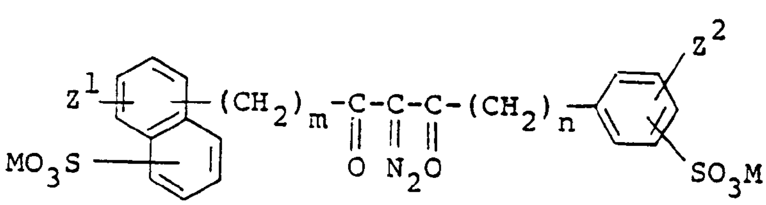

(V-3)

où $Z^1$, $Z^2$, M, m et n sont tels que définis ci-dessus, suivie d'une réaction avec un agent halogénant.

**11.** Procédé pour préparer un composé de formule :

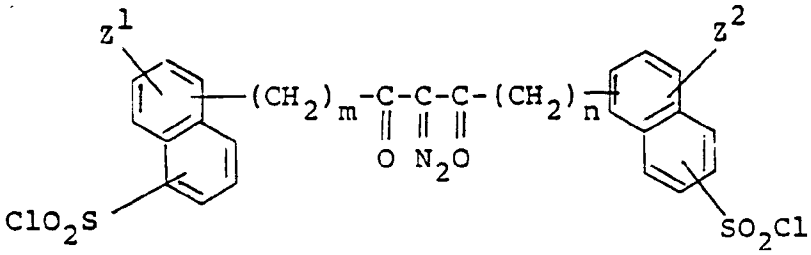

(VI')

où $Z^1$ et $Z^2$ sont indépendamment un atome d'hydrogène, un atome d'halogène, un groupe alkyle inférieur ou un groupe alcoxy inférieur ; m est un nombre entier de 1 à 20 ; et n est un nombre entier de 1 à 20, qui comprend la réaction d'un composé de formule :

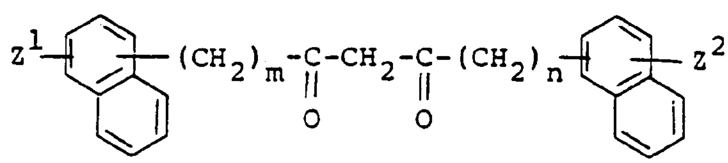

(VI-1)

où $Z^1$, $Z^2$, m et n sont tels que définis ci-dessus, avec un agent diazotant en présence d'une base organique pour donner un composé de formule :

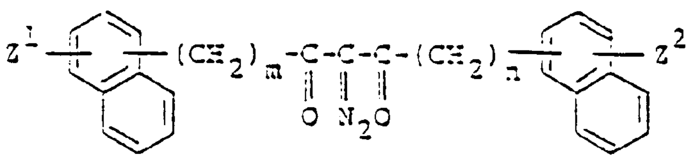

(VI-4)

où $Z^1$, $Z^2$, m et n sont tels que définis ci-dessus, suivie d'une réaction avec de l'acide chlorosulfonique.

**12.** Procédé pour préparer un composé de formule (VI') de la revendication 11, qui comprend

la réaction du composé de formule (VI-1) de la revendication 11 avec un agent sulfonant, suivie d'une neutralisation pour donner un composé de formule :

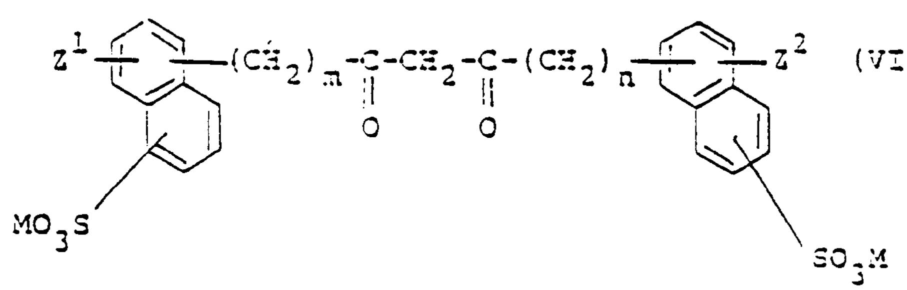

(VI-2)

où M est un atome de métal alcalin, un groupe ammonium ou un résidu d'une base organique ; $Z^1$, $Z^2$, m et n sont tels que définis ci-dessus,

la réaction du composé de formule (VI-2) avec un agent diazotant en présence d'une base pour donner un composé de formule :

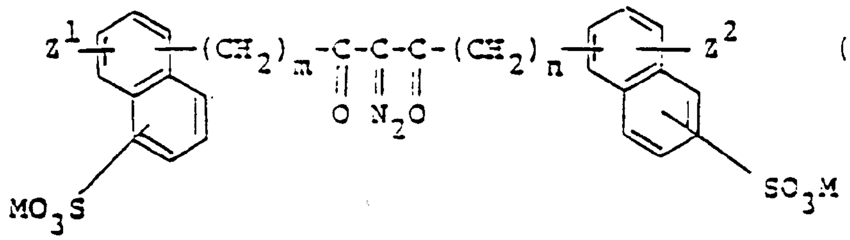

(VI-3)

où M, $Z^1$, $Z^2$, m et n sont tels que définis ci-dessus, suivie d'une réaction avec un agent chlorant.

**13.** Procédé pour préparer un composé de formule :

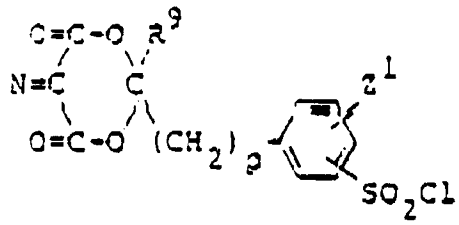

(VII')

où $Z^1$ est un atome d'hydrogène, un atome d'halogène, un groupe alkyle inférieur ou un groupe alcoxy inférieur ; $R^9$ est un groupe alkyle, un groupe aralkyle, un groupe hydroxyalkyle, ou un groupe alcoxyalkyle ; et p est un nombre entier de 1 à 20, qui comprend la réaction d'un composé de formule :

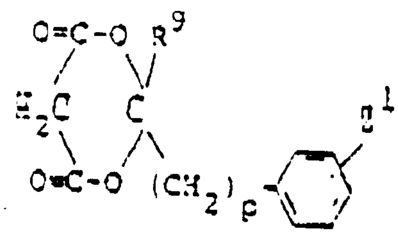

(VII-1)

où $Z^1$, $R^9$ et p sont tels que définis ci-dessus, avec un agent diazotant en présence d'une base organique pour donner un composé de formule :

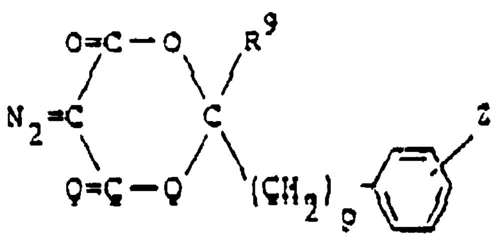

(VII-4)

où $Z^1$, $R^9$ et p sont tels que définis ci-dessus, suivie d'une réaction avec de l'acide chlorosulfonique.

**14.** Procédé pour préparer le composé de formule (VII') de la revendication 13, qui comprend la réaction du composé de formule (VII-1) de la revendication 13 avec un agent sulfonant, suivie d'une neutralisation pour donner un composé de formule :

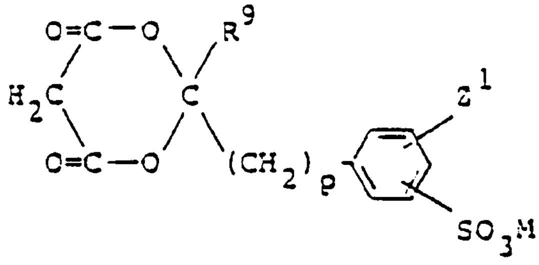

(VII-2)

où M est un atome de métal alcalin, un groupe ammonium ou un résidu d'une base organique ; $Z^1$, $R^9$ et p sont tels que définis ci-dessus,

la réaction du composé de formule (VII-2) avec un agent diazotant en présence d'une base organique pour donner un composé de formule :

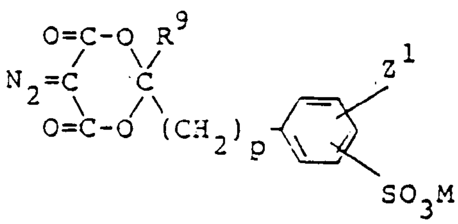

(VII-3)

où M, $Z^1$, $R^9$ et p sont tels que définis ci-dessus, suivie d'une réaction avec un agent chlorant.

**15.** Procédé de préparation de plaques photogravées employant un agent photosensible, dans lequel l'agent est un composé selon la revendication 1.

FIG. 1(a)

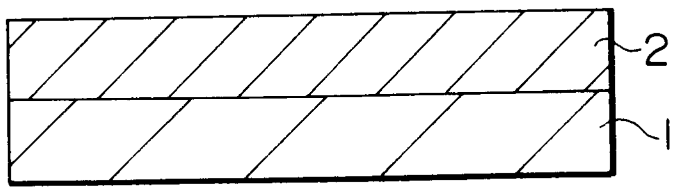


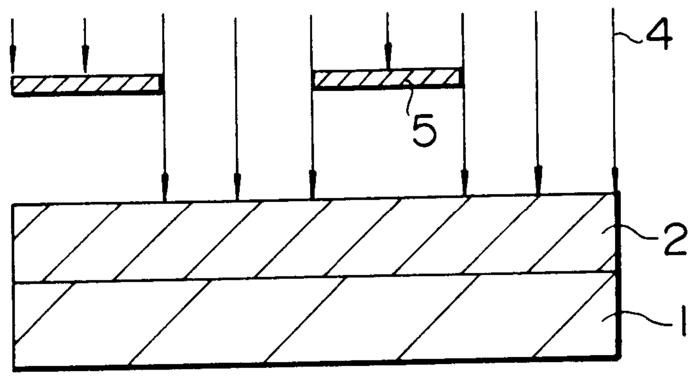


FIG. 1(b)

FIG. 1(c)

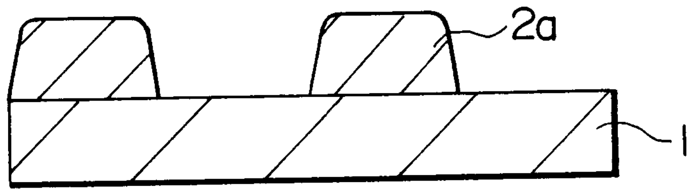

FIG. 2

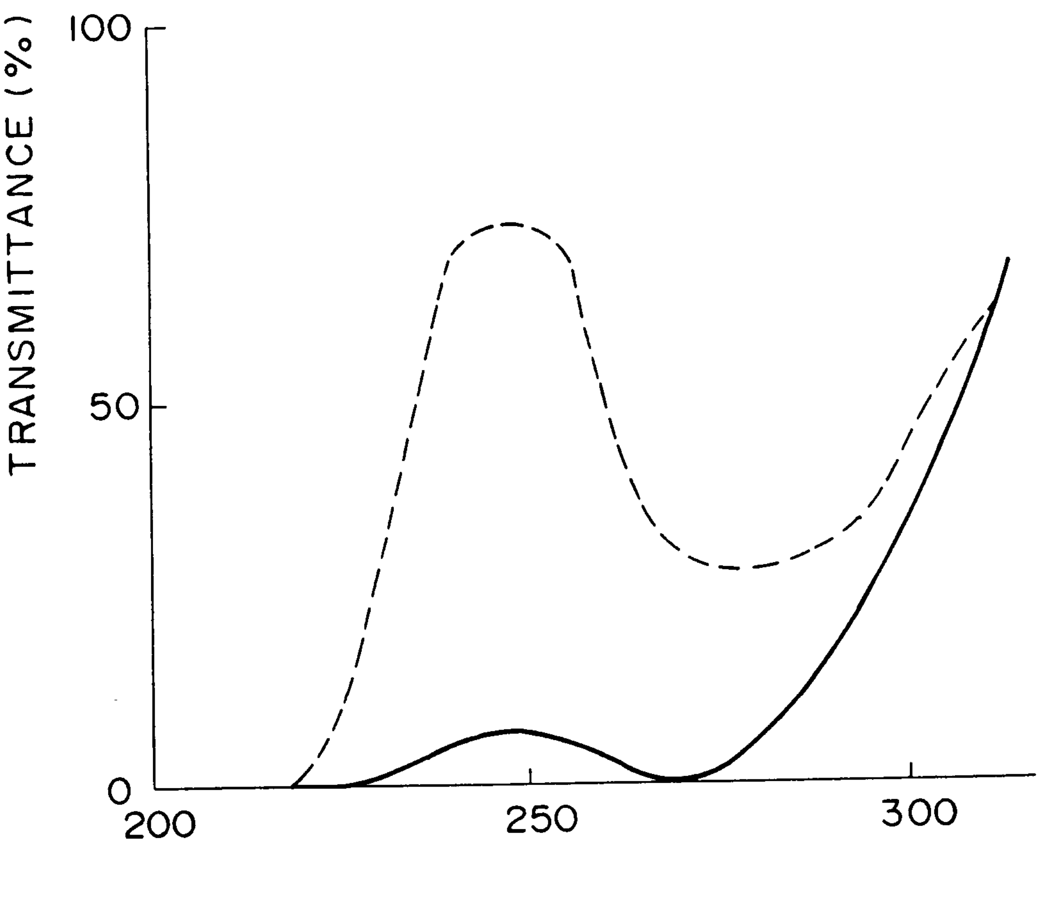

FIG. 3